# EUROPEAN PATENT SPECIFICATION

(11) **EP 2 149 585 B1**
(45) Date of publication and mention of the grant of the patent: **14.08.2013**
(21) Application number: 09075395.5
(22) Date of filing: 04.11.2004
(51) Int. Cl.: C07K 16/28, A61K 38/20, A61K 39/395

(54) **Use of antagonistic anti-CD40 monoclonal antibodies**
Verwendung von antagonistischen anti-CD40-monoklonale Antikörper
Procédés d'utilisation d'anticorps monoclonaux antagonistes anti-CD40

(30) Priority: 04.11.2003 US 517337; 26.11.2003 US 525579; 27.04.2004 US 565710
(43) Date of publication of application: 03.02.2010
(62) Divisional of application: 04810510.0
(73) Proprietor: Novartis Vaccines and Diagnostics, Inc., Emeryville, CA 94608 (US)
(72) Inventor: Long, Li, Emeryville, CA 94662-8097 (US); Luqman, Mohammed, Emeryville, CA 94662-8097 (US); Yabannavar, Asha, Emeryville, CA 94662-8097 (US); Zaror, Isabel, Emeryville, CA 94662-8097 (US); Chen, Bao-Lu, Emeryville, CA 94662-8097 (US); Lu, Xiaofeng, Emeryville, CA 94662-8097 (US); Lee, Sang Hoon, Emeryville, CA 94662-8097 (US); Hurst, Deborah, Emeryville, CA 94662-8097 (US)
(74) Representative: Marshall, Cameron John

(56) References cited:
- WO-A-01/83755
- WO-A-02/28904
- WO-A-02/088186
- ELLMARK P ET AL: "Modulation of the CD40-CD40 ligand interaction using human anti-CD40 single-chain antibody fragments obtained from the n-CoDeR phage display library" IMMUNOLOGY, BLACKWELL PUBLISHING, OXFORD, GB, vol. 106, no. 4, 1 August 2002 (2002-08-01), pages 456-463, XP002326342 ISSN: 0019-2805
- MALMBORG A-C M ET AL: "Affinity and epitope profiling of mouse anti-CD40 monoclonal antibodies" SCANDINAVIAN JOURNAL OF IMMUNOLOGY, BLACKWELL SCIENCE PUBL., OXFORD, GB, vol. 57, no. 6, 1 June 2003 (2003-06-01), pages 517-524, XP002326343 ISSN: 0300-9475
- LITTLE M ET AL: "Of mice and men: hybridoma and recombinant antibodies" IMMUNOLOGY TODAY, ELSEVIER PUBLICATIONS, CAMBRIDGE, GB, vol. 21, no. 8, 1 August 2000 (2000-08-01), pages 364-370, XP004215163 ISSN: 0167-5699

## Description

### FIELD OF THE INVENTION

The invention relates to human antibodies capable of binding to CD40, methods of using the antibodies, and methods for treatment of diseases mediated by stimulation of CD40 signaling on CD40-expressing cells.

### BACKGROUND OF THE INVENTION

B cells play an important role during the normal *in vivo* immune response. A foreign antigen will bind to surface immunoglobulins on specific B cells, triggering a chain of events including endocytosis, processing, presentation of processed peptides on MHC-class II molecules, and up-regulation of the B7 antigen on the B cell surface. A specific T cell then binds to the B cell via T cell receptor (TCR) recognition of the processed antigen presented on the MHC-class II molecule. Stimulation through the TCR activates the T cell and initiates T-cell cytokine production. A second signal that further activates the T cell is an interaction between the CD28 antigen on T cells and the B7 antigen on B cells. When the above-mentioned signals are received, the CD40 ligand (CD40L or CD154), which is not expressed on resting human T cells, is up-regulated on the T-cell surface. Binding of the CD40 ligand to the CD40 antigen on the B cell surface stimulates the B cell, causing the B cell to mature into a plasma cell secreting high levels of soluble immunoglobulin.

CD40 is a 55 kDa cell-surface antigen present on the surface of both normal and neoplastic human B cells, dendritic cells, antigen presenting cells (APCs), endothelial cells, monocytic and epithelial cells. Transformed cells from patients with low- and high-grade B-cell lymphomas, B-cell acute lymphoblastic leukemia, multiple myeloma, chronic lymphocytic leukemia, and Hodgkin's disease express CD40. CD40 expression is also detected in two-thirds of acute myeloblastic leukemia cases and 50% of AIDS-related lymphomas. Malignant B cells from several tumors of B-cell lineage express a high degree of CD40 and appear to depend on CD40 signaling for survival and proliferation.

Immunoblastic B-cell lymphomas frequently arise in immunocompromised individuals such as allograft recipients and others receiving long-term immunosuppressive therapy, AIDS patients, and patients with primary immunodeficiency syndromes such as X-linked lymphoproliferative syndrome or Wiscott-Aldrich syndrome (Thomas et al. (1991) Adv. Cancer Res. 57:329; Straus et al. (1993) Ann. Intern. Med. 118:45).

The CD40 antigen is related to the human nerve growth factor (NGF) receptor, tumor necrosis factor-α (TNF-α) receptor, and Fas, suggesting that CD40 is a receptor for a ligand with important functions in B-cell activation. CD40 expression on APCs plays an important co-stimulatory role in the activation of both T-helper and cytotoxic T lymphocytes. The CD40 receptor is expressed on activated T cells, activated platelets, and inflamed vascular smooth muscle cells. CD40 receptors can also be found on eosinophils, synovial membranes in rheumatoid arthritis, dermal fibroblasts, and other non-lymphoid cell types. Binding of CD40L to the CD40 receptor stimulates B-cell proliferation and differentiation, antibody production, isotype switching, and B-cell memory generation.

WO 01/83755 and WO 02/28904 describe antibodies capable of binding to CD40, methods for producing these antibodies and methods for their use.

Ellmark et al. (Immunology 2002, 106, 456-463) reports on the modulation of the CD40-CD40 ligand interaction using human anti-CD40 single-chain antibody fragments.

### BRIEF SUMMARY OF THE INVENTION

A human anti-CD40 monoclonal antibody, for use in treating a cancer characterized by expression of CD40, wherein said human anti-CD40 monoclonal antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No.PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent
Deposit No. PTA-5542 or the monoclonal antibody CHIR-12.12 obtainable from the hybridoma cell line deposited with the ATCC as Patent Deposit No. PTA-5543 in a competitive binding assay,
wherein said antibody is administered in combination with one or more further therapeutic agents.

### BRIEF SUMMARY OF THE DISCLOSURE

Compositions and methods are disclosed for treating diseases mediated by stimulation of CD40 signaling on CD40-expressing cells, including lymphomas, autoimmune diseases, and transplant rejections. Compositions include monoclonal antibodies capable of binding to a human CD40 antigen located on the surface of a human CD40-expressing cell, wherein the binding prevents the growth or differentiation of the cell. Compositions also include monoclonal antibodies capable of specifically binding to a human CD40 antigen expressed on the surface of a human CD40-expressing cell, said monoclonal antibody being free of significant agonist activity, wherein administration of said monoclonal antibody results in significantly less tumor volume than a similar concentration of the chimeric anti-CD20 monoclonal antibody IDEC-C2B8 in a staged nude mouse xenograft tumor model using the Daudi human B cell lymphoma cell line. Compositions also include antigen-binding fragments of these monoclonal antibodies, hybridoma cell lines producing these antibodies, and isolated nucleic acid molecules encoding the amino acid sequences of these monoclonal antibodies. Also disclosed herein are pharamaceutical compositions comprising these anti-CD40 antibodies in a pharmaceutically acceptable carrier.

Methods are disclosed for preventing or treating a disease mediated by stimulation of CD40 signaling, comprising treating the patient with an anti-CD40 antibody or an antigen-binding fragment thereof that is free of significant agonist activity when bound to a CD40 antigen on a human CD40-expressing cell. Diseases mediated by stimulation of CD40-expressing cells include autoimmune diseases, cancers, and organ and tissue graft rejections. Lymphomas that can be treated or prevented by a method as disclosed herein include non-Hodgkin's lymphomas (high-grade lymphomas, intermediate-grade lymphomas, and low-grade lymphomas), Hodgkin's disease, acute lymphoblastic leukemias, myelomas, chronic lymphocytic leukemias, and myeloblastic leukemias.

Particular autoimmune diseases contemplated for treatment using the methods disclosed herein include systemic lupus erythematosus (SLE), rheumatoid arthritis, Crohn's disease, psoriasis, autoimmune thrombocytopenic purpura, multiple sclerosis, ankylosing spondylitis, myasthenia gravis, and pemphigus vulgaris. Such antibodies could also be used to prevent rejection of organ and tissue grafts by suppressing autoimmune responses, to treat lymphomas by depriving malignant B lymphocytes of the activating signal provided by CD40, and to deliver toxins to CD40-bearing cells in a specific manner.

Methods for inhibiting the growth, differentiation, and/or proliferation of human B cells and for inhibiting antibody production by B cells in a human patient are disclosed as are methods for inhibiting the growth of cancer cells of a B-cell lineage. Methods for identifying antibodies that have antagonist activity toward CD40-expressing cells are also disclosed.

The monoclonal antibodies disclosed herein have a strong affinity for CD40 and are characterized by a dissociation equilibrium constant (K_{D}) of at least 10⁻⁶ M, preferably at least about 10⁻⁷ M to about 10⁻⁸ M, more preferably at least about 10⁻⁸ M to about 10⁻¹² M. Monoclonal antibodies and antigen-binding fragments thereof that are suitable for use in the methods disclosed herein are capable of specifically binding to a human CD40 antigen expressed on the surface of a human cell. They are free of significant agonist activity but exhibit antagonist activity when bound to CD40 antigen on human cells. In one embodiment, the anti-CD40 antibody or fragment thereof exhibits antagonist activity when bound to CD40 antigen on normal human B cells. In another embodiment, the anti-CD40 antibody or fragment thereof exhibits antagonist activity when bound to CD40 antigen on malignant human B cells. Suitable monoclonal antibodies have human constant regions; preferably they also have wholly or partially humanized framework regions; and most preferably are fully human antibodies or antigen-binding fragments thereof. Examples of such monoclonal antibodies are the antibodies designated herein as CHIR-5.9 and CHIR-12.12; the monoclonal antibodies produced by the hybridoma cell lines designated 131.2F8.5.9 (referred to herein as the cell line 5.9) and 153.8E2.D10.D6.12.12 (referred to herein as the cell line 12.12); a monoclonal antibody comprising an amino acid sequence selected from the group consisting of the sequence shown in SEQ ID NO:6, the sequence shown in SEQ ID NO:7, the sequence shown in SEQ ID NO:8, both the sequence shown in SEQ ID NO:6 and SEQ ID NO:7, and both the sequence shown in SEQ ID NO:6 and SEQ ID NO:8; a monoclonal antibody comprising an amino acid sequence selected from the group consisting of the sequence shown in SEQ ID NO:2, the sequence shown in SEQ ID NO:4, the sequence shown in SEQ ID NO:5, both the sequence shown in SEQ ID NO:2 and SEQ ID NO:4, and both the sequence shown in SEQ ID NO:2 and SEQ ID NO:5; a monoclonal antibody comprising an amino acid sequence encoded by a nucleic acid molecule comprising a nucleotide sequence selected from the group consisting of the sequence shown in SEQ ID NO:1, the sequence shown in SEQ ID NO:3, and both the sequence shown in SEQ ID NO:1 and SEQ ID NO:3; and antigen-binding fragments of these monoclonal antibodies that retain the capability of specifically binding to human CD40, and which are free of significant agonist activity but exhibit antagonist activity when bound to CD40 antigen on human cells. Examples of such monoclonal antibodies also include a monoclonal antibody that binds to an epitope capable of binding the monoclonal antibody produced by the hybridoma cell line 12.12; a monoclonal antibody that binds to an epitope comprising residues 82-87 of the amino acid sequence shown in SEQ ID NO:10 or SEQ ID NO:12; a monoclonal antibody that competes with the monoclonal antibody CHIR-12.12 in a competitive binding assay; and a monoclonal antibody that is an antigen-binding fragment of the CHIR-12.12 monoclonal antibody or any of the foregoing monoclonal antibodies, where the fragment retains the capability of specifically binding to the human CD40 antigen. Those skilled in the art recognize that the antagonist antibodies and antigen-binding fragments of these antibodies disclosed herein include antibodies and antigen-binding fragments thereof that are produced recombinantly using methods well known in the art and described herein below, and include, for example, monoclonal antibodies CHIR-5.9 and CHIR-12.12 that have been recombinantly produced.

In one embodiment disclosed herein, methods of treatment comprise administering to a patient a therapeutically effective dose of a pharmaceutical composition comprising suitable antagonist anti-CD40 antibodies or antigen-binding fragments thereof. A therapeutically effective dose of the anti-CD40 antibody or fragment thereof is in the range from about 0.01 mg/kg to about 40 mg/kg, from about 0.01 mg/kg to about 30 mg/kg, from about 0.1 mg/kg to about 30 mg/kg, from about 1 mg/kg to about 30 mg/kg, from about 3 mg/kg to about 30 mg/kg, from about 3 mg/kg to about 25 mg/kg, from about 3 mg/kg to about 20 mg/kg, from about 5 mg/kg to about 15 mg/kg, or from about 7 mg/kg to about 12 mg/kg. It is recognized that the method of treatment may comprise a single administration of a therapeutically effective dose or multiple administrations of a therapeutically effective dose of the antagonist anti-CD40 antibody or antigen-binding fragment thereof.

The antagonist anti-CD40 antibodies identified herein as being suitable for use in the methods disclosed herein may be modified. Modifications of these antagonist anti-CD40 antibodies include, but are not limited to, immunologically active chimeric anti-CD40 antibodies, humanized anti-CD40 antibodies, and immunologically active murine anti-CD40 antibodies.

### BRIEF DESCRIPTION OF THE DRAWINGS

Figure 1 shows binding of CHIR-5.9 and CHIR-12.12 monoclonal antibodies to CD40 on the surface of lymphoma cell line (Ramos).
Figures 2A and 2B illustrate binding properties of the CHIR-5.9 and CHIR-12.12 monoclonal anti-CD40 antibodies relative toCD40 ligand. Figure 2A shows that binding of CHIR-5.9 and CHIR-12.12 monoclonal antibodies to cell surface CD40 prevents subsequent CD40-ligand binding. Figure 2B shows that the CHIR-5.9 and CHIR-12.12 monoclonal antibodies can compete off CD40 ligand pre-bound to cell surface CD40.
Figures 3A and 3B show ADCC activity of the candidate monoclonal antibodies CHIR-5.9 and CHIR-12.12 against cancer cells from the lymph nodes of non-Hodgkin's lymphoma (NHL) patients. Enriched NK cells from a normal volunteer donor either fresh after isolation (Figure 3A) or after culturing overnight at 37°C (Figure 3B) were used as effector cells in this assay. As NHL cells also express CD20, the target antigen for rituximab (Rituxan®), ADCC activity of the candidate mAbs was compared with that ofrituximab.
Figure 4 demonstrates *in vivo* anti-tumor activity of monoclonal antibodies CHIR-5.9 and CHIR-12.12 compared to that of rituximab using an unstaged nude mouse xenograft B cell lymphoma (Namalwa) model.
Figure 5 demonstrates *in vivo* anti-tumor activity of monoclonal antibodies CHIR-5.9 and CHIR-12.12 compared to that of rituximab using an unstaged nude mouse xenograft B cell lymphoma (Daudi) model. RC, resistance to tumor challenge.
Figure 6 demonstrates *in vivo* anti-tumor activity of monoclonal antibodies CHIR-5.9 and CHIR-12.12 compared to that of rituximab using a staged nude mouse xenograft B cell lymphoma (Daudi) model. CR, complete regression.
Figure 7 shows the protocol used for determining the number of CD20 and CD40 molecules on Namalwa and Daudi cells.
Figure 8 shows comparative ADCC of the mAb CHIR-12.12 and rituximab against Daudi lymphoma cells.

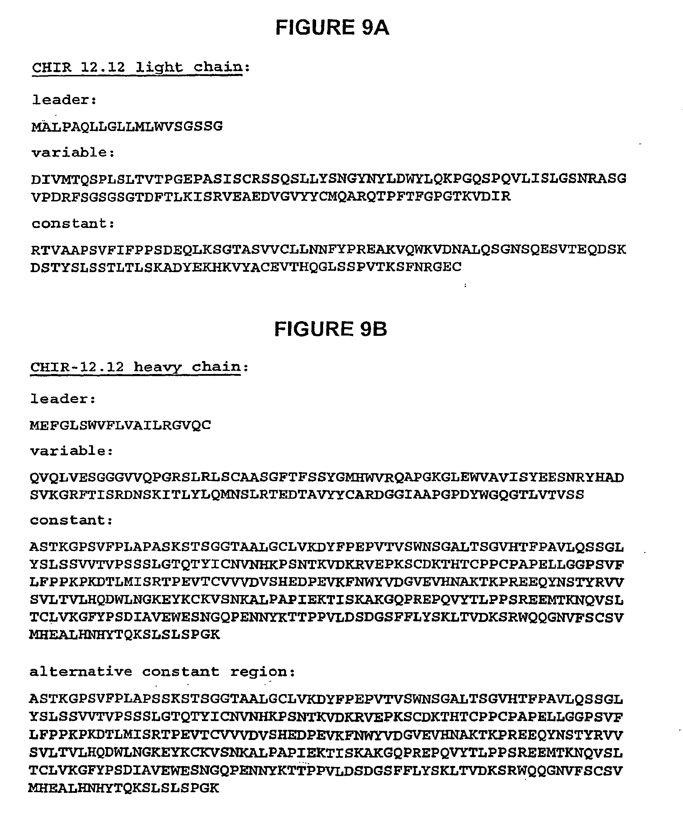

Figure 9 sets forth the amino acid sequences for the light and heavy chains of the mAb CHIR-12.12. The leader (residues 1-20 of SEQ ID NO:2), variable (residues 21-132 of SEQ ID NO:2), and constant (residues 133-239 of SEQ ID NO:2) regions of the light chain are shown in Figure 9A. The leader (residues 1-19 of SEQ ID NO:4), variable (residues 20-139 of SEQ ID NO:4), and constant (residues 140-469 of SEQ ID NO:4) regions of the heavy chain are shown in Figure 9B. The alternative constant region for the heavy chain of the mAb CHIR-12.12 shown in Figure 9B reflects a substitution of a serine residue for the alanine residue at position 153 of SEQ ID NO:4. The complete sequence for this variant of the heavy chain of the mAb CHIR-12.12 is set forth in SEQ ID NO:5.
Figure 10 shows the coding sequence for the light chain (Figure 10A; SEQ ID NO:1) and heavy chain (Figure 10B; SEQ ID NO:3) for the mAb CHIR-12.12.

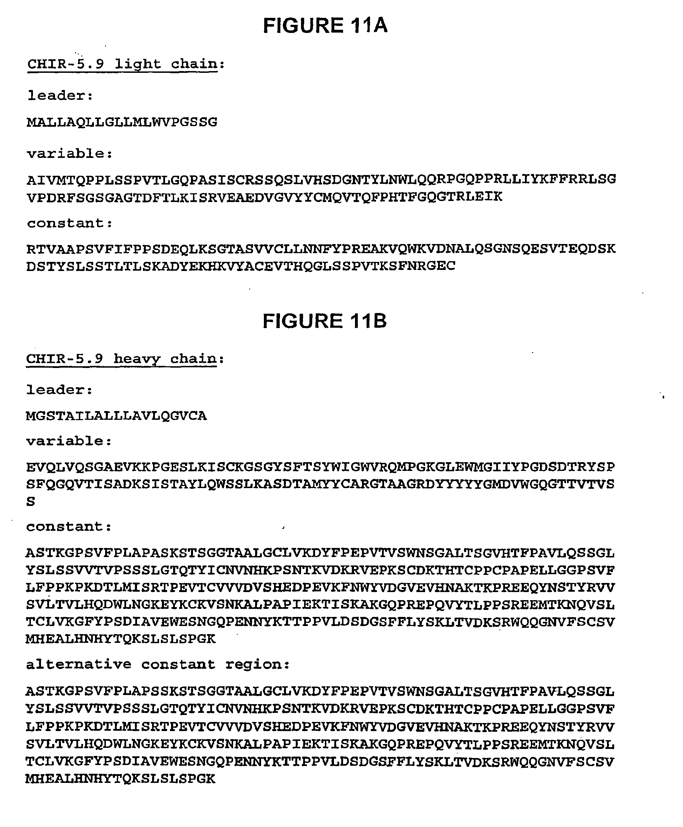

Figure 11 sets forth the amino acid sequences for the light and heavy chains of mAb CHIR-5.9. The leader (residues 1-20 of SEQ ID NO:6), variable (residues 21-132 of SEQ ID NO:6), and constant (residues 133-239 of SEQ ID NO:6) regions of the light chain are shown in Figure 11A. The leader (residues 1-19 of SEQ ID NO:7), variable (residues 20-144 of SEQ ID NO:7), and constant (residues 145-474 of SEQ ID NO:7) regions of the heavy chain are shown in Figure 11B. The alternative constant region for the heavy chain of the mAb CHIR-5.9 shown in Figure 11B reflects a substitution of a serine residue for the alanine residue at position 158 of SEQ ID NO:7. The complete sequence for this variant of the heavy chain of the mAB CHIR-5.9 is set forth in SEQ ID NO:8.
Figure 12 shows the coding sequence (Figure 12A; SEQ ID NO:9) for the short isoform of human CD40 (amino acid sequence shown in Figure 12B; SEQ ID NO:10), and the coding sequence (Figure 12C; SEQ ID NO:11) for the long isoform of human CD40 (amino acid sequence shown in Figure 12D).
Figure 13 shows thermal melting temperature of CHIR-12.12 in different pH formulations measured by differential scanning calorimetry (DSC).

### DETAILED DESCRIPTION

The scope of the present invention is defined by the claims and any information that does not fall within the claims is provided for information only. "Tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to lymphoma and leukemia.

"Antibodies" and "immunoglobulins" (Igs) are glycoproteins having the same structural characteristics. While antibodies exhibit binding specificity to an antigen, immunoglobulins include both antibodies and other antibody-like molecules that lack antigen specificity. Polypeptides of the latter kind are, for example, produced at low levels by the lymph system and at increased levels by myelomas.

The term "antibody" is used in the broadest sense and covers fully assembled antibodies, antibody fragments that can bind antigen (e.g., Fab', F'(ab)₂, Fv, single chain antibodies, diabodies), and recombinant peptides comprising the foregoing.

The term "monoclonal antibody" as used herein refers to an antibody obtained from a population of substantially homogeneous antibodies, i.e., the individual antibodies comprising the population are identical except for possible naturally occurring mutations that may be present in minor amounts.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity determining regions (CDRs) or hypervariable regions both in the light-chain and the heavy-chain variable domains. The more highly conserved portions of variable domains are called the framework (FR) regions. The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat et al. (1991) NIH Publ. No. 91-3242, Vol. I, pages 647-669).

The constant domains are not involved directly in binding an antibody to an antigen, but exhibit various effecter functions, such as Fc receptor (FcR) binding, participation of the antibody in antibody-dependent cellular toxicity, opsonization, initiation of complement dependent cytotoxicity, and mast cell degranulation.

The term "hypervariable region" when used herein refers to the amino acid residues of an antibody that are responsible for antigen binding. The hypervariable region comprises amino acid residues from a "complementarity determining region" or"CDR" (i.e., residues 24-34 (L1), 50-56 (L2), and 89-97 (L3) in the light-chain variable domain and 31-35 (H1), 50-65 (H2), and 95-102 (H3) in the heavy-chain variable domain; Kabat et al. (1991) Sequences of Proteins of Immological Interest (5th ed., Public Health Service, National Institute of Health, Bethesda, MD) and/or those residues from a "hypervariable loop" (i.e., residues 26-32(L1), 50-52 (L2), and 91-96 (L3) in the light-chain variable domain and 26-32(H1), 53-55 (H2), and 96-101 (H3) in the heavy-chain variable domain; Clothia and Lesk (1987) J. Mol. Biol. 196:901-917). "Framework" or "FR" residues are those variable domain residues other than the hypervariable region residues.

"Antibody fragments" comprise a portion of an intact antibody, preferably the antigen-binding or variable region of the intact antibody. Examples of antibody fragments include Fab, Fab', F(ab')2, and Fv fragments; diabodies; linear antibodies (Zapata et al. (1995) Protein Eng. 8(10):1057-1062); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen.

"Fv" is the minimum antibody fragment that contains a complete antigen recognition and binding site. In a two-chain Fv species, this region consists of a dimer of one heavy- and one light-chain variable domain in tight, non-covalent association. In a single-chain Fv species, one heavy- and one light-chain variable domain can be covalently linked by flexible peptide linker such that the light and heavy chains can associate in a "dimeric" structure analogous to that in a two-chain Fv species. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (C_{H}1) of the heavy chain. Fab fragments differ from Fab' fragments by the addition of a few residues at the carboxy terminus of the heavy-chain C_{H}1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of human immunoglobulins: IgA, IgD, IgE, IgG, and IgM, and several of these may be further divided into subclasses (isotypes), e.g., IgG1, IgG2, IgG3, IgG4, IgA, and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known. Different isotypes have different effector functions. For example, human IgG1 and IgG3 isotypes mediate antibody-dependent cell-mediated cytotoxicity (ADCC) activity.

The word "label" when used herein refers to a detectable compound or composition that is conjugated directly or indirectly to the antibody so as to generate a "labeled" antibody. The label may be detectable by itself (e.g., radioisotope labels or fluorescent labels) or, in the case of an enzymatic label, may catalyze chemical alteration of a substrate compound or composition that is detectable. Radionuclides that can serve as detectable labels include, for example, I-131, I-123, I-125, Y-90, Re-188, Re-186, At-211, Cu-67, Bi-212, and Pd-109. The label might also be a non-detectable entity such as a toxin.

The term "antagonist" is used in the broadest sense, and includes any molecule that partially or fully blocks, inhibits, or neutralizes a biological activity of a native target disclosed herein or the transcription or translation thereof.

"Carriers" as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, succinate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN, polyethylene glycol (PEG), and Pluronics. Administration "in combination with" one or more further therapeutic agents includes simultaneous (concurrent) and consecutive administration in any order.

A "host cell," as used herein, refers to a microorganism or a eukaryotic cell or cell line cultured as a unicellular entity that can be, or has been, used as a recipient for a recombinant vector or other transfer polynucleotides, and include the progeny of the original cell that has been transfected. It is understood that the progeny of a single cell may not necessarily be completely identical in morphology or in genomic or total DNA complement as the original parent, due to natural, accidental, or deliberate mutation.

"Human effector cells" are leukocytes that express one or more FcRs and perform effector functions. Preferably, the cells express at least FcγRIII and carry out antigen-dependent cell-mediated cyotoxicity (ADCC) effector function. Examples of human leukocytes that mediate ADCC include peripheral blood mononuclear cells (PBMC), natural killer (NK) cells, monocytes, macrophages, eosinophils, and neutrophils, with PBMCs and NK cells being preferred. Antibodies that have ADCC activity are typically of the IgG1 or IgG3 isotype. Note that in addition to isolating IgG1 and IgG3 antibodies, such ADCC-mediating antibodies can be made by engineering a variable region from a non-ADCC antibody or variable region fragment to an IgG1 or IgG3 isotype constant region.

The terms "Fc receptor" or "FcR" are used to describe a receptor that binds to the Fc region of an antibody. The preferred FcR is a native-sequence human FcR. Moreover, a preferred FcR is one that binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see Daeron (1997) Annu. Rev. Immunol. 15:203-234). FcRs are reviewed in Ravetch and Kinet (1991) Annu. Rev. Immunol. 9:457-9.92 (1991); Capel et al. (1994) Immunomethods 4:25-34; and de Haas et al. (1995) J. Lab. Clin. Med. 126:330-341. Other FcRs, including those to be identified in the future, are encompassed by the term "FcR" herein. The term also includes the neonatal receptor, FcRn, which is responsible for the transfer of maternal IgGs to the fetus (Guyer et al. (1976) J. Immunol. 117:587 and Kim et al. (1994) J. Immunol. 24:249 (1994)).

There are a number of ways to make human antibodies. For example, secreting cells can be immortalized by infection with the Epstein-Barr virus (EBV). However, EBV-infected cells are difficult to clone and usually produce only relatively low yields of immunoglobulin (James and Bell (1987) J. Immunol. Methods 100:5-40). In the future, the immortalization of human B cells might possibly be achieved by introducing a defined combination of transforming genes. Such a possibility is highlighted by a recent demonstration that the expression of the telomerase catalytic subunit together with the SV40 large oncoprotein and an oncogenic allele of H-ras resulted in the tumorigenic conversion of normal human epithelial and fibroblast cells (Hahn et al. (1999) Nature 400:464-468). It is now possible to produce transgenic animals (e.g., mice) that are capable, upon immunization, of producing a repertoire of human antibodies in the absence of endogenous immunoglobulin production (Jakobovits et al. (1993) Nature 362:255-258; Lonberg and Huszar (1995) Int. Rev. Immunol. 13:65-93; Fishwild et al. (1996) Nat. Biotechnol. 14:845-851; Mendez et al. (1997) Nat. Genet. 15:146-156; Green (1999) J. Immunol. Methods 231:11-23; Tomizuka et al. (2000) Proc. Natl. Acad. Sci. USA 97:722-727; reviewed in Little et al. (2000) Immunol. Today 21:364-370). For example, it has been described that the homozygous deletion of the antibody heavy-chain joining region (J_{H}) gene in chimeric and germ-line mutant mice results in complete inhibition of endogenous antibody production (Jakobovits et al. (1993) Proc. Natl. Acad. Sci. USA 90:2551-2555). Transfer of the human germ-line immunoglobulin gene array in such germ-line mutant mice results in the production of human antibodies upon antigen challenge (Jakobovits et al. (1993) Nature 362:255-258). Mendez et al. (1997) (Nature Genetics 15:146-156) have generated a line of transgenic mice that, when challenged with an antigen, generates high affinity fully human antibodies. This was achieved by germ-line integration of megabase human heavy-chain and light-chain loci into mice with deletion into endogenous J_{H} segment as described above. These mice (XenoMouse^{®} II technology (Abgenix; Fremont, California)) harbor 1,020 kb of human heavy-chain locus containing approximately 66 V_{H} genes, complete D_{H} and J_{H} regions, and three different constant regions, and also harbors 800 kb of human K locus containing 32 Vκ genes, Jκ segments, and Cκ genes. The antibodies produced in these mice closely resemble that seen in humans in all respects, including gene rearrangement, assembly, and repertoire. The human antibodies are preferentially expressed over endogenous antibodies due to deletion in endogenous segment that prevents gene rearrangement in the murine locus. Such mice may be immunized with an antigen of particular interest.

Sera from such immunized animals may be screened for antibody reactivity against the initial antigen. Lymphocytes may be isolated from lymph nodes or spleen cells and may further be selected for B cells by selecting for CD138-negative and CD19-positive cells. In one aspect, such B cell cultures (BCCs) may be fused to myeloma cells to generate hybridomas as detailed above.

In another aspect, such B cell cultures may be screened further for reactivity against the initial antigen, preferably. Such screening includes ELISA with the target/antigen protein, a competition assay with known antibodies that bind the antigen of interest, and in vitro binding to transiently transfected CHO or other cells that express the target antigen.

The present disclosure relates to compositions and methods for treating human patients with diseases mediated by stimulation of CD40 signaling on CD40-expressing cells. The methods involve treatment with an anti-CD40 antibody of the invention, or an antigen-binding fragment thereof, where administration of the antibody or antigen-binding fragment thereof promotes a positive therapeutic response within the patient undergoing this method of therapy. Anti-CD40 antibodies suitable for use in the methods of the invention specifically bind a human CD40 antigen expressed on the surface of a human cell and are free of significant agonist activity, but exhibit antagonist activity when bound to the CD40 antigen on a human CD40-expressing cell. These anti-CD40 antibodies and antigen-binding fragments thereof are referred to herein as antagonist anti-CD40 antibodies. Such antibodies include, but are not limited to, the fully human monoclonal antibodies CHIR-5.9 and CHIR-12.12 described below and monoclonal antibodies having the binding characteristics of monoclonal antibodies CHIR-5.9 and CHIR-12.12. Those skilled in the art recognize that the antagonist antibodies and antigen-binding fragments of these antibodies disclosed herein include antibodies and antigen-binding fragments thereof that are produced recombinantly using methods well known in the art and described herein below, and include, for example, monoclonal antibodies CHIR-5.9 and CHIR-12.12 that have been recombinantly produced.

Antibodies that have the binding characteristics of monoclonal antibodies CHIR-5.9 and CHIR-12.12 include antibodies that competitively interfere with binding CD40 and/or bind the same epitopes as CHIR-5.9 and CHIR-12.12. One of skill could determine whether an antibody competitively interferes with CHIR-5.9 or CHIR-12.12 using standard methods.

When these antibodies bind CD40 displayed on the surface of human cells, such as human B cells, the antibodies are free of significant agonist activity; in some embodiments, their binding to CD40 displayed on the surface of human cells results in inhibition of proliferation and differentiation of these human cells. Thus, the antagonist anti-CD40 antibodies suitable for use in the methods of the invention include those monoclonal antibodies that can exhibit antagonist activity toward normal and malignant human cells expressing the cell-surface CD40 antigen.

In some embodiments, the anti-CD40 antibodies of the invention exhibit increased anti-tumor activity relative to the chimeric anti-CD20 monoclonal antibody IDEC-C2B8, where anti-tumor activity is assayed with equivalent amounts of these antibodies in a nude mouse xenograft tumor model using human lymphoma cell lines. IDEC-C2B8 (IDEC Pharmaceuticals Corp., San Diego, California; commercially available under the tradename Rituxan®, also referred to as rituximab) is a chimeric anti-CD20 monoclonal antibody containing human IgG1 and kappa constant regions with murine variable regions isolated from a murine anti-CD20 monoclonal antibody, IDEC-2B8 (Reff et al. (1994) Blood 83:435-445). Rituximab® is licensed for treatment of relapsed B cell low-grade or follicular non-Hodgkin's lymphoma (NHL). The discovery of antibodies with superior anti-tumor activity compared to Rituximab® could drastically improve methods of cancer therapy for B cell lymphomas, particularly B cell non-Hodgkin's lymphoma.

Suitable nude mouse xenograft tumor models include those using the human Burkitt's lymphoma cell lines known as Namalwa and Daudi. Preferred embodiments assay anti-tumor activity in a staged nude mouse xenograft tumor model using the Daudi human lymphoma cell line as described herein below in Example 17. A staged nude mouse xenograft tumor model using the Daudi lymphoma cell line is more effective at distinguishing the therapeutic efficacy of a given antibody than is an unstaged model, as in the staged model antibody dosing is initiated only after the tumor has reached a measurable size. In the unstaged model, antibody dosing is initiated generally within about 1 day of tumor inoculation and before a palpable tumor is present. The ability of an antibody to outperform Rituxan® (i.e., to exhibit increased anti-tumor activity) in a staged model is a strong indication that the antibody will be more therapeutically effective than Rituxan®. Moreover, in the Daudi model, anti-CD20, the target for Rituxan® is expressed on the cell surface at a higher level than is CD40.

By "equivalent amount" of the anti-CD40 antibody of the invention and Rituxan® is intended the same mg dose is administered on a per weight basis. Thus, where the anti-CD40 antibody of the invention is dosed at 0.01 mg/kg body weight of the mouse used in the tumor model, Rituxan® is also dosed at 0.01 mg/kg body weight of the mouse. Similarly, where the anti-CD40antibody of the invention is dosed at 0.1, 1, or 10 mg/kg body weight of the mouse used in the tumor model, the Rituxan® is also dosed at 0.1, 1, or 10 mg/kg, respectively, of the body weight of the mouse.

When administered in the nude mouse xenograft tumor model, some antibodies of the invention result in significantly less tumor volume than an equivalent amount of Rituxan®. Thus, for example, the fully human monoclonal antibody CHIR-12.12 exhibits at least a 20% increase in anti-tumor activity relative to that observed with an equivalent dose of Rituxan when assayed in the staged nude mouse xenograft tumor model using the Daudi human lymphoma cell line in the manner described in Examples herein below, and can exhibit as much as a 50% to 60% increase in anti-tumor activity in this assay. This increased anti-tumor activity is reflected in the greater reduction in tumor volume observed with the anti-CD40 antibody of the invention when compared to the equivalent dose of Rituxan®. Thus, for example, depending upon the length of time after tumor inoculation, the monoclonal antibody CHIR-12.12 can exhibit a tumor volume that is about one-third to about one-half that observed for an equivalent dose of Rituxan®.

Another difference in antibody efficacy is to measure *in vitro* the concentration of antibody needed to obtain the maximum lysis of tumor cells *in vitro* in the presence of NK cells. For example, the anti-CD40 antibodies of the invention reach maximum lysis of Daudi cells at an EC50 of less than ½, and preferably ¼, and most preferably, 1/10 the concentration of Rituxan®.

In addition to the monoclonal antibody CHIR-12.12, other anti-CD40 antibodies that would share the characteristics of having significantly greater efficacy than equivalent amounts of Rituxan® in the assays described above include, but are not limited to: (1) the monoclonal antibody produced by the hybridoma cell line 12.12; (2) a monoclonal antibody comprising an amino acid sequence selected from the group consisting of the sequence in SEQ ID NO:2, the sequence in SEQ ID NO:4, the sequence in SEQ ID NO:5, both the sequence in SEQ ID NO:2 and SEQ ID NO:4, and both the sequence in SEQ ID NO:2 and SEQ ID NO:5; (3) a monoclonal antibody having an amino acid sequence encoded by a nucleic acid molecule comprising a nucleotide sequence selected from the group consisting of the nucleotide sequence in SEQ ID NO:1, the nucleotide sequence in SEQ ID NO:3, and both the sequence in SEQ ID NO:1 and SEQ ID NO:3; (4) a monoclonal antibody that binds to an epitope capable of binding the monoclonal antibody produced by the hybridoma cell line 12.12; (5) a monoclonal antibody that binds to an epitope comprising residues 82-87 of the amino acid sequence in SEQ ID NO:10 or SEQ ID NO:12; (6) a monoclonal antibody that competes with the monoclonal antibody CHIR-12.12 in a competitive binding assay; and (7) a monoclonal antibody that is an antigen-binding fragment of the CHIR-12.12 monoclonal antibody or the foregoing monoclonal antibodies in preceding items (1)-(6), where the fragment retains the capability of specifically binding to the human CD40 antigen.

### Antagonist Anti-CD40 Antibodies

The monoclonal antibodies CHIR-5.9 and CHIR-12.12 represent suitable antagonist anti-CD40 antibodies for use in the methods of the present invention. The CHIR-5.9 and CHIR-12.12 antibodies are fully human anti-CD40 monoclonal antibodies of the IgG₁ isotype produced from the hybridoma cell lines 131.2F8.5.9 (referred to herein as the cell line 5.9) and 153.8E2.D10.D6.12.12 (referred to herein as the cell line 12.12). These cell lines were created using splenocytes from immunized xenotypic mice containing the human IgG₁ heavy chain locus and the human κ chain locus (XenoMouse^{®} technology; Abgenix; Fremont, California). The spleen cells were fused with the mouse myeloma SP2/0 cells (Sierra BioSource). The resulting hybridomas were sub-cloned several times to create the stable monoclonal cell lines 5.9 and 12.12. Other antibodies of the invention may be prepared similarly using mice transgenic for human immunoglobulin loci or by other methods known in the art and/or described herein.

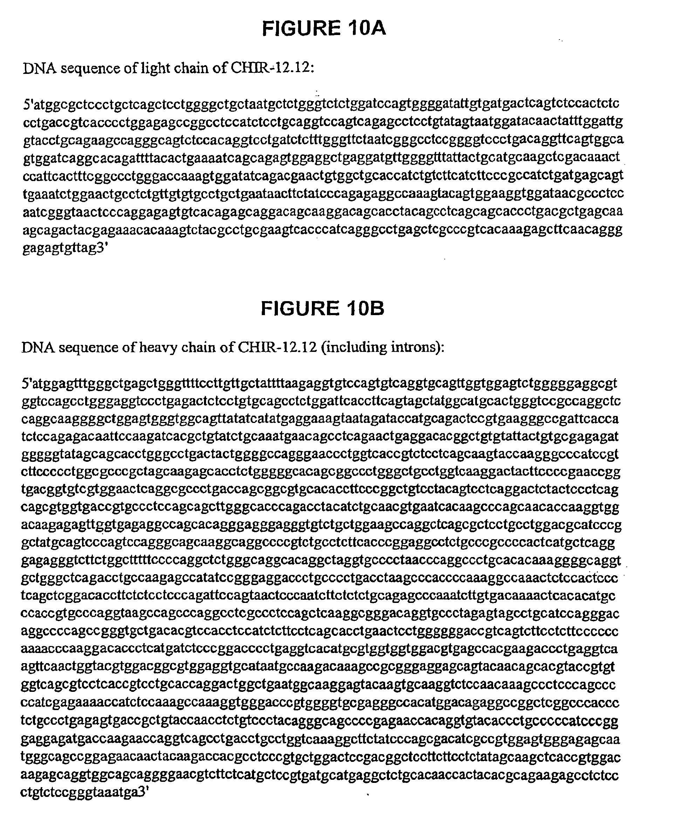

The nucleotide and amino acid sequences of the variable regions of the CHIR-12.12 antibody, and the amino acid sequences of the variable regions of the CHIR-5.9 antibody, are disclosed. More particularly, the amino acid sequences for the leader, variable, and constant regions for the light chain and heavy chain for mAb CHIR-12.12 are set forth in Figures 9A and 9B, respectively. See also SEQ ID NO:2 (complete sequence for the light chain of mAb CHIR-12.12), SEQ ID NO:4 (complete sequence for the heavy chain for mAb CHIR-12.12), and SEQ ID NO:5 (complete sequence for a variant of the heavy chain for mAb CHIR-12.12 set forth in SEQ ID NO:4, where the variant comprises a serine substitution for the alanine residue at position 153 of SEQ ID NO:4). The nucleotide sequences encoding the light chain and heavy chain for mAb CHIR-12.12 are set forth in Figures 11A and 11B, respectively. See also SEQ ID NO:1 (coding sequence for the light chain for mAb CHIR-12.12), and SEQ ID NO:3 (coding sequence for the heavy chain for mAb CHIR-12.12). The amino acid sequences for the leader, variable, and constant regions for the light chain and heavy chain of the CHIR-5.9 mAb are set forth in Figures 10A and 10B, respectively. See also SEQ ID NO:6 (complete sequence for the light chain of mAb CHIR-5.9), SEQ ID NO:7 (complete sequence for the heavy chain of mAb CHIR-5.9), and SEQ ID NO:8 (complete sequence for a variant of the heavy chain of mAb CHIR-5.9 set forth in SEQ ID NO:7, where the variant comprises a serine substitution for the alanine residue at position 158 of SEQ ID NO:7). Further, hybridomas expressing CHIR-5.9 and CHIR-12.12 antibodies have been deposited with the ATCC with a patent deposit designation of PTA-5542 and PTA-5543, respectively.

In addition to antagonist activity, it is preferable that anti-CD40 antibodies of this invention have another mechanism of action against a tumor cell. For example, native CHIR-5.9 and CHIR-12.12 antibodies have ADCC activity. Alternatively, the variable regions of the CHIR-5.9 and CHIR-12.12 antibodies can be expressed on another antibody isotype that has ADCC activity. It is also possible to conjugate native forms, recombinant forms, or antigen-binding fragments of CHIR-5.9 or CHIR-12.12 to a cytotoxin, a therapeutic agent, or a radioactive metal ion or radioisotope, as noted herein below.

The CHIR-5.9 and CHIR-12.12 monoclonal antibodies bind soluble CD40 in ELISA-type assays, prevent the binding of CD40-ligand to cell-surface CD40, and displace the pre-bound CD40-ligand, as determined by flow cytometric assays. Antibodies CHIR-5.9 and CHIR-12.12 compete with each other for binding to CD40 but not with 15B8, the anti-CD40 monoclonal antibody described in WO 02/28904. When tested *in vitro* for effects on proliferation of B cells from normal human subjects, CHIR-5.9 and CHIR-12.12 act as antagonist anti-CD40 antibodies. Furthermore, CHIR-5.9 and CHIR-12.12 do not induce strong proliferation of human lymphocytes from normal subjects. These antibodies are able to kill CD40-expressing target cells by antibody dependent cellular cytotoxicity (ADCC). The binding affinity of CHIR-5.9 for human CD40 is 1.2x10⁻⁸ M and the binding affinity of CHIR-12.12 is 5x10⁻¹⁰M, as determined by the Biacore^{™} assay.

Suitable antagonist anti-CD40 antibodies for use in the methods of the present invention exhibit a strong single-site binding affinity for the CD40 cell-surface antigen. The monoclonal antibodies of the invention exhibit a dissociation equilibrium constant (K_{D}) for CD40 of at least 10⁻⁵ M, at least 3X10⁻⁵ M, preferably at least 10⁻⁶ M to 10⁻⁷ M, more preferably at least 10⁻⁸ M to about 10⁻¹² M, measured using a standard assay such as Biacore™. Biacore analysis is known in the art and details are provided in the "BIAapplications handbook." Methods described in WO 01/27160 can be used to modulate the binding affinity.

By "CD40 antigen," "CD40 cell surface antigen," "CD40 receptor," or "CD40" is intended a transmembrane glycoprotein that belongs to the tumor necrosis factor (TNF) receptor family (see, for example, U.S. Patent Nos. 5,674,492 and 4,708,871; Stamenkovic et al. (1989) EMBO 8:1403; Clark (1990) Tissue Antigens 36:33; Barclay et al. (1997) The Leucocyte Antigen Facts Book (2d ed.; Academic Press, San Diego)). Two isoforms of human CD40, encoded by alternatively spliced transcript variants of this gene, have been identified. The first isoform (also known as the "long isoform" or "isoform 1") is expressed as a 277-amino-acid precursor polypeptide (SEQ ID NO:12 (first reported as GenBank Accession No. CAA43045, and identified as isoform 1 in GenBank Accession No. NP_001241), encoded by SEQ ID NO:11 (see GenBank Accession Nos. X60592 and NM_001250)), which has a signal sequence represented by the first 19 residues. The second isoform (also known as the "short isoform" or "isoform 2") is expressed as a 203-amino-acid precursor polypeptide (SEQ ID NO:10 (GenBank Accession No. NP_690593), encoded by SEQ ID NO:9 (GenBank Accession No. NM_152854)), which also has a signal sequence represented by the first 19 residues. The precursor polypeptides of these two isoforms of human CD40 share in common their first 165 residues (i.e., residues 1-165 of SEQ ID NO:10 and SEQ ID NO:12). The precursor polypeptide of the short isoform (shown in SEQ ID NO:10) is encoded by a transcript variant (SEQ ID NO:9) that lacks a coding segment, which leads to a translation frame shift; the resulting CD40 isoform contains a shorter and distinct C-terminus (residues 166-203 of SEQ ID NO:10) from that contained in the long isoform of CD40 (C-terminus shown in residues 166-277 of SEQ ID NO:12). For purposes of the present invention, the term "CD40 antigen," "CD40 cell surface antigen," "CD40 receptor," or "CD40" encompasses both the short and long isoforms of CD40. The anti-CD40 antibodies of the present invention bind to an epitope of human CD40 that resides at the same location within either the short isoform or long isoform of this cell surface antigen as noted herein below.

The CD40 antigen is displayed on the surface of a variety of cell types, as described elsewhere herein. By "displayed on the surface" and "expressed on the surface" is intended that all or a portion of the CD40 antigen is exposed to the exterior of the cell. The displayed or expressed CD40 antigen may be fully or partially glycosylated.

By "agonist activity" is intended that the substance functions as an agonist. An agonist combines with a receptor on a cell and initiates a reaction or activity that is similar to or the same as that initiated by the receptor's natural ligand. An agonist of CD40 induces any or all of, but not limited to, the following responses: B cell proliferation and differentiation, antibody production, intercellular adhesion, B cell memory generation, isotype switching, up-regulation of cell-surface expression of MHC Class II and CD80/86, and secretion of pro-inflammatory cytokines such as IL-8, IL-12, and TNF. By "antagonist activity" is intended that the substance functions as an antagonist. An antagonist of CD40 prevents or reduces induction of any of the responses induced by binding of the CD40 receptor to an agonist ligand, particularly CD40L. The antagonist may reduce induction of any one or more of the responses to agonist binding by 5%, 10%, 15%, 20%, 25%, 30%, 35%, preferably 40%, 45%, 50%, 55%, 60%, more preferably 70%, 80%, 85%, and most preferably 90%, 95%, 99%, or 100%. Methods for measuring anti-CD40 antibody and CD40-ligand binding specificity and antagonist activity are known to one of skill in the art and include, but are not limited to, standard competitive binding assays, assays for monitoring immunoglobulin secretion by B cells, B cell proliferation assays, Banchereau-Like-B cell proliferation assays, T cell helper assays for antibody production, co-stimulation of B cell proliferation assays, and assays for up-regulation of B cell activation markers. See, for example, such assays disclosed in WO 00/75348 and U.S. Patent No. 6,087,329.

By "significant" agonist activity is intended an agonist activity of at least 30%, 35%, 40%, 45%, 50%, 60%, 70%, 75%, 80%, 85%, 90%, 95%, or 100% greater than the agonist activity induced by a neutral substance or negative control as measured in an assay of a B cell response. Preferably, "significant" agonist activity is an agonist activity that is at least 2-fold greater or at least 3-fold greater than the agonist activity induced by a neutral substance or negative control as measured in an assay of a B cell response. Thus, for example, where the B cell response of interest is B cell proliferation, "significant" agonist activity would be induction of a level of B cell proliferation that is at least 2-fold greater or at least 3-fold greater than the level of B cell proliferation induced by a neutral substance or negative control. In one embodiment, a non-specific immunoglobulin, for example IgG1, that does not bind to CD40 serves as the negative control. A substance "free of significant agonist activity" would exhibit an agonist activity of not more than about 25% greater than the agonist activity induced by a neutral substance or negative control, preferably not more than about 20% greater, 15% greater, 10% greater, 5% greater, 1% greater, 0.5% greater, or even not more than about 0.1% greater than the agonist activity induced by a neutral substance or negative control as measured in an assay of a B cell response. The antagonist anti-CD40 antibodies useful in the methods of the present invention are free of significant agonist activity as noted above when bound to a CD40 antigen on a human cell. In one embodiment of the invention, the antagonist anti-CD40 antibody is free of significant agonist activity in one B cell response. In another embodiment of the invention, the antagonist anti-CD40 antibody is free of significant agonist activity in assays of more than one B cell response (e.g., proliferation and differentiation, or proliferation, differentiation, and antibody production).

As used herein "anti-CD40 antibody" encompasses any antibody that specifically recognizes the CD40 B cell surface antigen, including polyclonal antibodies, monoclonal antibodies, single-chain antibodies, and fragments thereof such as Fab, F(ab')₂, Fᵥ, and other fragments which retain the antigen binding function of the parent anti-CD40 antibody. Of particular interest to the present invention are the antagonist anti-CD40 antibodies disclosed herein that share the binding characteristics of the monoclonal antibodies CHIR-5.9 and CHIR-12.12 described above. Such antibodies include, but are not limited to the following: (1) the monoclonal antibodies produced by the hybridoma cell lines designated 131.2F8.5.9 (referred to herein as the cell line 5.9) and 153.8E2.D10.D6.12.12 (referred to herein as the cell line 12.12), deposited with the ATCC as Patent Deposit No. PTA-5542 and Patent Deposit No. PTA-5543, respectively; (2) a monoclonal antibody comprising an amino acid sequence selected from the group consisting of the sequence shown in SEQ ID NO:2, the sequence shown in SEQ ID NO:4, the sequence shown in SEQ ID NO:5, both the sequences shown in SEQ ID NO:2 and SEQ ID NO:4, and both the sequences shown in SEQ ID NO:2 and SEQ ID NO:5; (3) a monoclonal antibody comprising an amino acid sequence selected from the group consisting of the sequence shown in SEQ ID NO:6, the sequence shown in SEQ ID NO:7, the sequence shown in SEQ ID NO:8, both the sequences shown in SEQ ID NO:6 and SEQ ID NO:7, and both the sequences shown in SEQ ID NO:6 and SEQ ID NO:8; (4) a monoclonal antibody having an amino acid sequence encoded by a nucleic acid molecule comprising a nucleotide sequence selected from the group consisting of the nucleotide sequence shown in SEQ ID NO:1, the nucleotide sequence shown in SEQ ID NO:3, and both the sequences shown in SEQ ID NO:1 and SEQ ID NO:3; (5) a monoclonal antibody that binds to an epitope capable of binding the monoclonal antibody produced by the hybridoma cell line 5.9 or the hybridoma cell line 12.12; (6) a monoclonal antibody that binds to an epitope comprising residues 82-87 of the amino acid sequence shown in SEQ ID NO:10 or SEQ ID NO:12; (7) a monoclonal antibody that competes with the monoclonal antibody CHIR-5.9 or CHIR-12.12 in a competitive binding assay; and (8) a monoclonal antibody that is an antigen-binding fragment of the CHIR-12.12 or CHIR-5.9 monoclonal antibody or the foregoing monoclonal antibodies in preceding items (1)-(7), where the fragment retains the capability of specifically binding to the human CD40 antigen.

### Production of Antagonist Anti-CD40 Antibodies

The antagonist anti-CD40 antibodies disclosed herein and for use in the methods of the present invention can be produced using any antibody production method known to those of skill in the art. Thus, polyclonal sera may be prepared by conventional methods. In general, a solution containing the CD40 antigen is first used to immunize a suitable animal, preferably a mouse, rat, rabbit, or goat. Rabbits or goats are preferred for the preparation of polyclonal sera due to the volume of serum obtainable, and the availability of labeled anti-rabbit and anti-goat antibodies.

Polyclonal sera can be prepared in a transgenic animal, preferably a mouse bearing human immunoglobulin loci. In a preferred embodiment, Sf9 cells expressing CD40 are used as the immunogen. Immunization can also be performed by mixing or emulsifying the antigen-containing solution in saline, preferably in an adjuvant such as Freund's complete adjuvant, and injecting the mixture or emulsion parenterally (generally subcutaneously or intramuscularly). A dose of 50-200 µg/injection is typically sufficient. Immunization is generally boosted 2-6 weeks later with one or more injections of the protein in saline, preferably using Freund's incomplete adjuvant. One may alternatively generate antibodies by *in vitro* immunization using methods known in the art, which for the purposes of this invention is considered equivalent to *in vivo* immunization. Polyclonal antisera are obtained by bleeding the immunized animal into a glass or plastic container, incubating the blood at 25°C for one hour, followed by incubating at 4°C for 2-18 hours. The serum is recovered by centrifugation (e.g., 1,000 x g for 10 minutes). About 20-50 ml per bleed may be obtained from rabbits.

Production of the Sf 9 (*Spodoptera frugiperda*) cells is disclosed in U.S. Patent No. 6,004,552, incorporated herein by reference. Briefly, sequences encoding human CD40 were recombined into a baculovirus using transfer vectors. The plasmids were co-transfected with wild-type baculovirus DNA into Sf 9 cells. Recombinant baculovirus-infected Sf9 cells were identified and clonally purified.

Preferably the antibody is monoclonal in nature. By "monoclonal antibody" is intended an antibody obtained from a population of substantially homogeneous antibodies, i.e., the individual antibodies comprising the population are identical except for possible naturally occurring mutations that may be present in minor amounts. The term is not limited regarding the species or source of the antibody. The term encompasses whole immunoglobulins as well as fragments such as Fab, F(ab')2, Fv, and others which retain the antigen binding function of the antibody. Monoclonal antibodies are highly specific, being directed against a single antigenic site, i.e., the CD40 cell surface antigen in the present invention. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al. (1975) Nature 256:495, or may be made by recombinant DNA methods (see, e.g., U.S. Patent No. 4,816,567). The "monoclonal antibodies" may also be isolated from phage antibody libraries using the techniques described in, for example, Clackson et al. (1991) Nature 352:624-628; Marks et al. (1991) J. Mol. Biol. 222:581-597; and

U.S. Patent No. 5,514,548.

By "epitope" is intended the part of an antigenic molecule to which an antibody is produced and to which the antibody will bind. Epitopes can comprise linear amino acid residues (i.e., residues within the epitope are arranged sequentially one after another in a linear fashion), nonlinear amino acid residues (referred to herein as "nonlinear epitopes"; these epitopes are not arranged sequentially), or both linear and nonlinear amino acid residues.

Monoclonal antibodies can be prepared using the method of Kohler et al. (1975) Nature 256:495-496, or a modification thereof. Typically, a mouse is immunized with a solution containing an antigen. Immunization can be performed by mixing or emulsifying the antigen-containing solution in saline, preferably in an adjuvant such as Freund's complete adjuvant, and injecting the mixture or emulsion parenterally. Any method of immunization known in the art may be used to obtain the monoclonal antibodies of the invention. After immunization of the animal, the spleen (and optionally, several large lymph nodes) are removed and dissociated into single cells. The spleen cells may be screened by applying a cell suspension to a plate or well coated with the antigen of interest. The B cells expressing membrane bound immunoglobulin specific for the antigen bind to the plate and are not rinsed away. Resulting B cells, or all dissociated spleen cells, are then induced to fuse with myeloma cells to form hybridomas, and are cultured in a selective medium. The resulting cells are plated by serial dilution and are assayed for the production of antibodies that specifically bind the antigen of interest (and that do not bind to unrelated antigens). The selected monoclonal antibody (mAb)-secreting hybridomas are then cultured either *in vitro* (e.g., in tissue culture bottles or hollow fiber reactors), or *in vivo* (as ascites in mice).

Where the antagonist anti-CD40 antibodies of the invention are to be prepared using recombinant DNA methods, the DNA encoding the monoclonal antibodies is readily isolated and sequenced using conventional procedures (*e.g*., by using oligonucleotide probes that are capable of binding specifically to genes encoding the heavy and light chains of murine antibodies). The hybridoma cells described herein serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al. (1993) Curr. Opinion in Immunol. 5:256 and Phickthun (1992) Immunol. Revs. 130:151. Alternatively, antibody can be produced in a cell line such as a CHO cell line, as disclosed in U.S. Patent Nos. 5,545,403; 5,545,405; and 5,998,144. Briefly the cell line is transfected with vectors capable of expressing a light chain and a heavy chain, respectively. By transfecting the two proteins on separate vectors, chimeric antibodies can be produced. Another advantage is the correct glycosylation of the antibody.

In some embodiments, the antagonist anti-CD40 antibody, for example, the CHIR-12.12 or CHIR-5.9 antibody, or antigen-binding fragment thereof is produced in CHO cells using the GS gene expression system (Lonza Biologics, Portsmouth, New Hampshire), which uses glutamine synthetase as a marker. See, also U.S. Patent Nos. 5,122,464; 5,591,639; 5,658,759; 5,770,359; 5,827,739; 5,879,936; 5,891,693; and 5,981,216.

Monoclonal antibodies to CD40 are known in the art. See, for example, the sections dedicated to B-cell antigen in McMichael, ed. (1987; 1989) Leukocyte Typing III and IV (Oxford University Press, New York); U.S. Patent Nos. 5,674,492; 5,874,082; 5,677,165; 6,056,959; WO 00/63395; International Publication Nos. WO 02/28905 and WO 02/28904; Gordon et al. (1988) J. Immunol. 140:1425; Valle et al. (1989) Eur. J. Immunol. 19:1463; Clark et al. (1986) PNAS 83:4494; Paulie et al. (1989) J. Immunol. 142:590; Gordon et al. (1987) Eur. J. Immunol. 17:1535; Jabara et al. (1990) J. Exp. Med. 172:1861; Zhang et al. (1991) J. Immunol. 146:1836; Gascan et al. (1991) J. Immunol. 147:8; Banchereau et al. (1991) Clin. Immunol. Spectrum 3:8; and Banchereau et al. (1991) Science 251:70. Of particular interest to the present invention are the antagonist anti-CD40 antibodies disclosed herein that share the binding characteristics of the monoclonal antibodies CHIR-5.9 and CHIR-12.12 described above.

The term "CD40-antigen epitope" as used herein refers to a molecule that is capable of immunoreactivity with the anti-CD40 monoclonal antibodies of this invention, excluding the CD40 antigen itself. CD40-antigen epitopes may comprise proteins, protein fragments, peptides, carbohydrates, lipids, and other molecules, but for the purposes of the present invention are most commonly proteins, short oligopeptides, oligopeptide mimics (i e, organic compounds which mimic the antibody binding properties of the CD40 antigen), or combinations thereof Suitable oligopeptide mimics are described, inter alia, in PCT application US 91/04282.

Additionally, the term "anti-CD40 antibody" as used herein encompasses chimeric anti-CD40 antibodies; such chimeric anti-CD40 antibodies for use in the methods of the invention have the binding characteristics of the CHIR-5.9 and CHIR-12.12 monoclonal antibodies described herein. By "chimeric" antibodies is intended antibodies that are most preferably derived using recombinant deoxyribonucleic acid techniques and which comprise both human (including immunologically "related" species, e.g., chimpanzee) and non-human components. Thus, the constant region of the chimeric antibody is most preferably substantially identical to the constant region of a natural human antibody; the variable region of the chimeric antibody is most preferably derived from a non-human source and has the desired antigenic specificity to the CD40 cell-surface antigen. The non-human source can be any vertebrate source that can be used to generate antibodies to a human CD40 cell-surface antigen or material comprising a human CD40 cell-surface antigen. Such non-human sources include, but are not limited to, rodents (e.g., rabbit, rat, mouse, etc.; see, for example, U.S. Patent No. 4,816,567) and non-human primates (e.g., Old World Monkey, Ape, etc.; see, for example, U.S. Patent Nos. 5,750,105 and 5,756,096). As used herein, the phrase "immunologically active" when used in reference to chimeric anti-CD40 antibodies means a chimeric antibody that binds human CD40.

Chimeric and humanized anti-CD40 antibodies are also encompassed by the term anti-CD40 antibody as used herein. Chimeric antibodies comprise segments of antibodies derived from different species. Rituxan® is an example of a chimeric antibody with a murine variable region and a human constant region.

By "humanized" is intended forms of anti-CD40 antibodies that contain minimal sequence derived from non-human immunoglobulin sequences. For the most part, humanized antibodies are human immunoglobulins (recipient antibody) in which residues from a hypervariable region (also known as complementarity determining region or CDR) of the recipient are replaced by residues from a hypervariable region of a non-human species (donor antibody) such as mouse, rat, rabbit, or nonhuman primate having the desired specificity, affinity, and capacity. The phrase "complementarity determining region" refers to amino acid sequences which together define the binding affinity and specificity of the natural Fv region of a native immunoglobulin binding site. See, e.g., Chothia et al (1987) J. Mol. Biol. 196:901-917; Kabat et al (1991) U. S. Dept. of Health and Human Services, NIH Publication No. 91-3242). The phrase "constant region" refers to the portion of the antibody molecule that confers effector functions. In previous work directed towards producing non-immunogenic antibodies for use in therapy of human disease, mouse constant regions were substituted by human constant regions. The constant regions of the subject humanized antibodies were derived from human immunoglobulins. However, these humanized antibodies still elicited an unwanted and potentially dangerous immune response in humans and there was a loss of affinity. Humanized anti-CD40 antibodies for use in the methods of the present invention have binding characteristics similar to those exhibited by the CHIR-5.9 and CHIR-12.12 monoclonal antibodies described herein.

Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting rodent or mutant rodent CDRs or CDR sequences for the corresponding sequences of a human antibody. See also U.S. Patent Nos. 5,225,539; 5,585,089; 5,693,761; 5,693,762; 5,859,205. In some instances, residues within the framework regions of one or more variable regions of the human immunoglobulin are replaced by corresponding non-human residues (see, for example, U.S. Patent Nos. 5,585,089; 5,693,761; 5,693,762; and 6,180,370). Furthermore, humanized antibodies may comprise residues that are not found in the recipient antibody or in the donor antibody. These modifications are made to further refine antibody performance (e.g., to obtain desired affinity). In general, the humanized antibody will comprise substantially all of at least one, and typically two, variable domains, in which all or substantially all of the hypervariable regions correspond to those of a non-human immunoglobulin and all or substantially all of the framework regions are those of a human immunoglobulin sequence. The humanized antibody optionally also will comprise at least a portion of an immunoglobulin constant region (Fc), typically that of a human immunoglobulin. For further details see Jones et al. (1986) Nature 331:522-525; Riechmann et al. (1988) Nature 332:323-329; and Presta (1992) Curr. Op. Struct. Biol. 2:593-596. Accordingly, such "humanized" antibodies may include antibodies wherein substantially less than an intact human variable domain has been substituted by the corresponding sequence from a non-human species. In practice, humanized antibodies are typically human antibodies in which some CDR residues and possibly some framework residues are substituted by residues from analogous sites in rodent antibodies. See, for example, U.S. Patent Nos. 5,225,539; 5,585,089; 5,693,761; 5,693,762; 5,859,205. See also U.S. Patent No. 6,180,370, and International Publication No. WO 01/27160, where humanized antibodies and techniques for producing humanized antibodies having improved affinity for a predetermined antigen are disclosed.

Also encompassed by the term anti-CD40 antibodies are xenogeneic or modified anti-CD40 antibodies produced in a non-human mammalian host, more particularly a transgenic mouse, characterized by inactivated endogenous immunoglobulin (Ig) loci. In such transgenic animals, competent endogenous genes for the expression of light and heavy subunits of host immunoglobulins are rendered non-functional and substituted with the analogous human immunoglobulin loci. These transgenic animals produce human antibodies in the substantial absence of light or heavy host immunoglobulin subunits. See, for example, U.S. Patent Nos. 5,877,397 and 5,939,598.

Preferably, fully human antibodies to CD40 are obtained by immunizing transgenic mice. One such mouse is obtained using XenoMouse^{®} technology (Abgenix; Fremont, California), and is disclosed in U.S. Patent Nos. 6,075,181, 6,091,001, and 6,114,598. To produce the antibodies disclosed herein, mice transgenic for the human Ig G₁ heavy chain locus and the human κ light chain locus were immunized with Sf 9 cells expressing human CD40. Mice can also be transgenic for other isotypes. Fully human antibodies useful in the methods of the present invention are characterized by binding properties similar to those exhibited by the CHIR-5.9 and CHIR-12.12 monoclonal antibodies disclosed herein.

Fragments of the anti-CD40 antibodies are suitable for use in the methods of the invention so long as they retain the desired affinity of the full-length antibody. Thus, a fragment of an anti-CD40 antibody will retain the ability to bind to the CD40 B cell surface antigen. Such fragments are characterized by properties similar to the corresponding full-length antagonist anti-CD40 antibody, that is, the fragments will specifically bind a human CD40 antigen expressed on the surface of a human cell, and are free of significant agonist activity but exhibit antagonist activity when bound to a CD40 antigen on a human CD40-expressing cell. Such fragments are referred to herein as "antigen-binding" fragments.

Suitable antigen-binding fragments of an antibody comprise a portion of a full-length antibody, generally the antigen-binding or variable region thereof. Examples of antibody fragments include, but are not limited to, Fab, F(ab')₂, and Fv fragments and single-chain antibody molecules. By "Fab" is intended a monovalent antigen-binding fragment of an immunoglobulin that is composed of the light chain and part of the heavy chain. By F(ab')₂ is intended a bivalent antigen-binding fragment of an immunoglobulin that contains both light chains and part of both heavy chains. By "single-chain Fv" or "sFv" antibody fragments is intended fragments comprising the V_{H} and V_{L} domains of an antibody, wherein these domains are present in a single polypeptide chain. See, for example, U.S. Patent Nos. 4,946,778, 5,260,203, 5,455,030, and 5,856,456. Generally, the Fv polypeptide further comprises a polypeptide linker between the V_{H} and V_{L} domains that enables the sFv to form the desired structure for antigen binding. For a review of sFv see Pluckthun (1994) in The Pharmacology of Monoclonal Antibodies, Vol. 113, ed. Rosenburg and Moore (Springer-Verlag, New York), pp. 269-315. Antigen-binding fragments of the antagonist anti-CD40 antibodies disclosed herein can also be conjugated to a cytotoxin to effect killing of the target cancer cells, as described herein below.

Antibodies or antibody fragments can be isolated from antibody phage libraries generated using the techniques described in, for example, McCafferty et al. (1990) Nature 348:552-554 (1990) and U.S. Patent No. 5,514,548. Clackson et al. (1991) Nature 352:624-628 and Marks et al. (1991) J. Mol. Biol. 222:581-597 describe the isolation of murine and human antibodies, respectively, using phage libraries. Subsequent publications describe the production of high affinity (nM range) human antibodies by chain shuffling (Marks et al. (1992) Bio/Technology 10:779-783), as well as combinatorial infection and *in vivo* recombination as a strategy for constructing very large phage libraries (Waterhouse et al. (1993) Nucleic. Acids Res. 21:2265-2266). Thus, these techniques are viable alternatives to traditional monoclonal antibody hybridoma techniques for isolation of monoclonal antibodies.

Various techniques have been developed for the production of antibody fragments. Traditionally, these fragments were derived via proteolytic digestion of intact antibodies (see, e.g., Morimoto et al. (1992) Journal of Biochemical and Biophysical Methods 24:107-117 (1992) and Brennan et al. (1985) Science 229:81). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al. (1992) Bio/Technology 10:163-167). According to another approach, F(ab')₂ fragments can be isolated directly from recombinant host cell culture. Other techniques for the production of antibody fragments will be apparent to the skilled practitioner.

Antagonist anti-CD40 antibodies useful in the methods of the present invention include the CHIR-5.9 and CHIR-12-12 monoclonal antibodies disclosed herein as well as antibodies differing from this antibody but retaining the CDRs; and antibodies with one or more amino acid addition(s), deletion(s), or substitution(s), wherein the antagonist activity is measured by inhibition of B-cell proliferation and/or differentiation. The invention also encompasses de-immunized antagonist anti-CD40 antibodies, which can be produced as described in, for example, International Publication Nos. WO 98/52976 and WO 0034317. In this manner, residues within the antagonist anti-CD40 antibodies of the invention are modified so as to render the antibodies non- or less immunogenic to humans while retaining their antagonist activity toward human CD40-expressing cells, wherein such activity is measured by assays noted elsewhere herein. Also included within the scope of the claims are fusion proteins comprising an antagonist anti-CD40 antibody of the invention, or a fragment thereof, which fusion proteins can be synthesized or expressed from corresponding polynucleotide vectors, as is known in the art. Such fusion proteins are described with reference to conjugation of antibodies as noted below.

The antibodies of the present invention can have sequence variations produced using methods described in, for example, Patent Publication Nos. EP 0 983 303 A1, WO 00/34317, and WO 98/52976. For example, it has been shown that sequences within the CDR can cause an antibody to bind to MHC Class II and trigger an unwanted helper T-cell response. A conservative substitution can allow the antibody to retain binding activity yet lose its ability to trigger an unwanted T-cell response. Any such conservative or non-conservative substitutions can be made using art-recognized methods, such as those noted elsewhere herein, and the resulting antibodies will fall within the scope of the invention. The variant antibodies can be routinely tested for antagonist activity, affinity, and specificity using methods described herein.

An antibody produced by any of the methods described above, or any other method not disclosed herein, will fall within the scope of this disclosure if it possesses at least one of the following biological activities: inhibition of immunoglobulin secretion by normal human peripheral B cells stimulated by T cells; inhibition of survival and/or proliferation of normal human peripheral B cells stimulated by Jurkat T cells; inhibition of survival and/or proliferation of normal human peripheral B cells stimulated by CD40L-expressing cells or soluble CD40 ligand (sCD40L); inhibition of "survival" anti-apoptotic intracellular signals in any cell stimulated by sCD40L or solid-phase CD40L; inhibition of CD40 signal transduction in any cell upon ligation with sCD40L or solid-phase CD40L; and inhibition of proliferation of human malignant B cells as noted below. These assays can be performed as described in the Examples herein. See also the assays described in Schultze et al. (1998) Proc. Natl. Acad. Sci. USA 92:8200-8204; Denton et al. (1998) Pediatr. Transplant. 2:6-15; Evans et al. (2000) J. Immunol. 164:688-697; Noelle (1998) Agents Actions Suppl. 49:17-22; Lederman et al. (1996) Curr. Opin. Hematol. 3:77-86; Coligan et al. (1991) Current Protocols in Immunology 13:12; Kwekkeboom et al. (1993) Immunology 79:439-444; and U.S. Patent Nos. 5,674,492 and 5,847,082.

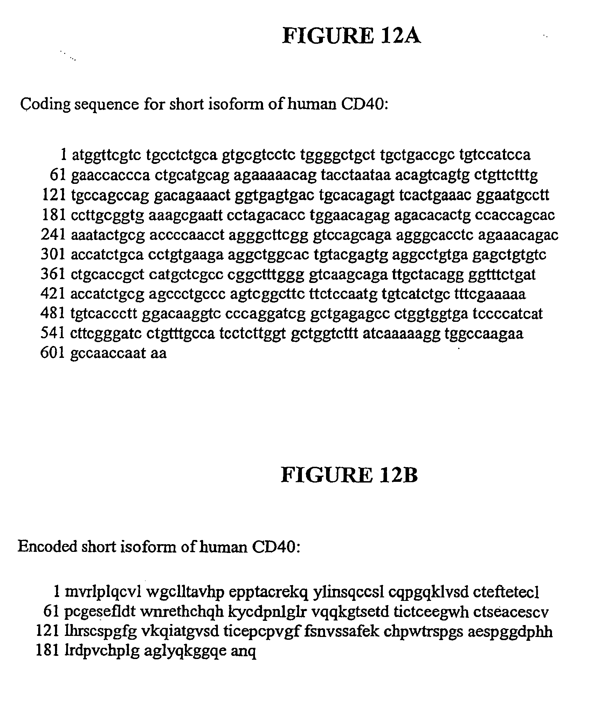

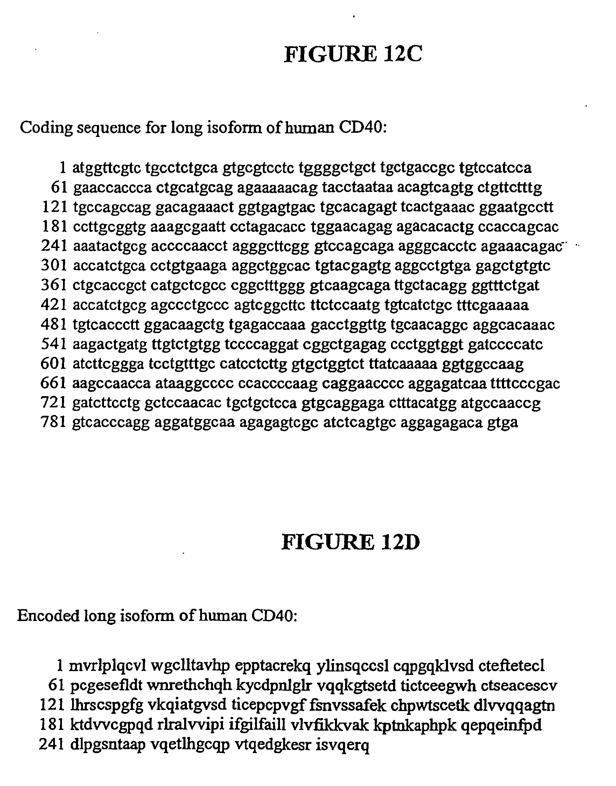

A representative assay to detect antagonist anti-CD40 antibodies specific to the CD40-antigen epitopes identified herein is a "competitive binding assay." Competitive binding assays are serological assays in which unknowns are detected and quantitated by their ability to inhibit the binding of a labeled known ligand to its specific antibody. This is also referred to as a competitive inhibition assay. In a representative competitive binding assay, labeled CD40 polypeptide is precipitated by candidate antibodies in a sample, for example, in combination with monoclonal antibodies raised against one or more epitopes of the monoclonal antibodies of the invention. Anti-CD40 antibodies that specifically react with an epitope of interest can be identified by screening a series of antibodies prepared against a CD40 protein or fragment of the protein comprising the particular epitope of the CD40 protein of interest. For example, for human CD40, epitopes of interest include epitopes comprising linear and/or nonlinear amino acid residues of the short isoform of human CD40 (see GenBank Accession No. NP_690593) set forth in Figure 12B (SEQ ID NO:10), encoded by the sequence set forth in Figure 12A (SEQ ID NO:9; see also GenBank Accession No. NM_152854), or of the long isoform of human CD40 (see GenBank Accession Nos. CAA43045 and NP_001241) set forth in Figure 12D (SEQ ID NO:12), encoded by the sequence set forth in Figure 12C (SEQ ID NO:11; see GenBank Accession Nos. X60592 and NM_001250). Alternatively, competitive binding assays with previously identified suitable antagonist anti-CD40 antibodies could be used to select monoclonal antibodies comparable to the previously identified antibodies.

Antibodies employed in such immunoassays may be labeled or unlabeled. Unlabeled antibodies may be employed in agglutination; labeled antibodies may be employed in a wide variety of assays, employing a wide variety of labels. Detection of the formation of an antibody-antigen complex between an anti-CD40 antibody and an epitope of interest can be facilitated by attaching a detectable substance to the antibody. Suitable detection means include the use of labels such as radionuclides, enzymes, coenzymes, fluorescers, chemiluminescers, chromogens, enzyme substrates or co-factors, enzyme inhibitors, prosthetic group complexes, free radicals, particles, dyes, and the like. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material is luminol; examples of bioluminescent materials include luciferase, luciferin, and aequorin; and examples of suitable radioactive material include ¹¹⁵I, ¹³¹I, ³⁵S, or ³H. Such labeled reagents may be used in a variety of well-known assays, such as radioimmunoassays, enzyme immunoassays, e.g., ELISA, fluorescent immunoassays, and the like. See for example, U.S. Patent Nos. 3,766,162; 3,791,932; 3,817,837; and 4,233,402.

Any of the previously described antagonist anti-CD40 antibodies or antibody fragments thereof may be conjugated prior to use in the methods of the present invention. Methods for producing conjugated antibodies are known in the art. Thus, the anti-CD40 antibody may be labeled using an indirect labeling or indirect labeling approach. By "indirect labeling" or "indirect labeling approach" is intended that a chelating agent is covalently attached to an antibody and at least one radionuclide is inserted into the chelating agent. See, for example, the chelating agents and radionuclides described in Srivagtava and Mease (1991) Nucl. Med. Bio. 18:589-603. Suitable labels include fluorophores, chromophores, radioactive atoms (particularly ³²P and ¹²⁵I), electron-dense reagents, enzymes, and ligands having specific binding partners. Enzymes are typically detected by their activity. For example, horseradish peroxidase is usually detected by its ability to convert 3,3', 5,5'-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefore. Other specific binding partners include biotin and avidin or streptavidin, Ig G and protein A, and the numerous receptor-ligand couples known in the art. It should be understood that the above description is not meant to categorize the various labels into distinct classes, as the same label may serve in several different modes. For example, ¹²⁵I may serve as a radioactive label or as an electron-dense reagent. HRP may serve as enzyme or as antigen for a mAb. Further, one may combine various labels for desired effect. For example, mAbs and avidin also require labels in the practice of this invention: thus, one might label a mAb with biotin, and detect its presence with avidin labeled with ¹²⁵I, or with an anti-biotin mAb labeled with HRP. Other permutations and possibilities will be readily apparent to those of ordinary skill in the art, and are considered as equivalents within the scope of the instant invention. ,

Alternatively, the anti-CD40 antibody may be labeled using "direct labeling" or a "direct labeling approach," where a radionuclide is covalently attached directly to an antibody (typically via an amino acid residue). Preferred radionuclides are provided in Srivagtava and Mease (1991) *supra.* The indirect labeling approach is particularly preferred. See also, for example, International Publication Nos. WO 00/52031 and WO 00/52473, where a linker is used to attach a radioactive label to antibodies; and the labeled forms of anti-CD40 antibodies described in U.S. Patent No. 6,015,542.

Further, an antibody (or fragment thereof) may be conjugated to a therapeutic moiety such as a cytotoxin, a therapeutic agent, or a radioactive metal ion or radioisotope. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). Radioisotopes include, but are not limited to, I-131, I-123,1-125, Y-90, Re-188, Re-186, At-211, Cu-67, Bi-212, Bi-213, Pd-109, Tc-99, In-111, and the like. The conjugates of the invention can be used for modifying a given biological response; the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, interferon-alpha, interferon-beta, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known. See, for example, Arnon et al. (1985) "Monoclonal Antibodies for Immunotargeting of Drugs in Cancer Therapy," in Monoclonal Antibodies and Cancer Therapy, ed. Reisfeld et al. (Alan R. Liss, Inc.), pp. 243-256; ed. Hellstrom et al. (1987) "Antibodies for Drug Delivery," in Controlled Drug Delivery, ed. Robinson et al. (2d ed; Marcel Dekker, Inc.), pp. 623-653; Thorpe (1985) "Antibody Carriers of Cytotoxic Agents in Cancer Therapy: A Review," in Monoclonal Antibodies '84: Biological and Clinical Applications, ed. Pinchera et al. pp. 475-506 (Editrice Kurtis, Milano, Italy, 1985); "Analysis, Results, and Future Prospective of the Therapeutic Use of Radiolabeled Antibody in Cancer Therapy," in Monoclonal Antibodies for Cancer Detection and Therapy, ed. Baldwin et al. (Academic Press, New York, 1985), pp. 303-316; and Thorpe et al. (1982) Immunol. Rev. 62:119-158.

Alternatively, an antibody can be conjugated to a second antibody to form an antibody heteroconjugate as described in U.S. Patent No. 4,676,980. In addition, linkers may be used between the labels and the antibodies of the invention (see U.S. Patent No. 4,831,175). Antibodies or, antigen-binding fragments thereof may be directly labeled with radioactive iodine, indium, yttrium, or other radioactive particle known in the art (U.S. Patent No. 5,595,721). Treatment may consist of a combination of treatment with conjugated and nonconjugated antibodies administered simultaneously or subsequently (WO 00/52031 and WO 00/52473).

### Variants of Antagonist Anti-CD40 Antibodies

Suitable biologically active variants of the antagonist anti-CD40 antibodies can be used in the methods of the present invention. Such variants will retain the desired binding properties of the parent antagonist anti-CD40 antibody. Methods for making antibody variants are generally available in the art.

For example, amino acid sequence variants of an antagonist anti-CD40 antibody, for example, the CHIR-5.9 or CHIR-12.12 monoclonal antibody described herein, can be prepared by mutations in the cloned DNA sequence encoding the antibody of interest. Methods for mutagenesis and nucleotide sequence alterations are well known in the art. See, for example, Walker and Gaastra, eds. (1983) Techniques in Molecular Biology (MacMillan Publishing Company, New York); Kunkel (1985) Proc. Natl. Acad Sci. USA 82:488-492; Kunkel et al. (1987) Methods Enzymol. 154:367-382; Sambrook et al. (1989) Molecular Cloning: A Laboratory Manual (Cold Spring Harbor, New York); U.S. Patent No. 4,873,192; and the references cited therein. Guidance as to appropriate amino acid substitutions that do not affect biological activity of the polypeptide of interest may be found in the model of Dayhoff et al. (1978) in Atlas of Protein Sequence and Structure (Natl. Biomed. Res. Found., Washington, D.C.). Conservative substitutions, such as exchanging one amino acid with another having similar properties, may be preferred. Examples of conservative substitutions include, but are not limited to, Gly⇔Ala, Val⇔Ile⇔Leu, Asp⇔Glu, Lys⇔Arg, Asn⇔Gln, and Pher⇔Trp⇔Tyr.

In constructing variants of the antagonist anti-CD40 antibody polypeptide of interest, modifications are made such that variants continue to possess the desired activity, i.e., similar binding affinity and are capable of specifically binding to a human CD40 antigen expressed on the surface of a human cell, and being free of significant agonist activity but exhibiting antagonist activity when bound to a CD40 antigen on a human CD40-expressing cell. Obviously, any mutations made in the DNA encoding the variant polypeptide must not place the sequence out of reading frame and preferably will not create complementary regions that could produce secondary mRNA structure. See EP Patent Application Publication No. 75,444.

In addition, the constant region of an antagonist anti-CD40 antibody can be mutated to alter effector function in a number of ways. For example, see U.S. Patent No. 6,737,056B1 and U.S. Patent Application Publication No. 2004/0132101A1, which disclose Fc mutations that optimize antibody binding to Fc receptors.

Preferably, variants of a reference antagonist anti-CD40 antibody have amino acid sequences that have at least 70% or 75% sequence identity, preferably at least 80% or 85% sequence identity, more preferably at least 90%, 91%, 92%, 93%, 94% or 95% sequence identity to the amino acid sequence for the reference antagonist anti-CD40 antibody molecule, for example, the CHIR-5.9 or CHIR-12.12 monoclonal antibody described herein, or to a shorter portion of the reference antibody molecule. More preferably, the molecules share at least 96%, 97%, 98% or 99% sequence identity. For purposes of the present invention, percent sequence identity is determined using the Smith-Waterman homology search algorithm using an affine gap search with a gap open penalty of 12 and a gap extension penalty of 2, BLOSUM matrix of 62. The Smith-Waterman homology search algorithm is taught in Smith and Waterman (1981) Adv. Appl. Math. 2:482-489. A variant may, for example, differ from the reference antagonist anti-CD40 antibody by as few as 1 to 15 amino acid residues, as few as 1 to 10 amino acid residues, such as 6-10, as few as 5, as few as 4, 3, 2, or even 1 amino acid residue.

With respect to optimal alignment of two amino acid sequences, the contiguous segment of the variant amino acid sequence may have additional amino acid residues or deleted amino acid residues with respect to the reference amino acid sequence. The contiguous segment used for comparison to the reference amino acid sequence will include at least 20 contiguous amino acid residues, and may be 30, 40, 50, or more amino acid residues. Corrections for sequence identity associated with conservative residue substitutions or gaps can be made (see Smith-Waterman homology search algorithm).

The precise chemical structure of a polypeptide capable of specifically binding CD40 and retaining antagonist activity, particularly when bound to CD40 antigen on malignant B cells, depends on a number of factors. As ionizable amino and carboxyl groups are present in the molecule, a particular polypeptide may be obtained as an acidic or basic salt, or in neutral form. All such preparations that retain their biological activity when placed in suitable environmental conditions are included in the definition of antagonist anti-CD40 antibodies as used herein. Further, the primary amino acid sequence of the polypeptide may be augmented by derivatization using sugar moieties (glycosylation) or by other supplementary molecules such as lipids, phosphate, acetyl groups and the like. It may also be augmented by conjugation with saccharides. Certain aspects of such augmentation are accomplished through post-translational processing systems of the producing host; other such modifications may be introduced *in vitro.* In any event, such modifications are included in the definition of an anti-CD40 antibody used herein so long as the antagonist properties of the anti-CD40 antibody are not destroyed. It is expected that such modifications may quantitatively or qualitatively affect the activity, either by enhancing or diminishing the activity of the polypeptide, in the various assays. Further, individual amino acid residues in the chain may be modified by oxidation, reduction, or other derivatization, and the polypeptide may be cleaved to obtain fragments that retain activity. Such alterations that do not destroy antagonist activity do not remove the polypeptide sequence from the definition of anti-CD40 antibodies of interest as used herein.

The art provides substantial guidance regarding the preparation and use of polypeptide variants. In preparing the anti-CD40 antibody variants, one of skill in the art can readily determine which modifications to the native protein nucleotide or amino acid sequence will result in a variant that is suitable for use as a therapeutically active component of a pharmaceutical composition used in the methods disclosed herein

### Methods of Therapy Using the Antagonist Anti-CD40 Antibodies

Methods disclosed herein are directed to the use of antagonist anti-CD40 antibodies to treat patients having a disease mediated by stimulation of CD40 signaling on CD40-expressing cells. By "CD40-expressing cell" is intended normal and malignant B cells expressing CD40 antigen. Methods for detecting CD40 expression in cells are well known in the art and include, but are not limited to, PCR techniques, immunohistochemistry, flow cytometry, Western blot, ELISA, and the like. By "malignant" B cell is intended any neoplastic B cell, including but not limited to B cells derived from lymphomas including low-, intermediate-, and high-grade B cell lymphomas, immunoblastic lymphomas, non-Hodgkin's lymphomas, Hodgkin's disease, Epstein-Barr Virus (EBV) induced lymphomas, and AIDS-related lymphomas, as well as B cell acute lymphoblastic leukemias, myelomas, chronic lymphocytic leukemias, acute myeloblastic leukemias, and the like.

"Treatment" is herein defined as the application or administration of an antagonist anti-CD40 antibody or antigen-binding fragment thereof to a patient, or application or administration of an antagonist anti-CD40 antibody or fragment thereof to an isolated tissue or cell line from a patient, where the patient has a disease, a symptom of a disease, or a predisposition toward a disease, where the purpose is to cure, heal, alleviate, relieve, alter, remedy, ameliorate, improve, or affect the disease, the symptoms of the disease, or the predisposition toward the disease. By "treatment" is also intended the application or administration of a pharmaceutical composition comprising the antagonist anti-CD40 antibodies or fragments thereof to a patient, or application or administration of a pharmaceutical composition comprising the anti-CD40 antibodies or fragments thereof to an isolated tissue or cell line from a patient, who has a disease, a symptom of a disease, or a predisposition toward a disease, where the purpose is to cure, heal, alleviate, relieve, alter, remedy, ameliorate, improve, or affect the disease, the symptoms of the disease, or the predisposition toward the disease.

By "anti-tumor activity" is intended a reduction in the rate of malignant CD40-expressing cell proliferation or accumulation, and hence a decline in growth rate of an existing tumor or in a tumor that arises during therapy, and/or destruction of existing neoplastic (tumor) cells or newly formed neoplastic cells, and hence a decrease in the overall size of a tumor during therapy. Therapy with at least one anti-CD40 antibody (or antigen-binding fragment thereof) causes a physiological response that is beneficial with respect to treatment of disease states associated with stimulation of CD40 signaling on CD40-expressing cells in a human.

The methods disclosed herein find use in the treatment of non-Hodgkin's lymphomas related to abnormal, uncontrollable B cell proliferation or accumulation. For purposes of the present disclosure, such lymphomas will be referred to according to the *Working Formulation* classification scheme, that is those B cell lymphomas categorized as low grade, intermediate grade, and high grade (see "The Non-Hodgkin's Lymphoma Pathologic Classification Project," Cancer 49(1982):2112-2135). Thus, low-grade B cell lymphomas include small lymphocytic, follicular small-cleaved cell, and follicular mixed small-cleaved and large cell lymphomas; intermediate-grade lymphomas include follicular large cell, diffuse small cleaved cell, diffuse mixed small and large cell, and diffuse large cell lymphomas; and high-grade lymphomas include large cell immunoblastic, lymphoblastic, and small non-cleaved cell lymphomas of the Burkitt's and non-Burkitt's type.

It is recognized that the methods disclosed herein are useful in the therapeutic treatment of B cell lymphomas that are classified according to the Revised European and American Lymphoma Classification (REAL) system. Such B cell lymphomas include, but are not limited to, lymphomas classified as precursor B cell neoplasms, such as B lymphoblastic leukemia/lymphoma; peripheral B cell neoplasms, including B cell chronic lymphocytic leukemia/small lymphocytic lymphoma, lymphoplasmacytoid lymphoma/immunocytoma, mantle cell lymphoma (MCL), follicle center lymphoma (follicular) (including diffuse small cell, diffuse mixed small and large cell, and diffuse large cell lymphomas), marginal zone B cell lymphoma (including extranodal, nodal, and splenic types), hairy cell leukemia, plasmacytoma/ myeloma, diffuse large cell B cell lymphoma of the subtype primary mediastinal (thymic), Burkitt's lymphoma, and Burkitt's like high grade B cell lymphoma; acute leukemias; acute lymphocytic leukemias; myeloblastic leukemias; acute myelocytic leukemias; promyelocytic leukemia; myelomonocytic leukemia; monocytic leukemia; erythroleukemia; granulocytic leukemia (chronic myelocytic leukemia); chronic lymphocytic leukemia; polycythemia vera; multiple myeloma; Waldenstrom's macroglobulinemia; heavy chain disease; and unclassifiable low-grade or high-grade B cell lymphomas.

It is recognized that the methods disclosed herein may be useful in preventing further tumor outgrowths arising during therapy. The methods disclosed herein are particularly useful in the treatment of subjects having low-grade B cell lymphomas, particularly those subjects having relapses following standard chemotherapy. Low-grade B cell lymphomas are more indolent than the intermediate- and high-grade B cell lymphomas and are characterized by a relapsing/remitting course. Thus, treatment of these lymphomas is improved using the methods disclosed herein, as relapse episodes are reduced in number and severity.

The antagonist anti-CD40 antibodies described herein may also find use in the treatment of inflammatory diseases and deficiencies or disorders of the immune system including, but not limited to, systemic lupus erythematosus, psoriasis, scleroderma, CREST syndrome, inflammatory myositis, Sjogren's syndrome, mixed connective tissue disease, rheumatoid arthritis, multiple sclerosis, inflammatory bowel disease, acute respiratory distress syndrome, pulmonary inflammation, idiopathic pulmonary fibrosis, osteoporosis, delayed type hypersensitivity, asthma, primary biliary cirrhosis, and idiopathic thrombocytopenic purpura.

In accordance with the methods disclosed herein, at least one antagonist anti-CD40 antibody (or antigen-binding fragment thereof) as defined elsewhere herein is used to promote a positive therapeutic response with respect to a malignant human B cell. By "positive therapeutic response" with respect to cancer treatment is intended an improvement in the disease in association with the anti-tumor activity of these antibodies or fragments thereof, and/or an improvement in the symptoms associated with the disease. That is, an anti-proliferative effect, the prevention of further tumor outgrowths, a reduction in tumor size, a reduction in the number of cancer cells, and/or a decrease in one or more symptoms mediated by stimulation of CD40-expressing cells can be observed. Thus, for example, an improvement in the disease may be characterized as a complete response. By "complete response" is intended an absence of clinically detectable disease with normalization of any previously abnormal radiographic studies, bone marrow, and cerebrospinal fluid (CSF). Such a response must persist for at least one month following treatment according to the methods disclosed herein. Alternatively, an improvement in the disease may be categorized as being a partial response. By "partial response" is intended at least about a 50% decrease in all measurable tumor burden (i.e., the number of tumor cells present in the subject) in the absence of new lesions and persisting for at least one month. Such a response is applicable to measurable tumors only.

Tumor response can be assessed for changes in tumor morphology (i.e., overall tumor burden, tumor size, and the like) using screening techniques such as magnetic resonance imaging (MRI) scan, x-radiographic imaging, computed tomographic (CT) scan, bioluminescent imaging, for example, luciferase imaging, bone scan imaging, and tumor biopsy sampling including bone marrow aspiration (BMA). In addition to these positive therapeutic responses, the subject undergoing therapy with the antagonist anti-CD40 antibody or antigen-binding fragment thereof may experience the beneficial effect of an improvement in the symptoms associated with the disease. Thus for B cell tumors, the subject may experience a decrease in the so-called B symptoms, i.e., night sweats, fever, weight loss, and/or urticaria.

By "therapeutically effective dose or amount" or "effective amount" is intended an amount of antagonist anti-CD40 antibody or antigen-binding fragment thereof that, when administered brings about a positive therapeutic response with respect to treatment of a patient with a disease comprising stimulation of CD40-expressing cells. In some embodiments of the invention, a therapeutically effective dose of the anti-CD40 antibody or fragment thereof is in the range from about 0.01 mg/kg to about 40 mg/kg, from about 0.01 mg/kg to about 30 mg/kg, from about 0.1 mg/kg to about 30 mg/kg, from about 1 mg/kg to about 30 mg/kg, from about 3 mg/kg to about 30 mg/kg, from about 3 mg/kg to about 25 mg/kg, from about 3 mg/kg to about 20 mg/kg, from about 5 mg/kg to about 15 mg/kg, or from about 7 mg/kg to about 12 mg/kg. It is recognized that the method of treatment may comprise a single administration of a therapeutically effective dose or multiple administrations of a therapeutically effective dose of the antagonist anti-CD40 antibody or antigen-binding fragment thereof.

A further embodiment of the disclosure is the use of antagonist anti-CD40 antibodies for diagnostic monitoring of protein levels in tissue as part of a clinical testing procedure, e.g., to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include ¹²⁵I, ¹³¹I, ³⁵S, or ³H.

The anti-CD40 antibodies described herein can further be used to provide reagents, e.g., labeled antibodies that can be used, for example, to identify cells expressing CD40. This can be very useful in determining the cell type of an unknown sample. Panels of monoclonal antibodies can be used to identify tissue by species and/or by organ type. In a similar fashion, these anti-CD40 antibodies can be used to screen tissue culture cells for contamination (i.e., screen for the presence of a mixture of CD40-expressing and non-CD40 expressing cells in a culture).

The antagonist anti-CD40 antibodies can be used in combination with known chemotherapeutics and cytokines for the treatment of disease states comprising stimulated CD40-expressing cells. For example, the anti-CD40 antibodies of the invention can be used in combination with cytokines such as interleukin-2. In another embodiment, the anti-CD40 antibodies of the invention can be used in combination with rituximab (IDEC-C2B8; Rituxan®; IDEC Pharmaceuticals Corp., San Diego, California).

In this manner, the antagonist anti-CD40 antibodies described herein, or antigen-binding fragments thereof, are administered in combination with at least one other cancer therapy, including, but not limited to, surgery or surgical procedures (e.g. splenectomy, hepatectomy, lymphadenectomy, leukophoresis, bone marrow transplantation, and the like); radiation therapy; chemotherapy, optionally in combination with autologous bone marrow transplant, where suitable chemotherapeutic agents include, but are not limited to, fludarabine or fludarabine phosphate, chlorambucil, vincristine, pentostatin, 2-chlorodeoxyadenosine (cladribine), cyclophosphamide, doxorubicin, prednisone, and combinations thereof, for example, anthracycline-containing regimens such as CAP (cyclophosphamide, doxorubicin plus prednisone), CHOP (cyclophosphamide, vincristine, prednisone plus doxorubicin), VAD (vincritsine, doxorubicin, plus dexamethasone), MP (melphalan plus prednisone), and other cytotoxic and/or therapeutic agents used in chemotherapy such as mitoxantrone, daunorubicin, idarubicin, asparaginase, and antimetabolites, including, but not limited to, cytarabine, methotrexate, 5-fluorouracil decarbazine, 6-thioguanine, 6-mercaptopurine, and nelarabine; other anti-cancer monoclonal antibody therapy (for example, alemtuzumab (Campath^{®}) or other anti-CD52 antibody targeting the CD52 cell-surface glycoprotein on malignant B cells; rituximab (Rituxan^{®}), the fully human antibody HuMax-CD20, R-1594, IMMU-106, TRU-015, AME-133, tositumomab/I-131 tositumomab (Bexxar®), ibritumomab tiuxetan (Zevalin®), or any other therapeutic anti-CD20 antibody targeting the CD20 antigen on malignant B cells; anti-CD19 antibody (for example, MT103, a bispecific antibody); anti-CD22 antibody (for example, the humanized monoclonal antibody epratuzumab); bevacizumab (Avastin®) or other anti-cancer antibody targeting human vascular endothelial growth factor; anti-CD22 antibody targeting the CD22 antigen on malignant B cells (for example, the monoclonal antibody BL-22, an alphaCD22 toxin); α-M-CSF antibody targeting macrophage colony stimulating factor; antibodies targeting the receptor activator of nuclear factor-kappaB (RANK) and its ligand (RANKIL), which are overexpressed in multiple myeloma; anti-CD23 antibody targeting the CD23 antigen on malignant B cells (for example, IDEC-152); anti-CD80 antibody targeting the CD80 antigen (for example, IDEC-114); anti-CD38 antibody targeting the CD38 antigen on malignant B cells; antibodies targeting major histocompatibility complex class II receptors (anti-MHC antibodies) expressed on malignant B cells; other anti-CD40 antibodies (for example, SGN-40) targeting the CD40 antigen on malignant B cells; and antibodies targeting tumor necrosis factor-related apoptosis-inducing ligand receptor 1 (TRAIL-R1) (for example, the agonistic human monoclonal antibody HGS-ETR1) and TRAIL-R2 expressed on a number of solid tumors and tumors of hematopoietic origin); small molecule-based cancer therapy, including, but not limited to, microtubule and/or topoisomerase inhibitors (for example, the mitotic inhibitor dolastatin and dolastatin analogues; the tubulin-binding agent T900607; XL119; and the topoisomerase inhibitor aminocamptothecin), SDX-105 (bendamustine hydrochloride), ixabepilone (an epothilone analog, also referred to as BMS-247550), protein kinase C inhibitors, for example, midostaurin ((PKC-412, CGP 41251, N-benzoylstaurosporine), pixantrone, eloxatin (an antineoplastic agent), ganite (gallium nitrate), Thalomid^{®} (thalidomide), immunomodulatory derivatives of thalidomide (for example, revlimid (formerly revimid)), Affinitak™ (antisense inhibitor of protein kinase C-alpha), SDX-101 (R-etodolac, inducing apoptosis of malignant lymphocytes), second-generation purine nucleoside analogs such as clofarabine, inhibitors of production of the protein Bcl-2 by cancer cells (for example, the antisense agents oblimersen and Genasense^{®}), proteasome inhibitors (for example, Velcade™ (bortezomib)), small molecule kinase inhibitors (for example, CHIR-258), small molecule VEGF inhibitors (for example, ZD-6474), small molecule inhibitors of heat shock protein (HSP) 90 (for example, 17-AAG), small molecule inhibitors of histone deacetylases (for example, hybrid/polar cytodifferentiation HPC) agents such as suberanilohydroxamic acid (SAHA), and FR-901228) and apoptotic agents such as Trisenox^{®} (arsenic trioxide) and Xcytrin^{®} (motexafin gadolinium); vaccine /immunotherapy-based cancer therapies, including, but not limited to, vaccine approaches (for example, Id-KLH, oncophage, vitalethine), personalized immunotherapy or active idiotype immunotherapy (for example, MyVax^{®} Personalized Immunotherapy, formally designated GTOP-99), Promune® (CpG 7909, a synthetic agonist for toll-like receptor 9 (TLR9)), interferon-alpha therapy, interleukin-2 (IL-2) therapy, IL-12 therapy, IL-15 therapy, and IL-21 therapy; steroid therapy; or other cancer therapy; where the additional cancer therapy is administered prior to, during, or subsequent to the antagonist anti-CD40 antibody therapy. Thus, where the combined therapies comprise administration of an antagonist anti-CD40 antibody or antigen-binding fragment thereof in combination with administration of another therapeutic agent, as with chemotherapy, radiation therapy, other anti-cancer antibody therapy, small molecule-based cancer therapy, or vaccine/immunotherapy-based cancer therapy, the methods disclosed herein encompass coadministration, using separate formulations or a single pharmaceutical formulation, or and consecutive administration in either order. Where the methods disclosed herein comprise combined therapeutic regimens, these therapies can be given simultaneously, i.e., the antagonist anti-CD40 antibody or antigen-binding fragment thereof is administered concurrently or within the same time frame as the other cancer therapy (i.e., the therapies are going on concurrently, but the antagonist anti-CD40 antibody or antigen-binding fragment thereof is not administered precisely at the same time as the other cancer therapy). Alternatively, the antagonist anti-CD40 antibody of the present invention or antigen-binding fragment thereof may also be administered prior to or subsequent to the other cancer therapy. Sequential administration of the different cancer therapies may be performed regardless of whether the treated subject responds to the first course of therapy to decrease the possibility of remission or relapse. Where the combined therapies comprise administration of the antagonist anti-CD40 antibody or antigen-binding fragment thereof in combination with administration of a cytotoxic agent, preferably the antagonist anti-CD40 antibody or antigen-binding fragment thereof is administered prior to administering the cytotoxic agent.

In some embodiments of the invention, the antagonist anti-CD40 antibodies described herein, or antigen-binding fragments thereof, are administered in combination with chemotherapy, and optionally in combination with autologous bone marrow transplantation, wherein the antibody and the chemotherapeutic agent(s) may be administered sequentially, in either order, or simultaneously (i.e., concurrently or within the same time frame). Examples of suitable chemotherapeutic agents include, but are not limited to, fludarabine or fludarabine phosphate, chlorambucil, vincristine, pentostatin, 2-chlorodeoxyadenosine (cladribine), cyclophosphamide, doxorubicin, prednisone, and combinations thereof, for example, anthracycline-containing regimens such as CAP (cyclophosphamide, doxorubicin plus prednisone), CHOP (cyclophosphamide, vincristine, prednisone plus doxorubicin), VAD (vincritsine, doxorubicin, plus dexamethasone), MP (melphalan plus prednisone), and other cytotoxic and/or therapeutic agents used in chemotherapy such as mitoxantrone, daunorubicin, idarubicin, asparaginase, and antimetabolites, including, but not limited to, cytarabine, methotrexate, 5-fluorouracil decarbazine, 6-thioguanine, 6-mercaptopurine, and nelarabine. In some embodiments, the antagonist anti-CD40 antibody, for example, the monoclonal antibody CHIR-12.12 or CHIR-5.9, or an antigen-binding fragment thereof is administered prior to treatment with the chemotherapeutic agent. In alternative embodiments, the antagonist anti-CD40 antibody is administered after treatment with the chemotherapeutic agent. In yet other embodiments, the chemotherapeutic agent is administered simultaneously with the antagonist anti-CD40 antibody or antigen-binding fragment thereof.

Thus, for example, in some embodiments, the antagonist anti-CD40 antibody, for example, the monoclonal antibody CHIR-12.12 or CHIR-5.9, or antigen-binding fragment thereof is administered in combination with fludarabine or fludarabine phosphate. In one such embodiment, the antagonist anti-CD40 antibody or antigen-binding fragment thereof is administered prior to administration of fludarabine or fludarabine phosphate. In alternative embodiments, the antagonist anti-CD40 antibody or antigen-binding fragment thereof is administered after treatment with fludarabine or fludarabine phosphate. In yet other embodiments, the fludarabine or fludarabine phosphate is administered simultaneously with the antagonist anti-CD40 antibody or antigen-binding fragment thereof.

In other embodiments of the invention, chlorambucil, an alkylating drug, is administered in combination with an antagonist anti-CD40 antibody described herein, for example, the monoclonal antibody CHIR-12.12 or CHIR-5.9, or an antigen-binding fragment thereof. In one such embodiment, the antagonist anti-CD40 antibody or antigen-binding fragment thereof is administered prior to administration of chlorambucil. In alternative embodiments, the antagonist anti-CD40 antibody or antigen-binding fragment thereof is administered after treatment with chlorambucil. In yet other embodiments, the chlorambucil is administered simultaneously with the antagonist anti-CD40 antibody or antigen-binding fragment thereof.

In yet other embodiments, anthracycline-containing regimens such as CAP (cyclophosphamide, doxorubicin plus prednisone) and CHOP (cyclophosphamide, vincristine, prednisone plus doxorubicin) may be combined with administration of an antagonist anti-CD40 antibody described herein, for example, the monoclonal antibody CHIR-12.12 or CHIR-5.9, or antigen-binding fragment thereof. In one such embodiment, the antagonist anti-CD40 antibody or antigen-binding fragment thereof is administered prior to administration of anthracycline-containing regimens. In other embodiments, the antagonist anti-CD40 antibody or antigen-binding fragment thereof is administered after treatment with anthracycline-containing regimens. In yet other embodiments, the anthracycline-containing regimen is administered simultaneously with the antagonist anti-CD40 antibody or antigen-binding fragment thereof.

In alternative embodiments, an antagonist anti-CD40 antibody described herein, for example, the monoclonal antibody CHIR-12.12 or CHIR-5.9, or an antigen-binding fragment thereof, is administered in combination with alemtuzumab (Campath^{®}; distributed by Berlex Laboratories, Richmond, California). Alemtuzumab is a recombinant humanized monoclonal antibody (Campath-1H) that targets the CD52 antigen expressed on malignant B cells. In one such embodiment, the antagonist anti-CD40 antibody or antigen-binding fragment thereof is administered prior to administration of alemtuzumab. In other embodiments, the antagonist anti-CD40 antibody or antigen-binding fragment thereof is administered after treatment with alemtuzumab. In yet other embodiments, the alemtuzumab is administered simultaneously with the antagonist anti-CD40 antibody or antigen-binding fragment thereof.

In alternative embodiments, an antagonist anti-CD40 antibody described herein, for example, the monoclonal antibody CHIR-12.12 or CHIR-5.9, or antigen-binding fragment thereof, is administered in combination with a therapeutic anti-CD20 antibody targeting the CD20 antigen on malignant B cells, for example, rituximab (Rituxan^{®}), the fully human antibody HuMax-CD20, R-1594, IMMU-106, TRU-015, AME-133, tositumomab/I-131 tositumomab (Bexxar®), or ibritumomab tiuxetan (Zevalin®). In one such embodiment, the antagonist anti-CD40 antibody or antigen-binding fragment thereof is administered prior to administration of the anti-CD20 antibody. In other embodiments, the antagonist anti-CD40 antibody or antigen-binding fragment thereof is administered after treatment with the anti-CD20 antibody. In yet other embodiments, the anti-CD20 antibody is administered simultaneously with the antagonist anti-CD40 antibody or antigen-binding fragment thereof.

In alternative embodiments, an antagonist anti-CD40 antibody described herein, for example, the monoclonal antibody CHIR-12.12 or CHIR-5.9, or antigen-binding fragment thereof, is administered in combination with a small molecule-based cancer therapy, including, but not limited to, microtubule and/or topoisomerase inhibitors (for example, the mitotic inhibitor dolastatin and dolastatin analogues; the tubulin-binding agent T900607; XL119; and the topoisomerase inhibitor aminocamptothecin), SDX-105 (bendamustine hydrochloride), ixabepilone (an epothilone analog, also referred to as BMS-247550), protein kinase C inhibitors, for example, midostaurin ((PKC-412, CGP 41251, N-benzoylstaurosporine), pixantrone, eloxatin (an antineoplastic agent), ganite (gallium nitrate), Thalomid^{®} (thalidomide), immunomodulatory derivatives of thalidomide (for example, revlimid (formerly revimid)), Affinitak™ (antisense inhibitor of protein kinase C-alpha), SDX-101 (R-etodolac, inducing apoptosis of malignant lymphocytes), second-generation purine nucleoside analogs such as clofarabine, inhibitors of production of the protein Bcl-2 by cancer cells (for example, the antisense agents oblimersen and Genasense^{®}), proteasome inhibitors (for example, Velcade™ (bortezomib)), small molecule kinase inhibitors (for example, CHIR-258), small molecule VEGF inhibitors (for example, ZD-6474), small molecule inhibitors of heat shock protein (HSP) 90 (for example, 17-AAG), small molecule inhibitors of histone deacetylases (for example, hybrid/polar cytodifferentiation HPC) agents such as suberanilohydroxamic acid (SAHA), and FR-901228) and apoptotic agents such as Trisenox^{®} (arsenic trioxide) and Xcytrin^{®} (motexafin gadolinium). In one such embodiment, the antagonist anti-CD40 antibody or antigen-binding fragment thereof is administered prior to administration of the small molecule-based cancer therapy. In other embodiments, the antagonist anti-CD40 antibody or antigen-binding fragment thereof is administered after treatment with the small molecule-based cancer therapy. In yet other embodiments, the small molecule-based cancer therapy is administered simultaneously with the antagonist anti-CD40 antibody or antigen-binding fragment thereof.

In yet other embodiments, an antagonist anti-CD40 antibody described herein, for example, the monoclonal antibody CHIR-12.12 or CHIR-5.9 or an antigen-binding fragment thereof, can be used in combination with vaccine/immunotherapy-based cancer therapy, including, but not limited to, vaccine approaches (for example, Id-KLH, oncophage, vitalethine), personalized immunotherapy or active idiotype immunotherapy (for example, MyVax^{®} Personalized Immunotherapy, formally designated GTOP-99), Promune® (CpG 7909, a synthetic agonist for toll-like receptor 9 (TLR9)), interferon-alpha therapy, interleukin-2 (IL-2) therapy, IL-12 therapy, IL-15 therapy, or IL-21 therapy; or steroid therapy. In one such embodiment, the antagonist anti-CD40 antibody or antigen-binding fragment thereof is administered prior to administration of the vaccine/immunotherapy-based cancer therapy. In other embodiments, the antagonist anti-CD40 antibody or antigen-binding fragment thereof is administered after treatment with the vaccine/immunotherapy-based cancer therapy. In yet other embodiments, the vaccine/immunotherapy-based cancer therapy is administered simultaneously with the antagonist anti-CD40 antibody or antigen-binding fragment thereof.

In one such embodiment, an antagonist anti-CD40 antibody described herein, for example, the monoclonal antibody CHIR-12.12 or CHIR-5.9, or an antigen-binding fragment thereof, can be used in combination with IL-2. IL-2, an agent known to expand the number of natural killer (NK) effector cells in treated patients, can be administered prior to, or concomitantly with, the antagonist anti-CD40 antibody of the invention or antigen-binding fragment thereof. This expanded number of NK effector cells may lead to enhanced ADCC activity of the administered antagonist anti-CD40 antibody or antigen-binding fragment thereof. In other embodiments, IL-21 serves as the immunotherapeutic agent to stimulate NK cell activity when administered in combination with an antagonist anti-CD40 antibody described herein, for example, the monoclonal antibody CHIR-12.12 or CHIR-5.9, or an antigen-binding fragment thereof.

Further, combination therapy with two or more therapeutic agents and an antagonist anti-CD40 antibody described herein can also be used for treatment of a treatment of disease states comprising stimulated CD40-expressing cells, for example, B cell-related cancers, and autoimmune and/or inflammatory disorders. Without being limiting, examples include triple combination therapy, where two chemotherapeutic agents are administered in combination with an antagonist anti-CD40 antibody described herein, and where a chemotherapeutic agent and another anti-cancer monoclonal antibody (for example, alemtuzumab, rituximab, or anti-CD23 antibody) are administered in combination with an antagonist anti-CD40 antibody described herein. Examples of such combinations include, but are not limited to, combinations of fludarabine, cyclophosphamide, and the antagonist anti-CD40 antibody, for example, the monoclonal antibody CHIR-12.12 or CHIR-5.9 or an antigen-binding fragment thereof; and combinations of fludarabine, an anti-CD20 antibody, for example, rituximab (Rituxan^{®}; IDEC Pharmaceuticals Corp., San Diego, California), and the antagonist anti-CD40 antibody, for example, the monoclonal antibody CHIR-12.12 or CHIR-5.9 or an antigen-binding fragment thereof.

### Pharmaceutical Formulations and Modes of Administration

The antagonist anti-CD40 antibodies of this invention are administered at a concentration that is therapeutically effective to prevent or treat CD40-expressing cell-mediated diseases such as SLE, PBC, ITP, multiple sclerosis, psoriasis, Crohn's disease, graft rejection, and B-cell lymphoma. To accomplish this goal, the antibodies may be formulated using a variety of acceptable excipients known in the art. Typically, the antibodies are administered by injection, either intravenously or intraperitoneally. Methods to accomplish this administration are known to those of ordinary skill in the art. It may also be possible to obtain compositions which may be topically or orally administered, or which may be capable of transmission across mucous membranes.

Intravenous administration occurs preferably by infusion over a period of about 1 to about 10 hours, more preferably over about 1 to about 8 hours, even more preferably over about 2 to about 7 hours, still more preferably over about 4 to about 6 hours, depending upon the anti-CD40 antibody being administered. The initial infusion with the pharmaceutical composition may be given over a period of about 4 to about 6 hours with subsequent infusions delivered more quickly. Subsequent infusions may be administered over a period of about 1 to about 6 hours, including, for example, about 1 to about 4 hours, about 1 to about 3 hours, or about 1 to about 2 hours.

A pharmaceutical composition of the invention is formulated to be compatible with its intended route of administration. Examples of possible routes of administration include parenteral, (e.g., intravenous (IV), intramuscular (IM), intradermal, subcutaneous (SC), or infusion), oral and pulmonary (e.g., inhalation), nasal, transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

The anti-CD40 antibodies are typically provided by standard technique within a pharmaceutically acceptable buffer, for example, sterile saline, sterile buffered water, propylene glycol, combinations of the foregoing, etc. Methods for preparing parenterally administrable agents are described in Remington's Pharmaceutical Sciences (18th ed.; Mack Publishing Company, Eaton, Pennsylvania, 1990). See also, for example, WO 98/56418, which describes stabilized antibody pharmaceutical formulations suitable for use in the methods disclosed herein.

The amount of at least one anti-CD40 antibody or fragment thereof to be administered is readily determined by one of ordinary skill in the art without undue experimentation. Factors influencing the mode of administration and the respective amount of at least one antagonist anti-CD40 antibody (or fragment thereof) include, but are not limited to, the the severity of the disease, the history of the disease, and the age, height, weight, health, and physical condition of the individual undergoing therapy. Similarly, the amount of antagonist anti-CD40 antibody or fragment thereof to be administered will be dependent upon the mode of administration and whether the subject will undergo a single dose or multiple doses of this anti-tumor agent. Generally, a higher dosage of anti-CD40 antibody or fragment thereof is preferred with increasing weight of the subject undergoing therapy. The dose of anti-CD40 antibody or fragment thereof to be administered is in the range from about 0.003 mg/kg to about 50 mg/kg, preferably in the range of 0.01 mg/kg to about 40 mg/kg. Thus, for example, the dose can be 0.01 mg/kg, 0.03 mg/kg, 0.1 mg/kg, 0.3 mg/kg, 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, or 50 mg/kg.

In another embodiment of the disclosure, the method comprises administration of multiple doses of antagonist anti-CD40 antibody or fragment thereof. The method may comprise administration of 1, 2, 3, 4, 5, 6, 7, 8, 9, 10, 15, 20, 25, 30, 35, 40, or more therapeutically effective doses of a pharmaceutical composition comprising an antagonist anti-CD40 antibody or fragment thereof. The frequency and duration of administration of multiple doses of the pharmaceutical compositions comprising anti-CD40 antibody or fragment thereof can be readily determined by one of skill in the art without undue experimentation. Moreover, treatment of a subject with a therapeutically effective amount of an antibody can include a single treatment or, preferably, can include a series of treatments. In a preferred example, a subject is treated with antagonist anti-CD40 antibody or antigen-binding fragment thereof in the range of between about 0.1 to 20 mg/kg body weight, once per week for between about 1 to 10 weeks, preferably between about 2 to 8 weeks, more preferably between about 3 to 7 weeks, and even more preferably for about 4, 5, or 6 weeks. Treatment may occur annually to prevent relapse or upon indication of relapse. It will also be appreciated that the effective dosage of antibody or antigen-binding fragment thereof used for treatment may increase or decrease over the course of a particular treatment. Changes in dosage may result and become apparent from the results of diagnostic assays as described herein.

Thus, in one embodiment, the dosing regimen includes a first administration of a therapeutically effective dose of at least one anti-CD40 antibody or fragment thereof on days 1, 7, 14, and 21 of a treatment period. In another embodiment, the dosing regimen includes a first administration of a therapeutically effective dose of at least one anti-CD40 antibody or fragment thereof on days 1, 2, 3, 4, 5, 6, and 7 of a week in a treatment period. Further embodiments include a dosing regimen having a first administration of a therapeutically effective dose of at least one anti-CD40 antibody or fragment thereof on days 1, 3, 5, and 7 of a week in a treatment period; a dosing regimen including a first administration of a therapeutically effective dose of at least one anti-CD40 antibody or fragment thereof on days 1 and 3 of a week in a treatment period; and a preferred dosing regimen including a first administration of a therapeutically effective dose of at least one anti-CD40 antibody or fragment thereof on day 1 of a week in a treatment period. The treatment period may comprise 1 week, 2 weeks, 3 weeks, a month, 3 months, 6 months, or a year. Treatment periods may be subsequent or separated from each other by a day, a week, 2 weeks, a month, 3 months, 6 months, or a year.

In some embodiments, the therapeutically effective doses of antagonist anti-CD40 antibody or antigen-binding fragment thereof ranges from about 0.003 mg/kg to about 50 mg/kg, from about 0.01 mg/kg to about 40 mg/kg, from about 0.01 mg/kg to about 30 mg/kg, from about 0.1 mg/kg to about 30 mg/kg, from about 0.5 mg/kg to about 30 mg/kg, from about 1 mg/kg to about 30 mg/kg, from about 3 mg/kg to about 30 mg/kg, from about 3 mg/kg to about 25 mg/kg, from about 3 mg/kg to about 20 mg/kg, from about 5 mg/kg to about 15 mg/kg, or from about 7 mg/kg to about 12 mg/kg. Thus, for example, the dose of any one antagonist anti-CD40 antibody or antigen-binding fragment thereof, for example the anti-CD40 monoclonal antibody CHIR-12.12 or CHIR-5.9 or antigen-binding fragment thereof, can be 0.003 mg/kg, 0.01 mg/kg, 0.03 mg/kg, 0.1 mg/kg, 0.3 mg/kg, 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, 50 mg/kg, or other such doses falling within the range of about 0.003 mg/kg to about 50 mg/kg. The same therapeutically effective dose of an antagonist anti-CD40 antibody or antigen-binding fragment thereof can be administered throughout each week of antibody dosing. Alternatively, different therapeutically effective doses of an antagonist anti-CD40 antibody or antigen-binding fragment thereof can be used over the course of a treatment period.

In other embodiments, the initial therapeutically effective dose of an antagonist anti-CD40 antibody or antigen-binding fragment thereof as defined elsewhere herein can be in the lower dosing range (i.e., about 0.003 mg/kg to about 20 mg/kg) with subsequent doses falling within the higher dosing range (i.e., from about 20 mg/kg to about 50 mg/kg).

In alternative embodiments, the initial therapeutically effective dose of an antagonist anti-CD40 antibody or antigen-binding fragment thereof as defined elsewhere herein can be in the upper dosing range (i.e., about 20 mg/kg to about 50 mg/kg) with subsequent doses falling within the lower dosing range (i.e., 0.003 mg/kg to about 20 mg/kg). Thus, in one embodiment, the initial therapeutically effective dose of the antagonist anti-CD40 antibody or antigen-binding fragment thereof is about 20 mg/kg to about 35 mg/kg, including about 20 mg/kg, about 25 mg/kg, about 30 mg/kg, and about 35 mg/kg, and subsequent therapeutically effective doses of the antagonist anti-CD40 antibody or antigen binding fragment thereof are about 5 mg/kg to about 15 mg/kg, including about 5 mg/kg, 8 mg/kg, 10 mg/kg, 12 mg/kg, and about 15 mg/kg.

In some embodiments of the invention, antagonist anti-CD40 antibody therapy is initiated by administering a "loading dose" of the antibody or antigen-binding fragment thereof to the subject in need of antagonist anti-CD40 antibody therapy. By "loading dose" is intended an initial dose of the antagonist anti-CD40 antibody or antigen-binding fragment thereof that is administered to the subject, where the dose of the antibody or antigen-binding fragment thereof administered falls within the higher dosing range (i.e., from about 20 mg/kg to about 50 mg/kg). The "loading dose" can be administered as a single administration, for example, a single infusion where the antibody or antigen-binding fragment thereof is administered IV, or as multiple administrations, for example, multiple infusions where the antibody or antigen-binding fragment thereof is administered IV, so long as the complete "loading dose" is administered within about a 24-hour period. Following administration of the "loading dose," the subject is then administered one or more additional therapeutically effective doses of the antagonist anti-CD40 antibody or antigen-binding fragment thereof. Subsequent therapeutically effective doses can be administered, for example, according to a weekly dosing schedule, or once every two weeks, once every three weeks, or once every four weeks. In such embodiments, the subsequent therapeutically effective doses generally fall within the lower dosing range (i.e., 0.003 mg/kg to about 20 mg/kg).

Alternatively, in some embodiments, following the "loading dose, " the subsequent therapeutically effective doses of the antagonist anti-CD40 antibody or antigen-binding fragment thereof are administered according to a "maintenance schedule," wherein the therapeutically effective dose of the antibody or antigen-binding fragment thereof is administered once a month, once every 6 weeks, once every two months, once every 10 weeks, once every three months, once every 14 weeks, once every four months, once every 18 weeks, once every five months, once every 22 weeks, once every six months, once every 7 months, once every 8 months, once every 9 months, once every 10 months, once every 11 months, or once every 12 months. In such embodiments, the therapeutically effective doses of the antagonist anti-CD40 antibody or antigen-binding fragment thereof fall within the lower dosing range (i.e., 0.003 mg/kg to about 20 mg/kg), particularly when the subsequent doses are administered at more frequent intervals, for example, once every two weeks to once every month, or within the higher dosing range (i.e., from about 20 mg/kg to about 50 mg/kg), particularly when the subsequent doses are administered at less frequent intervals, for example, where subsequent doses are administered about one month to about 12 months apart.

The antagonist anti-CD40 antibodies present in the pharmaceutical compositions described herein for use in the methods disclosed herein may be native or obtained by recombinant techniques, and may be from any source, including mammalian sources such as, e.g., mouse, rat, rabbit, primate, pig, and human. Preferably such polypeptides are derived from a human source, and more preferably are recombinant, human proteins from hybridoma cell lines.

The pharmaceutical compositions useful in the methods disclosed herein may comprise biologically active variants of the antagonist anti-CD40 antibodies of the invention. Such variants should retain the desired biological activity of the native polypeptide such that the pharmaceutical composition comprising the variant polypeptide has the same therapeutic effect as the pharmaceutical composition comprising the native polypeptide when administered to a subject. That is, the variant anti-CD40 antibody will serve as a therapeutically active component in the pharmaceutical composition in a manner similar to that observed for the native antagonist antibody, for example CHIR-5.9 or CHIR-12.12 as expressed by the hybridoma cell line 5.9 or 12.12, respectively. Methods are available in the art for determining whether a variant anti-CD40 antibody retains the desired biological activity, and hence serves as a therapeutically active component in the pharmaceutical composition. Biological activity of antibody variants can be measured using assays specifically designed for measuring activity of the native antagonist antibody, including assays described herein.

Any pharmaceutical composition comprising an antagonist anti-CD40 antibody having the binding properties described herein as the therapeutically active component can be used in the methods disclosed herein. Thus liquid, lyophilized, of spray-dried compositions comprising one or more of the antagonist anti-CD40 antibodies of the invention may be prepared as an aqueous or nonaqueous solution or suspension for subsequent administration to a subject in accordance with the methods disclosed herein. Each of these compositions will comprise at least one of the antagonist anti-CD40 antibodies of the present invention as a therapeutically or prophylactically active component. By "therapeutically or prophylactically active component" is intended the anti-CD40 antibody is specifically incorporated into the composition to bring about a desired therapeutic or prophylactic response with regard to treatment, prevention, or diagnosis of a disease or condition within a subject when the pharmaceutical composition is administered to that subject. Preferably the pharmaceutical compositions comprise appropriate stabilizing agents, bulking agents, or both to minimize problems associated with loss of protein stability and biological activity during preparation and storage.

Formulants may be added to pharmaceutical compositions comprising an antagonist anti-CD40 antibody of the invention. These formulants may include, but are not limited to, oils, polymers, vitamins, carbohydrates, amine acids, salts, buffers, albumin, surfactants, or bulking agents. Preferably carbohydrates include sugar or sugar alcohols such as mono-, di-, or polysaccharides, or water soluble glucans. The saccharides or glucans can include fructose, glucose, mannose, sorbose, xylose, maltose, sucrose, dextran, pullulan, dextrin, α and β cyclodextrin, soluble starch, hydroxyethyl starch, and carboxymethylcellulose, or mixtures thereof. "Sugar alcohol" is defined as a C₄ to C₈ hydrocarbon having a hydroxyl group and includes galactitol, inositol, mannitol, xylitol, sorbitol, glycerol, and arabitol. These sugars or sugar alcohols may be used individually or in combination. The sugar or sugar alcohol concentration is between 1.0% and 7% w/v., more preferably between 2.0% and 6.0% w/v. Preferably amino acids include levorotary (L) forms of carnitine, arginine, and betaine; however, other amino acids may be added. Preferred polymers include polyvinylpyrrolidone (RVP) with an average molecular weight between 2,000 and 3,000, or polyethylene glycol (PEG) with an average molecular weight between 3,000 and 5,000. Surfactants that can be added to the formulation are shown in EP Nos. 270,799 and 268,110.

Additionally, antibodies can be chemically modified by covalent conjugation to a polymer to increase their circulating half-life, for example. Preferred polymers, and methods to attach them to peptides, are shown in U.S. Patent Nos. 4,766,106; 4,179,337; 4,495,285; and 4,609,546. Preferred polymers are polyoxyethylated polyols and polyethylene glycol (PEG). PEG is soluble in water at room temperature and has the general formula: R(O-CH₂ --CHO₂)ₙ O--R where R can be hydrogen, or a protective group such as an alkyl or alkanol group. Preferably, the protective group has between 1 and 8 carbons, more preferably it is methyl. The symbol n is a positive integer, preferably between 1 and 1,000, more preferably between 2 and 500. The PEG has a preferred average molecular weight between 1,000 and 40,000, more preferably between 2,000 and 20,000, most preferably between 3,000 and 12,000. Preferably, PEG has at least one hydroxy group, more preferably it is a terminal hydroxy group. It is this hydroxy group which is preferably activated to react with a free amino group on the inhibitor. However, it will be understood that the type and amount of the reactive groups may be varied to achieve a covalently conjugated PEG/antibody of the present invention.

Water-soluble polyoxyethylated polyols are also useful in the present invention. They include polyoxyethylated sorbitol, polyoxyethylated glucose, polyoxyethylated glycerol (POG), and the like. POG is preferred. One reason is because the glycerol backbone of polyoxyethylated glycerol is the same backbone occurring naturally in, for example, animals and humans in mono-, di-, triglycerides. Therefore, this branching would not necessarily be seen as a foreign agent in the body. The POG has a preferred molecular weight in the same range as PEG The structure for POG is shown in Knauf et al. (1988) J. Bio. Chem. 263:15064-15070, and a discussion of POG/IL-2 conjugates is found in U.S. Patent No. 4,766,106.

Another drug delivery system for increasing circulatory half-life is the liposome. Methods of preparing liposome delivery systems are discussed in Gabizon et al. (1982) Cancer Research 42:4734; Cafiso (1981) Biochem Biophys Acta 649:129; and Szoka (1980) Ann. Rev. Biophys. Eng. 9:467. Other drug delivery systems are known in the art and are described in, e.g., Poznansky et al. (1980) Drug Delivery Systems (R.L. Juliano, ed., Oxford, N.Y.) pp. 253-315; Poznansky (1984) Pharm Revs 36:277.

The formulants to be incorporated into a pharmaceutical composition should provide for the stability of the antagonist anti-CD40 antibody or antigen-binding fragment thereof. That is, the antagonist anti-CD40 antibody or antigen-binding fragment thereof should retain its physical and/or chemical stability and have the desired biological activity, i.e., one or more of the antagonist activities defined herein above, including, but not limited to, inhibition of immunoglobulin secretion by normal human peripheral B cells stimulated by T cells; inhibition of survival and/or proliferation of normal human peripheral B cells stimulated by Jurkat T cells; inhibition of survival and/or proliferation of normal human peripheral B cells stimulated by CD40L-expressing cells or soluble CD40 ligand (sCD40L); inhibition of "survival" anti-apoptotic intracellular signals in any cell stimulated by sCD40L or solid-phase CD40L; inhibition of CD40 signal transduction in any cell upon ligation with sCD40L or solid-phase CD40L; and inhibition of proliferation of human malignant B cells as noted elsewhere herein.

Methods for monitoring protein stability are well known in the art. See, for example, Jones (1993) Adv. Drug Delivery Rev. 10:29-90; Lee, ed. (1991) Peptide and Protein Drug Delivery (Marcel Dekker, Inc., New York, New York); and the stability assays disclosed herein below. Generally, protein stability is measured at a chosen temperature for a specified period of time. In preferred embodiments, a stable antibody pharmaceutical formulation provides for stability of the antagonist anti-CD40 antibody or antigen-binding fragment thereof when stored at room temperature (about 25°C) for at least 1 month, at least 3 months, or at least 6 months, and/or is stable at about 2-8°C for at least 6 months, at least 9 months, at least 12 months, at least 18 months, at least 24 months.

A protein such as an antibody, when formulated in a pharmaceutical composition, is considered to retain its physical stability at a given point in time if it shows no visual signs (i.e., discoloration or loss of clarity) or measurable signs (for example, using size-exclusion chromatography (SEC) or UV light scattering) of precipitation, aggregation, and/or denaturation in that pharmaceutical composition. With respect to chemical stability, a protein such as an antibody, when formulated in a pharmaceutical composition, is considered to retain its chemical stability at a given point in time if measurements of chemical stability are indicative that the protein (i.e., antibody) retains the biological activity of interest in that pharmaceutical composition. Methods for monitoring changes in chemical stability are well known in the art and include, but are not limited to, methods to detect chemically altered forms of the protein such as result from clipping, using, for example, SDS-PAGE, SEC, and/or matrix-assisted laser desorption ionization/time of flight mass spectrometry; and degradation associated with changes in molecular charge (for example, associated with deamidation), using, for example, ion-exchange chromatography. See, for example, the methods disclosed herein below.

An antagonist anti-CD40 antibody or antigen-binding fragment thereof, when formulated in a pharmaceutical composition, is considered to retain a desired biological activity at a given point in time if the desired biological activity at that time is within about 30%, preferably within about 20% of the desired biological activity exhibited at the time the pharmaceutical composition was prepared as determined in a suitable assay for the desired biological activity. Assays for measuring the desired biological activity of the antagonist anti-CD40 antibodies disclosed herein, and antigen-binding fragments thereof, can be performed as described in the Examples herein. See also the assays described in Schultze et al. (1998) Proc. Natl. Acad. Sci. USA 92:8200-8204; Denton et al. (1998) Pediatr. Transplant. 2:6-15; Evans et al. (2000) J. Immunol. 164:688-697; Noelle (1998) Agents Actions Suppl. 49:17-22; Lederman et al. (1996) Curr. Opin. Hematol. 3:77-86; Coligan et al. (1991) Current Protocols in Immunology 13:12; Kwekkeboom et al. (1993) Immunology 79:439-444; and U.S. Patent Nos. 5,674,492 and 5,847,082.

In some embodiments of the invention, the antagonist anti-CD40 antibody, for example, the CHIR-12.12 or CHIR-5.9 monoclonal antibody, or antigen-binding fragment thereof is formulated in a liquid pharmaceutical formulation. The antagonist anti-CD40 antibody or antigen binding fragment thereof can be prepared using any method known in the art, including those methods disclosed herein above. In one embodiment, the antagonist anti-CD40 antibody, for example, the CHIR-12.12 or CHIR-5.9 monoclonal antibody, or antigen-binding fragment thereof is recombinantly produced in a CHO cell line.

Following its preparation and purification, the antagonist anti-CD40 antibody or antigen-binding fragment thereof can be formulated as a liquid pharmaceutical formulation in the manner set forth herein. Where the antagonist anti-CD40 antibody or antigen-binding fragment thereof is to be stored prior to its formulation, it can be frozen, ro example, at ≤-20°C, and then thawed at room temperature for further formulation. The liquid pharmaceutical formulation comprises a therapeutically effective amount of the antagonist anti-CD40 antibody or antigen-binding fragment thereof. The amount of antibody or antigen-binding fragment thereof present in the formulation takes into consideration the route of administration and desired dose volume.

In this manner, the liquid pharmaceutical composition comprises the antagonist anti-CD40 antibody, for example, the CHIR-12.12 or CHIR-5.9 antibody, or antigen-binding fragment thereof at a concentration of about 0.1 mg/ml to about 50.0 mg/ml, about 0.5 mg/ml to about 40.0 mg/ml, about 1.0 mg/ml to about 30.0 mg/ml, about 5.0 mg/ml to about 25.0 mg/ml, about 5.0 mg/ml to about 20.0 mg/ml, or about 15.0 mg/ml to about 25.0 mg/ml. In some embodiments, the liquid pharmaceutical composition comprises the antagonist anti-CD40 antibody or antigen-binding fragment thereof at a concentration of about 0.1 mg/ml to about 5.0 mg/ml, about 5.0 mg/ml to about 10.0 mg/ml, about 10.0 mg/ml to about 15.0 mg/ml, about 15.0 mg/ml to about 20.0 mg/ml, about 20.0 mg/ml to about 25.0 mg/ml, about 25.0 mg/ml to about 30.0 mg/ml, about 30.0 mg/ml to about 35.0 mg/ml, about 35.0 mg/ml to about 40.0 mg/ml, about 40.0 mg/ml to about 45.0 mg/ml, or about 45.0 mg/ml to about 50.0 mg/ml. In other embodiments, the liquid pharmaceutical composition comprises the antagonist anti-CD40 antibody or antigen-binding fragment thereof at a concentration of about 15.0 mg/ml, about 16.0 mg/ml, about 17.0 mg/ml, about 18.0 mg/ml, about 19.0 mg/ml, about 20.0 mg/ml, about 21.0 mg/ml, about 22.0 mg/ml, about 23.0 mg/ml, about 24.0 mg/ml, or about 25.0 mg/ml. The liquid pharmaceutical composition comprises the antagonist anti-CD40 antibody, for example, the CHIR-12.12 or CHIR-5.9 antibody, or antigen-binding fragment thereof and a buffer that maintains the pH of the formulation in the range of about pH 5.0 to about pH 7.0, including about pH 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, and other such values within the range of about pH 5.0 to about pH 7.0. In some embodiments, the buffer maintains the pH of the formulation in the range of about pH 5.0 to about pH 6.5, about pH 5.0 to about pH 6.0, about pH 5.0 to about pH 5.5, about pH 5.5 to about 7.0, about pH 5.5 to about pH 6.5, or about pH 5.5 to about pH 6.0.

Any suitable buffer that maintains the pH of the liquid anti-CD40 antibody formulation in the range of about pH 5.0 to about pH 7.0 can be used in the formulation, so long as the physicochemical stability and desired biological activity of the antibody are retained as noted herein above. Suitable buffers include, but are not limited to, conventional acids and salts thereof, where the counter ion can be, for example, sodium, potassium, ammonium, calcium, or magnesium. Examples of conventional acids and salts thereof that can be used to buffer the pharmaceutical liquid formulation include, but are not limited to, succinic acid or succinate, citric acid or citrate, acetic acid or acetate, tartaric acid or tartarate, phosphoric acid or phosphate, gluconic acid or gluconate, glutamic acid or glutamate, aspartic acid or aspartate, maleic acid or maleate, and malic acid or malate buffers. The buffer concentration within the formulation can be from about 1 mM to about 50 mM, including about 1 mM, 2 mM, 5 mM, 8 mM, 10 mM, 15 mM, 20 mM, 25 mM, 30 mM, 35 mM, 40 mM, 45 mM, 50 mM, or other such values within the range of about 1 mM to about 50 mM. In some embodiments, the buffer concentration within the formulation is from about 5 mM to about 15 mM, including about 5 mM, 6 mM, 7 mM, 8 mM, 9 mM, 10 mM, 11 mM, 12 mM, 13 mM, 14 mM, 15 mM, or other such values within the range of about 5 mM to about 15 mM.

In some embodiments of the invention, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof and succinate buffer or citrate buffer at a concentration that maintains the pH of the formulation in the range of about pH 5.0 to about pH 7.0, preferably about pH 5.0 to about pH 6.5. By "succinate buffer" or "citrate buffer" is intended a buffer comprising a salt of succinic acid or a salt of citric acid, respectively. In a preferred embodiment, the succinate or citrate counterion is the sodium cation, and thus the buffer is sodium succinate or sodium citrate, respectively. However, any cation is expected to be effective. Other possible succinate or citrate cations include, but are not limited to, potassium, ammonium, calcium, and magnesium. As noted above, the succinate or citrate buffer concentration within the formulation can be from about 1 mM to about 50 mM, including about 1 mM, 2 mM, 5 mM, 8 mM, 10 mM, 15 mM, 20 mM, 25 mM, 30 mM, 35 mM, 40 mM, 45 mM, 50 mM, or other such values within the range of about 1 mM to about 50 mM. In some embodiments, the buffer concentration within the formulation is from about 5 mM to about 15 mM, including about 5 mM, 6 mM, 7 mM, 8 mM, 9 mM, 10 mM, 11 mM, 12 mM, 13 mM, 14 mM, or about 15 mM. In other embodiments, the liquid pharmaceutical formulation comprises the antagonist anti-CD40 antibody, for example, the CHIR-12.12 or CHIR-5.9 monoclonal antibody, or antigen-binding fragment thereof at a concentration of about 0.1 mg/ml to about 50.0 mg/ml, or about 5.0 mg/ml to about 25.0 mg/ml, and succinate or citrate buffer, for example, sodium succinate or sodium citrate buffer, at a concentration of about 1 mM to about 20 mM, about 5 mM to about 15 mM, preferably about 10 mM.

Where it is desirable for the liquid pharmaceutical formulation to be near isotonic, the liquid pharmaceutical formulation comprising a therapeutically effective amount of the antagonist anti-CD40 antibody, for example, the CHIR-12.12 or CHIR-5.9 monoclonal antibody, or antigen-binding fragment thereof, and a buffer to maintain the pH of the formulation within the range of about pH 5.0 to about pH 7.0 can further comprise an amount of an isotonizing agent sufficient to render the formulation near isotonic. By "near isotonic" is intended the aqueous formulation has an osmolarity of about 240 mmol/kg to about 360 mmol/kg, preferably about 240 to about 340 mmol/kg, more preferably about 250 to about 330 mmol/kg, even more preferably about 260 to about 320 mmol/kg, still more preferably about 270 to about 310 mmol/kg. Methods of determining the isotonicity of a solution are known to those skilled in the art. See, for example, Setnikar et al. (1959) J. Am. Pharm. Assoc. 48:628.

Those skilled in the art are familiar with a variety of pharmaceutically acceptable solutes useful in providing isotonicity in pharmaceutical compositions. The isotonizing agent can be any reagent capable of adjusting the osmotic pressure of the liquid pharmaceutical formulation of the present invention to a value nearly equal to that of a body fluid. It is desirable to use a physiologically acceptable isotonizing agent. Thus, the liquid pharmaceutical formulation comprising a therapeutically effective amount of the antagonist anti-CD40 antibody, for example, the CHIR-12.12 or CHIR-5.9 monoclonal antibody, or antigen-binding fragment thereof, and a buffer to maintain the pH of the formulation within the range of about pH 5.0 to about pH 7.0, can further comprise components that can be used to provide isotonicity, for example, sodium chloride; amino acids such as alanine, valine, and glycine; sugars and sugar alcohols (polyols), including, but not limited to, glucose, dextrose, fructose, sucrose, maltose, mannitol, trehalose, glycerol, sorbitol, and xylitol; acetic acid, other organic acids or their salts, and relatively minor amounts of citrates or phosphates. The ordinary skilled person would know of additional agents that are suitable for providing optimal tonicity of the liquid formulation.

In some preferred embodiments, the liquid pharmaceutical formulation comprising a therapeutically effective amount of the antagonist anti-CD40 antibody, for example, the CHIR-12.12 or CHIR-5.9 monoclonal antibody, or antigen-binding fragment thereof, and a buffer to maintain the pH of the formulation within the range of about pH 5.0 to about pH 7.0, further comprises sodium chloride as the isotonizing agent. The concentration of sodium chloride in the formulation will depend upon the contribution of other components to tonicity. In some embodiments, the concentration of sodium chloride is about 50 mM to about 300 mM, about 50 mM to about 250 mM, about 50 mM to about 200 mM, about 50 mM to about 175 mM, about 50 mM to about 150 mM, about 75 mM to about 175 mM, about 75 mM to about 150 mM, about 100 mM to about 175 mM, about 100 mM to about 200 mM, about 100 mM to about 150 mM, about 125 mM to about 175 mM, about 125 mM to about 150 mM, about 130 mM to about 170 mM, about 130 mM to about 160 mM, about 135 mM to about 155 mM, about 140 mM to about 155 mM, or about 145 mM to about 155 mM. In one such embodiment, the concentration of sodium chloride is about 150 mM. In other such embodiments, the concentration of sodium chloride is about 150 mM, the buffer is sodium succinate or sodium citrate buffer at a concentration of about 5 mM to about 15 mM, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, and the formulation has a pH of about pH 5.0 to about pH 7.0, about pH 5.0 to about pH 6.0, or about pH 5.5 to about pH 6.5. In other embodiments, the liquid pharmaceutical formulation comprises the antagonist anti-CD40 antibody, for example, the CHIR-12.12 or CHIR-5.9 monoclonal antibody, or antigen-binding fragment thereof, at a concentration of about 0.1 mg/ml to about 50.0 mg/ml or about 5.0 mg/ml to about 25.0 mg/ml, about 150 mM sodium chloride, and about 10 mM sodium succinate or sodium citrate, at a pH of about pH 5.5.

Protein degradation due to freeze thawing or mechanical shearing during processing of a liquid pharmaceutical formulations of the present invention can be inhibited by incorporation of surfactants into the formulation in order to lower the surface tension at the solution-air interface. Thus, in some embodiments, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, a buffer to maintain the pH of the formulation within the range of about pH 5.0 to about pH 7.0, and further comprises a surfactant. In other embodiments, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, a buffer to maintain the pH of the formulation within the range of about pH 5.0 to about pH 7.0, an isotonizing agent such as sodium chloride at a concentration of about 50 mM to about 300 mM, and further comprises a surfactant.

Typical surfactants employed are nonionic surfactants, including polyoxyethylene sorbitol esters such as polysorbate 80 (Tween 80) and polysorbate 20 (Tween 20); polyoxypropylene-polyoxyethylene esters such as Pluronic F68; polyoxyethylene alcohols such as Brij 35; simethicone; polyethylene glycol such as PEG400; lysophosphatidylcholine; and polyoxyethylene-p-t-octylphenol such as Triton X-100. Classic stabilization of pharmaceuticals by surfactants or emulsifiers is described, for example, in Levine et al. (1991) J. Parenteral Sci. Technol. 45(3): 160-165. A preferred surfactant employed in the practice of the present invention is polysorbate 80. Where a surfactant is included, it is typically added in an amount from about 0.001 % to about 1.0% (w/v), about 0.001% to about 0.5%, about 0.001% to about 0.4%, about 0.001% to about 0.3%, about 0.001% to about 0.2%, about 0.005% to about 0.5%, about 0.005% to about 0.2%, about 0.01% to about 0.5%, about 0.01% to about 0.2%, about 0.03% to about 0.5%, about 0.03% to about 0.3%, about 0.05% to about 0.5%, or about 0.05% to about 0.2%.

Thus, in some embodiments, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, the buffer is sodium succinate or sodium citrate buffer at a concentration of about 1 mM to about 50 mM, about 5 mM to about 25 mM, or about 5 mM to about 15 mM; the formulation has a pH of about pH 5.0 to about pH 7.0, about pH 5.0 to about pH 6.0, or about pH 5.5 to about pH 6.5; and the formulation further comprises a surfactant, for example, polysorbate 80, in an amount from about 0.001% to about 1.0% or about 0.001 % to about 0.5%. Such formulations can optionally comprise an isotonizing agent, such as sodium chloride at a concentration of about 50 mM to about 300 mM, about 50 mM to about 200 mM, or about 50 mM to about 150 mM. In other embodiments, the liquid pharmaceutical formulation comprises the antagonist anti-CD40 antibody, for example, the CHIR-12.12 or CHIR-5.9 monoclonal antibody, or antigen-binding fragment thereof, at a concentration of about 0.1 mg/ml to about 50.0 mg/ml or about 5.0 mg/ml to about 25.0 mg/ml, including about 20.0 mg/ml; about 50 mM to about 200 mM sodium chloride, including about 150 mM sodium chloride; sodium succinate or sodium citrate at about 5 mM to about 20 mM, including about 10 mM sodium succinate or sodium citrate; sodium chloride at a concentration of about 50 mM to about 200 mM, including about 150 mM; and optionally a surfactant, for example, polysorbate 80, in an amount from about 0.001 % to about 1.0%, including about 0.001 % to about 0.5%; where the liquid pharmaceutical formulation has a pH of about pH 5.0 to about pH 7.0, about pH 5.0 to about pH 6.0, about pH 5.0 to about pH 5.5, about pH 5.5 to about pH 6.5, or about pH 5.5 to about pH 6.0.

The liquid pharmaceutical formulation can be essentially free of any preservatives and other carriers, excipients, or stabilizers noted herein above. Alternatively, the formulation can include one or more preservatives, for example, antibacterial agents, pharmaceutically acceptable carriers, excipients, or stabilizers described herein above provided they do not adversely affect the physicochemical stability of the antagonist anti-CD40 antibody or antigen-binding fragment thereof. Examples of acceptable carriers, excipients, and stabilizers include, but are not limited to, additional buffering agents, cosolvents, surfactants, antioxidants including ascorbic acid and methionine, chelating agents such as EDTA, metal complexes (for example, Zn-protein complexes), and biodegradable polymers such as polyesters. A thorough discussion of formulation and selection of pharmaceutically acceptable carriers, stabilizers, and isomolytes can be found in Remington's Pharmaceutical Sciences (18th ed.; Mack Publishing Company, Eaton, Pennsylvania, 1990).

After the liquid pharmaceutical formulation or other pharmaceutical composition described herein is prepared, it can be lyophilized to prevent degradation. Methods for lyophilizing liquid compositions are known to those of ordinary skill in the art. Just prior to use, the composition may be reconstituted with a sterile diluent (Ringer's solution, distilled water, or sterile saline, for example) that may include additional ingredients. Upon reconstitution, the composition is preferably administered to subjects using those methods that are known to those skilled in the art.

### Use of Antagonist Anti-CD40 Antibodies in the Manufacture of Medicaments

The present disclosure also provides for the use of an antagonist anti-CD40 antibody or antigen-binding fragment thereof in the manufacture of a medicament for treating a subject for a cancer characterized by neoplastic B cell growth, wherein the medicament is coordinated with treatment with at least one other cancer therapy. Cancers characterized by neoplastic B cell growth include, but are not limited to, the B cell-related cancers discussed herein above, for example, non-Hodgkin's lymphoma, chronic lymphocytic leukemia, multiple myeloma, B cell lymphoma, high-grade B cell lymphoma, intermediate-grade B cell lymphoma, low-grade B cell lymphoma, B cell acute lympohoblastic leukemia, myeloblastic leukemia, Hodgkin's disease, plasmacytoma, follicular lymphoma, follicular small cleaved lymphoma, follicular large cell lymphoma, follicular mixed small cleaved lymphoma, diffuse small cleaved cell lymphoma, diffuse small lymphocytic lymphoma, prolymphocytic leukemia, lymphoplasmacytic lymphoma, marginal zone lymphoma, mucosal associated lymphoid tissue lymphoma, monocytoid B cell lymphoma, splenic lymphoma, hairy cell leukemia, diffuse large cell lymphoma, mediastinal large B cell lymphoma, lymphomatoid granulomatosis, intravascular lymphomatosis, diffuse mixed cell lymphoma, diffuse large cell lymphoma, immunoblastic lymphoma, Burkitt's lymphoma, AIDS-related lymphoma, and mantle cell lymphoma.

By "coordinated" is intended the medicament comprising the antagonist anti-CD40 antibody or antigen-binding fragment thereof is to be used either prior to, during, or after treatment of the subject with at least one other cancer therapy. Examples of other cancer therapies include, but are not limited to, surgery; radiation therapy; chemotherapy, optionally in combination with autologous bone marrow transplant, where suitable chemotherapeutic agents include, but are not limited to, fludarabine or fludarabine phosphate, chlorambucil, vincristine, pentostatin, 2-chlorodeoxyadenosine (cladribine), cyclophosphamide, doxorubicin, prednisone, and combinations thereof, for example, anthracycline-containing regimens such as CAP (cyclophosphamide, doxorubicin plus prednisone), CHOP (cyclophosphamide, vincristine, prednisone plus doxorubicin), VAD (vincritsine, doxorubicin, plus dexamethasone), MP (melphalan plus prednisone), and other cytotoxic and/or therapeutic agents used in chemotherapy such as mitoxantrone, daunorubicin, idarubicin, asparaginase, and antimetabolites, including, but not limited to, cytarabine, methotrexate, 5-fluorouracil decarbazine, 6-thioguanine, 6-mercaptopurine, and nelarabine; other anti-cancer monoclonal antibody therapy (for example, alemtuzumab (Campath^{®}) or other anti-CD52 antibody targeting the CD52 cell-surface glycoprotein on malignant B cells; rituximab (Rituxan^{®}), the fully human antibody HuMax-CD20, R-1594, IMMU-106, TRU-015, AME-133, tositumomab/I-131 tositumomab (Bexxar®), ibritumomab tiuxetan (Zevalin®), or any other therapeutic anti-CD20 antibody targeting the CD20 antigen on malignant B cells; anti-CD 19 antibody (for example, MT103, a bispecific antibody); anti-CD22 antibody (for example, the humanized monoclonal antibody epratuzumab); bevacizumab (Avastin®) or other anti-cancer antibody targeting human vascular endothelial growth factor; anti-CD22 antibody targeting the CD22 antigen on malignant B cells (for example, the monoclonal antibody BL-22, an alphaCD22 toxin); α-M-CSF antibody targeting macrophage colony stimulating factor; antibodies targeting the receptor activator of nuclear factor-kappaB (RANK) and its ligand (RANKL), which are overexpressed in multiple myeloma; anti-CD23 antibody targeting the CD23 antigen on malignant B cells (for example, IDEC-152); anti-CD38 antibody targeting the CD38 antigen on malignant B cells; antibodies targeting major histocompatibility complex class II receptors (anti-MHC antibodies) expressed on malignant B cells; other anti-CD40 antibodies (for example, SGN-40) targeting the CD40 antigen on malignant B cells; and antibodies targeting tumor necrosis factor-related apoptosis-inducing ligand receptor 1 (TRAIL-R1) (for example, the agonistic human monoclonal antibody HGS-ETR1) expressed on a number of solid tumors and tumors of hematopoietic origin); small molecule-based cancer therapy, including, but not limited to, microtubule and/or topoisomerase inhibitors (for example, the mitotic inhibitor dolastatin and dolastatin analogues; the tubulin-binding agent T900607; XL119; and the topoisomerase inhibitor aminocamptothecin), SDX-105 (bendamustine hydrochloride), ixabepilone (an epothilone analog, also referred to as BMS-247550), protein kinase C inhibitors, for example, midostaurin ((PKC-412, CGP 41251, N-benzoylstaurosporine), pixantrone, eloxatin (an antineoplastic agent), ganite (gallium nitrate), Thalomid^{®} (thalidomide), immunomodulatory derivatives of thalidomide (for example, revlimid (formerly revimid)), Affinitak™ (antisense inhibitor of protein kinase C-alpha), SDX-101 (R-etodolac, inducing apoptosis of malignant lymphocytes), second-generation purine nucleoside analogs such as clofarabine, inhibitors of production of the protein Bcl-2 by cancer cells (for example, the antisense agents oblimersen and Genasense^{®}), proteasome inhibitors (for example, Velcade™ (bortezomib)), small molecule kinase inhibitors (for example, CHIR-258), small molecule VEGF inhibitors (for example, ZD-6474), small molecule inhibitors of heat shock protein (HSP) 90 (for example, 17-AAG), small molecule inhibitors of histone deacetylases (for example, hybrid/polar cytodifferentiation HPC) agents such as suberanilohydroxamic acid (SAHA), and FR-901228) and apoptotic agents such as Trisenox^{®} (arsenic trioxide) and Xcytrin^{®} (motexafin gadolinium); vaccine /immunotherapy-based cancer therapies, including, but not limited to, vaccine approaches (for example, Id-KLH, oncophage, vitalethine), personalized immunotherapy or active idiotype immunotherapy (for example, MyVax^{®} Personalized Immunotherapy, formally designated GTOP-99), Promune® (CpG 7909, a synthetic agonist for toll-like receptor 9 (TLR9)), interferon-alpha therapy, interleukin-2 (IL-2) therapy, IL-12 therapy; IL-15 therapy, and IL-21 therapy; steroid therapy; or other cancer therapy; where treatment with the additional cancer therapy, or additional cancer therapies, occurs prior to, during, or subsequent to treatment of the subject with the medicament comprising the antagonist anti-CD40 antibody or antigen-binding fragment thereof, as noted herein above.

In some embodiments, the present disclosure provides for the use of the anti-CD40 antibody, for example, the monoclonal antibody CHIR-12.12 or CHIR-5.9, or antigen-binding fragment thereof in the manufacture of a medicament for treating a B cell lymphoma, for example non-Hodgkin's lymphoma, in a subject, wherein the medicament is coordinated with treatment with at least one other cancer therapy selected from the group consisting of chemotherapy, anti-cancer antibody therapy, small molecule-based cancer therapy, and vaccine/immunotherapy-based cancer therapy, wherein the medicament is to be used either prior to, during, or after treatment of the subject with the other cancer therapy or, in the case of multiple combination therapies, either prior to, during, or after treatment of the subject with the other cancer therapies.

Thus, for example, in some embodiments, the disclosure provides for the use of the monoclonal antibody CHIR-12.12 or CHIR-5.9, or antigen-binding fragment thereof, in the manufacture of a medicament for treating a B cell lymphoma, for example, non-Hodgkin's lymphoma, in a subject, wherein the medicament is coordinated with treatment with chemotherapy, where the chemotherapeutic agent is selected from the group consisting of cytoxan, doxorubicin, vincristine, prednisone, and combinations thereof, for example CHOP. In other embodiments, the disclosure provides for the use of the monoclonal antibody CHIR-12.12 or CHIR-5.9, or antigen-binding fragment thereof, in the manufacture of a medicament for treating a B cell lymphoma, for example non-Hodgkin's lymphoma, in a subject, wherein the medicament is coordinated with treatment with at least one other anti-cancer antibody selected from the group consisting of alemtuzumab (Campath^{®}) or other anti-CD52 antibody targeting the CD52 cell-surface glycoprotein on malignant B cells; rituximab (Rituxan^{®}), the fully human antibody HuMax-CD20, R-1594, IMMU-106, TRU-015, AME-133, tositumomab/I-131 tositumomab (Bexxar®), ibritumomab tiuxetan (Zevalin®), or any other therapeutic anti-CD20 antibody targeting the CD20 antigen on malignant B cells; anti-CD 19 antibody (for example, MT103, a bispecific antibody); anti-CD22 antibody (for example, the humanized monoclonal antibody epratuzumab); bevacizumab (Avastin®) or other anti-cancer antibody targeting human vascular endothelial growth factor; and any combinations thereof; wherein the medicament is to be used either prior to, during, or after treatment of the subject with the other cancer therapy or, in the case of multiple combination therapies, either prior to, during, or after treatment of the subject with the other cancer therapies.

In yet other embodiments, the present disclosure provides for the use of the monoclonal antibody CHIR-12.12 or CHIR-5.9, or antigen-binding fragment thereof, in the manufacture of a medicament for treating a B cell lymphoma, for example non-Hodgkin's lymphoma, in a subject, wherein the medicament is coordinated with treatment with at least one other small molecule-based cancer therapy selected from the group consisting of microtubule and/or topoisomerase inhibitors (for example, the mitotic inhibitor dolastatin and dolastatin analogues; the tubulin-binding agent T900607; XL119; and the topoisomerase inhibitor aminocamptothecin), SDX-105 (bendamustine hydrochloride), ixabepilone (an epothilone analog, also referred to as BMS-247550), protein kinase C inhibitors, for example, midostaurin ((PKC-412, CGP 41251, N-benzoylstaurosporine), pixantrone, eloxatin (an antineoplastic agent), ganite (gallium nitrate), Thalomid^{®} (thalidomide), an apoptotic agent such as Xcytrin^{®} (motexafin gadolinium), inhibitors of production of the protein Bcl-2 by cancer cells (for example, the antisense agents oblimersen and Genasense^{®}), nelarabine, and any combinations thereof; wherein the medicament is to be used either prior to, during, or after treatment of the subject with the other cancer therapy or, in the case of multiple combination therapies, either prior to, during, or after treatment of the subject with the other cancer therapies.

In still other embodiments, the present disclosure provides for the use of the monoclonal antibody CHIR-12.12 or CHIR-5.9, or antigen-binding fragment thereof, in the manufacture of a medicament for treating a B cell lymphoma, for example non-Hodgkin's lymphoma, in a subject, wherein the medicament is coordinated with treatment with at least one other vaccine/immunotherapy-based cancer therapy selected from the group consisting of vaccine approaches (for example, Id-KLH, oncophage, vitalethine), personalized immunotherapy or active idiotype immunotherapy (for example, MyVax^{®} Personalized Immunotherapy, formally designated GTOP-99), Promune® (CpG 7909, a synthetic agonist for toll-like receptor 9 (TLR9)), interleukin-2 (IL-2) therapy, IL-12 therapy; IL-15 therapy, and IL-21 therapy, and any combinations thereof; wherein the medicament is to be used either prior to, during, or after treatment of the subject with the other cancer therapy or, in the case of multiple combination therapies, either prior to, during, or after treatment of the subject with the other cancer therapies.

In some embodiments, the present disclosure provides for the use of the anti-CD40 antibody, for example, the monoclonal antibody CHIR-12.12 or CHIR-5.9, or antigen-binding fragment thereof in the manufacture of a medicament for treating a B cell-related leukemia, for example B-cell acute lymphocytic leukemia (B-ALL), in a subject, wherein the medicament is coordinated with treatment with at least one other cancer therapy selected from the group consisting of chemotherapy and anti-metabolite therapy, wherein the medicament is to be used either prior to, during, or after treatment of the subject with the other cancer therapy or, in the case of multiple combination therapies, either prior to, during, or after treatment of the subject with the other cancer therapies. Examples of such embodiments include, but are not limited to, those instances where the medicament comprising the antagonist anti-CD40 antibody, for example, the monoclonal antibody CHIR-12.12 or CHIR-5.9, or antigen-binding fragment thereof is coordinated with treatment with a chemotherapeutic agent or anti-metabolite selected from the group consisting of cytoxan, doxorubicin, vincristine, prednisone, cytarabine, mitoxantrone, idarubicin, asparaginase, methotrexate, 6-thioguanine, 6-mercaptopurine, and combinations thereof; wherein the medicament is to be used either prior to, during, or after treatment of the subject with the other cancer therapy or, in the case of multiple combination therapies, either prior to, during, or after treatment of the subject with the other cancer therapies. In one such example, the medicament is coordinated with treatment with cytarabine plus daunorubicin, cytarabine plus mitoxantrone, and/or cytarabine plus idarubicin; wherein the medicament is to be used either prior to, during, or after treatment of the B-ALL subject with the other cancer therapy or, in the case of multiple combination therapies, either prior to, during, or after treatment of the subject with the other cancer therapies.

Also disclosed herein is the use of an antagonist anti-CD40 antibody, for example, the monoclonal antibody CHIR-12.12 or CHIR-5.9 disclosed herein, or antigen-binding fragment thereof in the manufacture of a medicament for treating a subject for a cancer characterized by neoplastic B cell growth, including the B cell-related cancers described herein above, wherein the medicament is used in a subject that has been pretreated with at least one other cancer therapy. By "pretreated" or "pretreatment" is intended the subject has received one or more other cancer therapies (i.e., been treated with at least one other cancer therapy) prior to receiving the medicament comprising the antagonist anti-CD40 antibody or antigen-binding fragment thereof. "Pretreated" or "pretreatment" includes subjects that have been treated with at least one other cancer therapyy within 2 years, within 18 months, within 1 year, within 6 months, within 2 months, within 6 weeks, within 1 month, within 4 weeks, within 3 weeks, within 2 weeks, within 1 week; within 6 days, within 5 days, within 4 days, within 3 days, within 2 days, or even within 1 day prior to initiation of treatment with the medicament comprising the antagonist anti-CD40 antibody, for example, the monoclonal antibody CHIR-12.12 or CHIR-5.9 disclosed herein, or antigen-binding fragment thereof. It is not necessary that the subject was a responder to pretreatment with the prior cancer therapy, or prior cancer therapies. Thus, the subject that receives the medicament comprising the antagonist anti-CD40 antibody or antigen-binding fragment thereof could have responded, or could have failed to respond (i.e. the cancer was refractory), to pretreatment with the prior cancer therapy, or to one or more of the prior cancer therapies where pretreatment comprised multiple cancer therapies. Examples of other cancer therapies for which a subject can have received pretreatment prior to receiving the medicament comprising the antagonist anti-CD40 antibody or antigen-binding fragment thereof include, but are not limited to, surgery; radiation therapy; chemotherapy, optionally in combination with autologous bone marrow transplant, where suitable chemotherapeutic agents include, but are not limited to, those listed herein above; other anti-cancer monoclonal antibody therapy, including, but not limited to, those anti-cancer antibodies listed herein above; small molecule-based cancer therapy, including, but not limited to, the small molecules listed herein above; vaccine/immunotherapy-based cancer therapies, including, but limited to, those listed herein above; steroid therapy; other cancer therapy; or any combination thereof.

"Treatment" in the context of coordinated use of a medicament described herein with one or more other cancer therapies is herein defined as the application or administration of the medicament or of the other cancer therapy to a subject, or application or administration of the medicament or other cancer therapy to an isolated tissue or cell line from a subject, where the subject has a cancer characterized by neoplastic B cell growth, a symptom associated with such a cancer, or a predisposition toward development of such a cancer, where the purpose is to cure, heal, alleviate, relieve, alter, remedy, ameliorate, improve, or affect the cancer, any associated symptoms of the cancer, or the predisposition toward the development of the cancer.

The following examples are offered by way of illustration and not by way of limitation.

### EXPERIMENTAL

The antagonist anti-CD40 antibodies used in the examples below are CHIR-5.9 and CHIR-12.12. The CHIR-5.9 and CHIR-12.12 anti-CD40 antibodies are human IgG₁ subtype anti-human CD40 monoclonal antibodies (mAbs) generated by immunization of transgenic mice bearing the human IgG₁ heavy chain locus and the human κ light chain locus (XenoMouse^{®} technology; Abgenix; Fremont, California). As shown by FACS analysis, CHIR-5.9 and CHIR-12.12 bind specifically to human CD40 and can prevent CD40 ligand binding. Both mAbs can compete off CD40-ligand pre-bound to cell surface CD40. Both antibodies are strong antagonists and inhibit *in vitro* CD40 ligand-mediated proliferation of normal B cells, as well as cancer cells from NHL and CLL patients. *In vitro,* both antibodies kill cancer cell lines as well as primary cancer cells from NHL patients by ADCC. Dose-dependent anti-tumor activity was seen in a xenograft human lymphoma model. The binding affinity of CHIR-5.9 to human CD40 is 1.2x10⁻⁸ M and the binding affinity of CHIR-12.12 to human CD40 is 5x10⁻¹⁰M.

Mouse hybridoma line 131.2F8.5.9 (CMCC#12047) and hybridoma line 153.8E2.D10.D6.12.12 (CMCC#12056) have been deposited with the American Type Culture Collection [ATCC; 10801 University Blvd., Manassas, Virginia 20110-2209 (USA)] under Patent Deposit Number PTA-5542 and PTA-5543, respectively.

The following protocols have been used in the examples described below.

### ELISA Assay for Immunoglobulin Quantification

The concentrations of human IgM and IgG were estimated by ELISA. 96-well ELISA plates were coated with 2 µg/ml goat anti-human IgG MAb (The Jackson Laboratory, Bar Harbor, Maine) or with 2 µg/ml goat anti-human IgM MAb 4102 (Bio Source International, California) in 0.05 M carbonate buffer (pH 9.6), by incubation for 16 hours at 4°C. Plates were washed 3 times with PBS-0.05 % Tween-20 (PBS-Tween) and saturated with BSA for 1 hour. After 2 washes the plates were incubated for 2 hour at 37°C with different dilutions of the test samples. After 3 washes, bound Ig was detected by incubation for 2 hour at 37°C with 1 µg/ml peroxidase-labeled goat anti-human IgG MAb or goat anti-human IgM Mab. Plates were washed 4 times, and bound peroxidase activity was revealed by the addition of O-phenylenediamine as a substrate. Human IgG or IgM standards (Caltaq, Burlingame, California) was used to establish a standard curve for each assay.

### Isolation of the Peripheral Blood Mononuclear Cells (PBMC) from Human Peripheral Blood

20 ml of Ficoll-Paque solution (low endotoxin; Pharmacia) was added per 50 ml polystyrene tube, in 3 tubes, 30 minutes before adding the blood. The Ficoll-Paque solution was warmed up to room temperature. 3 L of bleach in 1:10 dilution was prepared, and used to wash all the tubes and pipettes contacting the blood. The blood was layered on the top of the Ficoll-Paque solution without disturbing the Ficoll layer, at 1.5 ml blood/1 ml of Ficoll-Paque. The tubes were centrifuged at 1700 rpm for 30 minutes at room temperature with the brake on the centrifuge turned off. As much of the top layer (plasma) as possible was removed, minimizing the vacuum in order to avoid removing the second layer of solution. The second layer, which contains the B and T lymphocytes, was collected using a sterile Pasteur pipette, and place in two 50-ml polystyrene tubes. The collection was diluted with 3 x the volume of cold RPMI with no additives, and the tubes were centrifuged at 1000 RPM for 10 minutes. The media was removed by aspiration, and the cells from both 50-ml tubes were resuspended in a total of 10 ml cold RPMI (with additives) and transferred to a 15-ml tube. The cells were counted using the hemacytometer, then centrifuged at 1000 RPM for 10 minutes. The media was removed and the cells resuspended in 4 ml RPMI. This fraction contained the PBMC.

### Isolation of the B cells from PBMC

100 µl of Dynabeads (anti-h CD19) were placed in a 5-ml plastic tube. 3 ml of sterile PBS were added to the beads and mixed, and placed in the magnetic holder, then allowed to sit for 2 minutes. The solution was removed using a Pasteur pipette. 3 ml of sterile PBS were added, mixed, and placed in the magnetic holder, then allowed to sit for 2 minutes. This procedure with sterile PBS was repeated one more time for a total of 3 washes. The PBMC was added into the beads and incubated, while mixing, for 30 minutes at 40°C. The tube containing the PBMC and beads was placed into the magnetic holder for 2 minutes, then the solution was transferred to a new 5-ml tube in the magnetic holder. After 2 minutes, the solution was transferred to a new 15-ml tube. This step was repeated four more times, and the solutions of the first four times were collected in the 15-ml tube, then centrifuged at 1000 RPM for 5 minutes. This step produced the pellet for T-cell separation.

100 µl RPMI (with additives) was added to collect the beads, and the solution was transferred into a 0.7-ml tube. 10 µl of Dynal Detacha Beads were added into the suspension at room temperature, and it was allowed to rotate for 45 minutes. The suspension was transferred into a new 5-ml tube and 3-ml of RPMI (with additives) were added. The tube was placed in the magnetic holder for 2 minutes. The solution was transferred into a new 5-ml tube in the holder for 2 minutes, then to a 15-ml tube. The previous step was repeated three more times, collecting the solution in the 15-ml tube. The 15-ml tube was centrifuged at 1000 RPM for 10 minutes, and the cells resuspended in 10 ml RMPI. The washing step was repeated 2 more times for a total of 3 washes. The cells were counted before the last centrifugation. This step completed the B-cell purification. Cells were stored in 90% FCS and 10% DMSO and frozen at - 800°C.

### Isolation of the T Cells

The human T cell Enrichment Column (R&D systems, anti-h CD 3 column kit) was prepared using 20 ml of 1 X column wash buffer by mixing 2 ml of 10 X column wash buffer and 18 ml of sterile distilled water. The column was cleaned with 70% ethanol and placed on top of a 15-ml tube. The top cap of the column was removed first to avoid drawing air into the bottom of the column. Next, the bottom cap was removed, and the tip was cleaned with 70% ethanol. The fluid within the column was allowed to drain into the 15-ml tube. A new sterile 15-ml tube was placed under the column after the column buffer had drained to the level of the white filter. The B-cell depleted PBMC fraction was suspended in 1 ml of buffer and added to the top of the column. The cells were allowed to incubate with the column at room temperature for 10 minutes. The T-cells were eluted from the column with 4 aliquots of 2 ml each of 1 X column wash buffer. The collected T-cells were centrifuged at 1000 RPM for 5 minutes. The supernatant was removed and the cells resuspended in 10 ml RPMI. Cells were counted and centrifuged one more time. The supernatant was removed, completing the T-cell purification. Cells were stored in 90% FCS and 10% DMSO and frozen at -80°C.

For the above procedures, the RPMLcomposition contained 10% FCS (inactivated at 56°C for 45 min), 1 % Pen/Strep (100 u/ml Penicillin, 0.1 µg/ml Streptomycin), 1% Glutamate, 1% sodium puravate, 50 µM 2-ME.

### Flow Cytofluorometric Assay

Ramos cells (106 cells/sample) were incubated in 100 µl primary antibody (10 µg/ml in PBS-BSA) for 20 min at 4°C. After 3 washes with PBS-BSA or HBSS- BSA, the cells were incubated in 100 µl FITC-labeled F(ab')2 fragments of goat anti-(human IgG) antibodies (Caltaq) for 20 min at 4°C. After 3 washes with PBS-BSA and 1 wash with PBS, the cells were resuspended in 0.5-ml PBS. Analyses were performed with a FACSCAN V (Becton Dickinson, San Jose, California).

### Generation of Hybridoma Clones

Splenocytes from immunized mice were fused with SP 2/0 or P 3 x 63Ag8.653 murine myeloma cells at a ratio of 10:1 using 50% polyethylene glycol as previously described by de Boer et al. (1988) J. Immunol. Meth. 113:143. The fused cells were resuspended in complete IMDM medium supplemented with hypoxanthine (0.1 mM), aminopterin (0.01 mM), thymidine (0.016 mM), and 0.5 ng/ml hIL-6 (Genzyme, Cambridge, Massachusetts). The fused cells were then distributed between the wells of 96-well tissue culture plates, so that each well contained 1 growing hybridoma on average.

After 10-14 days the supernatants of the hybridoma populations were screened for specific antibody production. For the screening of specific antibody production by the hybridoma clones, the supernatants from each well were pooled and tested for anti-CD 40 activity specificity by ELISA first. The positives were then used for fluorescent cell staining of EBV-transformed B cells as described for the FACS assay above. Positive hybridoma cells were cloned twice by limiting dilution in IMDM/FBS containing 0.5 ng/ml hIL-6.

### Example 1: Production of Anti-CD40 Antibodies

Several fully human, antagonist anti-CD40 monoclonal antibodies of IgG1 isotype were generated. Transgenic mice bearing the human IgG1 heavy chain locus and the human κ chain locus (Abgenix γ-1 XenoMouse^{®} technology (Abgenix; Fremont, California)) were used to generate these antibodies. SF9 insect cells expressing CD40 extracellular domain were used as immunogen. A total of 31 mice spleens were fused with the mouse myeloma SP2/0 cells to generate 895 antibodies that recognize recombinant CD40 in ELISA (Tables 1A and 1B). On average approximately 10% of hybridomas produced using Abgenix XenoMouse^{®} technology may contain mouse lambda light chain instead of human kappa chain. The antibodies containing mouse light lambda chain were selected out. A subset of 260 antibodies that also showed binding to cell-surface CD40 were selected for further analysis. Stable hybridomas selected during a series of subcloning procedures were used for further characterization in binding and functional assays.

**Table 1A. A Typical Fusion**

| | **anti-CD40 titer** | | | | |
|---|---|---|---|---|---|
| **Fusion #** | **(1:100K)** | **Fusion Efficiency** | **# of wells screened** | **# of ELISA+** | **# ofcell surface+** |
| 153 | 3 | 100% | 960 | 123 | 33 |
| 154 | 4.67 | 15% | 140 | 0 | 0 |
| 155 | 6 | ~40% | 960 | 3 | 3 |
| 156 | 3.17 | ~25% | 220 | 1 | 0 |
| 157 | 4.67 | 90% | 960 | 32 | 6 |
| 158 | 4.4 | 90% | 960 | 23 | 8 |
| 159 | 1.17 | 100% | 960 | 108 | 18 |
| 160 | 1.78 | 90% | 960 | 30 | 5 |
| Total | | | 6120 | 320 | 73 |

**Table 1B. Summary of Four Sets of Fusions**

| **# of mice** | **ELISA-positive hybridomas** | **Cell surface positive Hybridomas** |
|---|---|---|
| 31 | 895 | 260 |

**Table 2. Summary of initial set of data with anti-CD40 IgG1 antibodies**

| **Mother Hybridoma** | **Hybridoma clones** | **cell surface binding** | **Antagonist** | **ADCC** | **CDC** | **CMCC#** | **V-region DNA sequence** |
|---|---|---|---|---|---|---|---|
| | 131.2F5.8.5.1 | +++ | ++ | ND | ND | ND | |
| 131.2F5 | 131.2F5.8.5.9 | +++ | +++ | ++ | - | 12047 | Yes |
| | 131.2F5.8.5.11 | +++ | +++ | ++ | - | 12055 | Yes |
| | 153.3CSD8D7.8.4.7.1 | ++ | ND | ND | ND | ND | |
| 153.3C5 | 153.3C5D8D7.8.4.7.8 | ++ | ND | ND | ND | ND | |
| | 153.3C5D8D7.8.4.7.11 | +++ | +++ | + | ND | ND | |
| | 153.1D2.9.1 | +++ | ND | ND | ND | 12067 | |
| 153.1D2 | 153.1D2.9.8 | +++ | +++ | ++ | - | 12057 | |
| | 153.1D2.9.12 | +++ | ND | ND | ND | 12068 | |
| | 158.6F3.5.1 | +++ | +++ | ++ | - | 12054 | Yes |
| 158.6F3 | 158.6F3.5.7 | +++ | ND | ND | ND | 12061 | |
| | 158.6F3.5.10 | +++ | ND | ND | ND | 12062 | |
| | 153.8E2D10D6.12.7 | +++ | ND | ND | ND | 12075 | |
| 153.8E2_ | 153.8E2D10D6.12.9 | +++ | ND | ND | ND | 12063 | |
| | 153.8E2D10D6.12.12 | +++ | +++ | ++++ | - | 12056 | Yes |
| | 155.2C2E9F12.2.10.4 | +++ | +/- | ND | ND | 12064 | |
| 155.2C2 | 155.2C2E9F12.2.10.5 | +++ | ND | ND | ND | 12065 | |
| | 155.2C2E9F12.2.10.6 | +/- | ND | ND | ND | 12066 | |
| | 166.5E6G12.1 | +++ | ND | ND | ND | 12069 | |
| 166.5E6_ | 166.5E6G12.3 | +++ | ND | ND | ND | 12070 | |
| | 166.5E6G12.4 | +++ | + | ND | ND | 12071 | |
| 177.8C10 | 177.8C10B3H9 | +++ | ++ | ND | ND | ND | |
| | 183.4B3E11.6.1.5 | ++ | ND | ND | ND | ND | |
| 183.4B3 | 183.4B3E11.6.1.9 | ++ | ND | ND | ND | ND | |
| | 183.4B3E11.6.1.10 | +++ | ++ | ND | ND | ND | |
| | 183.2G5D2.8.7 | +++ | +/- | ND | ND | ND | |
| 183.2G5 | 183.2G5D2.8.8 | +++ | ND | ND | ND | ND | |
| | 183.2G5D2.8.9 | +++ | ND | ND | ND | ND | |
| | 184.6C11D3.2 | ++ | ND | ND | ND | 12078 | |
| 184.6C11 | 184.6C11D3.3 | ++ | ND | ND | ND | 12080 | |
| | 184.6C11D3.6 | +/- | +/- | ND | ND | 12079 | |
| | 185.3E4F12.5.6 | 444 | ND | ND | ND | 12072 | |
| 185.3E4_ | 185.3E4F12.5.11 | +++ | ND | ND | ND | 12073 | |
| | 185.3E4F12.5.12 | +++ | + | ND | ND | 12074 | |
| | 185.1A9E9.6.1 | + | ND | ND | ND | ND | |
| 185.1A9 | 185.1A9E9.6.6 | +++ | +++ | + | ND | ND | |
| | 185.9F11E10.3B5.1 | +++ | ND | ND | ND | ND | |
| 185.9F11 | 185.9F11E10.3B5.8 | +++ | ND | ND | ND | ND | |
| | 185.9F11E103B5.12 | +++ | +++ | ND | ND | ND | |

| | | | | | | | |
|---|---|---|---|---|---|---|---|
| Clones from 7 mother hybridomas were identified to have antagonist activity. Based on their relative antagonist potency and ADCC activities, two hybridoma clones were selected. Their names are: 131.2F8.5.9 (5.9) and 153.8E2.D10.D6.12.12 (12.12). | | | | | | | |

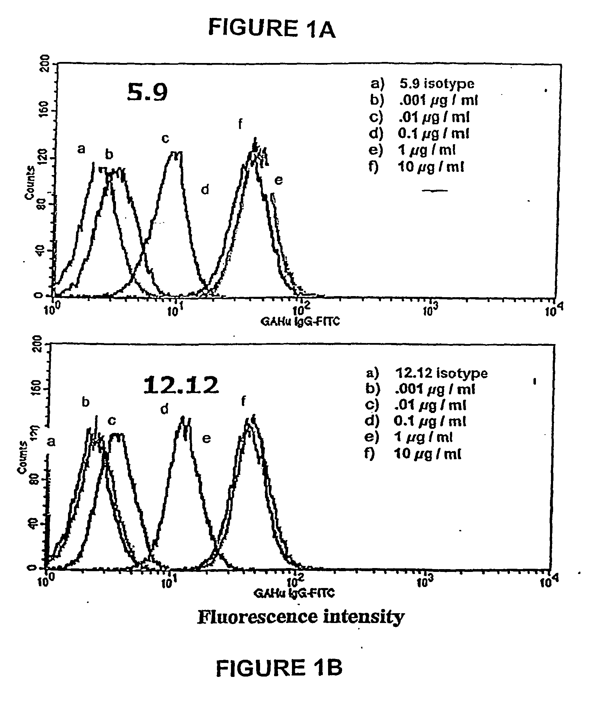

Clones from 7 other hybridomas were identified as having antagonist activity (Table 2 above). Based on their relative antagonistic potency and ADCC activities, two hybridoma clones were selected for further evaluation. They are named 131.2F8.5.9 (5.9) and 153.8E2.D10.D6.12.12 (12.12). The binding profile of these two antibodies to CD40+ lymphoma cell line is shown as a flow cytometric histogram in Figure 1.

### Example 2: Polynucleotide and Amino Acid Sequences of Human Anti-CD40 Antibodies

The cDNAs encoding the variable regions of the candidate antibodies were amplified by PCR, cloned, and sequenced. The amino acid sequences for the light chain and heavy chain of the CHIR-12.12 antibody are set forth in Figures 9A and 9B, respectively. See also SEQ ID NO:2 (light chain for mAb CHIR-12.12) and SEQ ID NO:4 (heavy chain for mAb CHIR-12.12). A variant of the heavy chain for mAb CHIR-12.12 is shown in Figure 9B (see also SEQ ID NO:5), which differs from SEQ ID NO:4 in having a serine residue substituted for the alanine residue at position 153 of SEQ ID NO:4. The nucleotide sequences encoding the light chain and heavy chain of the CHIR-12.12 antibody are set forth in Figures 10A and 10B, respectively. See also SEQ ID NO:1 (coding sequence for light chain for mAb CHIR-12.12) and SEQ ID NO:3 (coding sequence for heavy chain for mAb CHIR-12.12). The amino acid sequences for the light chain and heavy chain of the CHIR-5.9 antibody are set forth in Figures 11A and 11B, respectively. See also SEQ ID NO:6 (light chain for mAb CHIR-5.9) and SEQ ID NO:7 (heavy chain for mAb CHIR-5.9). A variant of the heavy chain for mAb CHIR-5.9 is shown in Figure 11B (see also SEQ ID NO:8), which differs from SEQ ID NO:7 in having a serine residue substituted for the alanine residue at position 158 of SEQ ID NO:7.

As expected for antibodies derived from independent hybridomas, there is substantial variation in the nucleotide sequences in the complementarity determining regions (CDRs). The diversity in the CDR3 region of V_{H} is believed to most significantly determine antibody specificity.

### Example 3: Effect of CHIR-5.9 and CHIR-12.12 on the CD40/CD40L Interaction In vitro

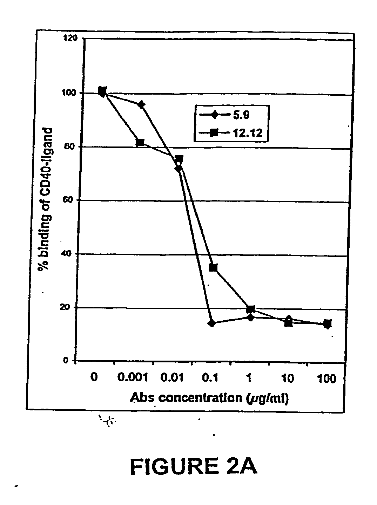

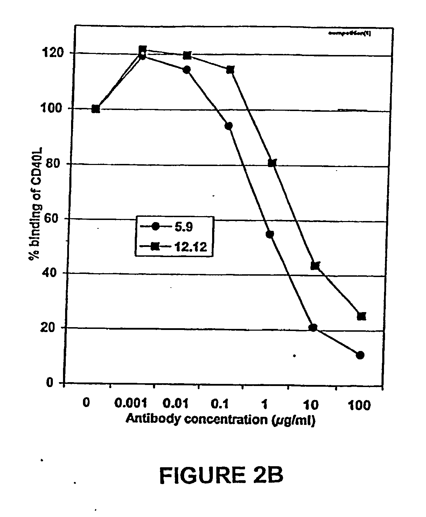

The candidate antibodies CHIR-5.9 and CHIR-12.12 prevent the binding of CD40 ligand to cell surface CD40 and displace the pre-bound CD40 ligand. Antibodies CHIR-5.9 and CHIR-12.12 were tested for their ability to prevent CD40-ligand binding to CD40 on the surface of a lymphoma cell line (Ramos). Binding of both antibodies (unlabeled) prevented the subsequent binding of PE-CD40 ligand as measured by flow cytometric assays (Figure 2A). In a second set of assays the two antibodies were tested for their ability to replace CD40 ligand pre-bound to cell surface CD40. Both antibodies were effective for competing out pre-bound CD40 ligand, with CHIR-5.9 being slightly more effective than CHIR-12.12 (Figure 2B).

### Example 4: CHIR-5.9 and CHIR-12.12 Bind to a Different Epitope on CD40 Than 15B8

The candidate monoclonal antibodies CHIR-5.9 and CHIR-12.12 compete with each other for binding to CD40 but not with 15B8, an IgG₂ anti-CD40 mAb (see International Publication No. WO 02/28904). Antibody competition binding studies using Biacore were designed using CM5 biosensor chips with protein A immobilized via amine coupling, which was used to capture either anti-CD40, CHIR-12.12, or 15B8. Normal association/dissociation binding curves are observed with varying concentrations of CD40-his (data not shown). For competition studies, either CHIR-12.12 or 15B8 were captured onto the protein A surface. Subsequently a CD40-his /CHIR-5.9 Fab complex (100 nM CD40:1 µM CHIR-5.9.Fab), at varying concentrations, was flowed across the modified surface. In the case of CHIR-12.12, there was no association of the complex observed, indicating CHIR-5.9 blocks binding of CHTR-12.12 to CD40-his. For 15B8, association of the Fab CHIR-5.9 complex was observed indicating CHIR-5.9 does not block binding of 15B8 to CD40 binding site. However, the off rate of the complex dramatically increased (data not shown).

It has also been determined that 15B8 and CHIR-12.12 do not compete for CD40-his binding. This experiment was set up by capturing CHIR-12.12 on the protein A biosensor chip, blocking residual protein A sites with control hIgG₁, binding CD40-his and then flowing 15B8 over the modified surface. 15B8 did bind under these conditions indicating CHIR-12.12 does not block 15B8 from binding to CD40.

### Example 5: Binding Properties of Selected Hybridomas

Protein A was immobilized onto CM5 biosensor chips via amine coupling. Human anti-CD40 monoclonal antibodies, at 1.5 µg/ml, were captured onto the modified biosensor surface for 1.5 minutes at 10 µl/min. Recombinant soluble CD40-his was flowed over the biosensor surface at varying concentrations. Antibody and antigen were diluted in 0.01 M HEPES pH 7.4, 0.15 M NaCl, 3 mM EDTA, 0.005% Surfactant P20 (HBS-EP). Kinetic and affinity constants were determined using the Biaevaluation software with a 1:1 interaction model/global fit.

As shown in Table 3 below, there is 121-fold difference in the off rate of CHIR-5.9 and CHIR-12.12 resulting in 24-fold higher affinity for CHIR-12.12.

| **Antibody** | **ka (M-1 sec-1))** | **kd (sec-1)** | **KD (nM)** |
|---|---|---|---|
| Anti-CD40, CHIR-5.9 | (12.35 ± 0.64) x 10⁵ | (15.0 ± 1.3) x 10⁻³ | 12.15 ± 0.35 |
| Anti-CD40, CHIR-12.12 | (2.41 ± 0.13) x 10⁵ | (1.24 ± 0.06) x 10⁻⁴ | 0.51 ± 0.02 |

### Example 6: CHIR-5.9 and CHIE-12.12 are Potent Antagonists for CD40-Mediated Proliferation of Human Lymphocytes from Normal Subjects

Engagement of CD40 by CD40 ligand induces proliferation of human B cells. An antagonist anti-CD40 antibody is expected to inhibit this proliferation. Two candidate antibodies (CHIR-5.9 and CH1R-12.12) were tested for their ability to inhibit CD40 ligand-induced proliferation of PBMC from normal human subjects. Formaldehyde-fixed CHO cells transfectant-expressing CD40 ligand (CD40L) were used as a source of CD40 ligand. Human PBMC were cultured for 4 days with the formaldehyde-fixed CHO cells expressing CD40-ligand in the presence of varying concentrations of anti-CD40 mAb CHIR-5.9 or CHIR-12.12. The proliferation was measured by tritiated-thymidine incorporation. Cells were pulsed with tritiated-labeled thymidine at 37°C for 14-18 hours.

Both antibodies were found to be very effective for inhibiting CD40 ligand-induced proliferation of human PBMC (Table 4A, mAb CHIR-5.9, Table 4B, mAb CHIR-12.12). The experiment was performed with multiple donors of PBMC (n=12 for CHIR-5.9 and n=2 for CHIR-12.12) to ensure that the observed inhibition was not a peculiarity of cells from a single donor. Follow-up assessments with 4 additional donors of PBMC were carried out for mAb CHIR-12.12 with similar trends observed. A wide range of antibody concentrations (0.01 µg/ml to 100 µg/ml) was used in these assays. Nearly complete inhibition of CD40 ligand-induced proliferation could be achieved at 0.1 µg/ml concentration of antibodies in most cases. Antibody concentration (pM) to inhibit 50% of CD-40 ligand-induced lymphocyte proliferation (IC50) for lymphocytes from 6 donors yielded an average IC50 (pM) of 47 (SD=21; donor 1, 24; donor 2, 66; donor 3, 45; donor 4, 84; donor 5, 30; donor 6, 35), which compares favorably with the average IC50 (pM) of 49.65 shown in Table 4B. Based on the current data set, both candidate antibodies seem similar in their potency for inhibition of CD40 ligand-induced proliferation of normal PBMC.

**Table 4A. Effect of mAb CHIR-5.9 on CD40L-induced PBMC proliferation.**

| **Exp#** | | **PBMC alone** | **CHO-CD40L alone** | **PBMC+ CHO-CD40L** | **Abs Conc (*µ*g/ml)** | **hulgG1** | | **CHIR-5.9** | |
|---|---|---|---|---|---|---|---|---|---|
| | | | | | | **CPM** | **% of inhibition** | **CPM** | **% of inhibition** |
| PBMC-010 | | 1851 | 121 | 4436 | 1 | 5080 | **-26** | 2622 | **74** |
| | | 1851 | 121 | 4436 | 0.25 | 5498 | **-43** | 2907 | **62** |
| | | 1851 | 121 | 4436 | 0.0625 | 6029 | **-65** | 2619 | **74** |
| | | 1851 | 121 | 4436 | 0.0156 | 5814 | **-56** | 1199 | **131** |
| PBMC-011 | Donor#1 | 2162 | 178 | 8222 | 10 | 13137 | **-84** | 2252 | **101** |
| | | 2162 | 178 | 8222 | 1 | 11785 | **-61** | 1438 | **115** |
| | | 2162 | 178 | 8222 | 0.1 | 10758 | **-43** | 1249 | **119** |
| | | 2162 | 178 | 8222 | 0.01 | 11322 | **-53** | 4705 | **60** |
| | Donor#2 | 2216 | 294 | 7873 | 10 | 16679 | **-164** | 2362 | **103** |
| | | 2216 | 294 | 7873 | 1 | 14148 | **-117** | 1202 | **124** |
| | | 2216 | 294 | 7873 | 0.1 | 12422 | **-85** | 756 | **133** |
| | | 2216 | 294 | 7873 | 0.01 | 13870 | **-112** | 6606 | **24** |
| | Donor#3 | 2396 | 241 | 11021 | 10 | 11641 | **-7** | 2631 | **100** |
| | | 2396 | 241 | 11021 | 1 | 13528 | **-30** | 1450 | **114** |
| | | 2396 | 241 | 11021 | 0.1 | 12176 | **-14** | 990 | **120** |
| | | 2396 | 241 | 11021 | 0.01 | 11895 | **-10** | 5357 | **68** |
| | Donor#4 | 4552 | 133 | 15301 | 10 | 22098 | **-64** | 3768 | **109** |
| | | 4552 | 133 | 15301 | 1 | 19448 | **-39** | 2040 | **125** |
| | | 4552 | 133 | 15301 | 0.1 | 18398 | **-29** | 1728 | **128** |
| | | 4552 | 133 | 15301 | 0.01 | 22767 | **-70** | 9481 | **55** |
| PBMC-012 | | 777 | 117 | 6041 | 10 | 7327 | **-25** | 2150 | **76** |
| | | 777 | 117 | 6041 | 1 | 6212 | **-3** | 1550 | **87** |
| | | 777 | 117 | 6041 | 0.1 | 7006 | **-19** | 828 | **101** |
| | | 777 | 117 | 6041 | 0.01 | 7524 | **-29** | 1213 | **94** |
| PBMC-014 | | 1857 | 73 | 7889 | 100 | 9399 | **-25** | 3379 | **76** |
| | | 1857 | 73 | 7889 | 20 | 8120 | **-4** | 3870 | **67** |
| | | 1857 | 73 | 7889 | 4 | 8368 | **-8** | 2552 | **90** |
| | | 1857 | 73 | 7889 | 0.8 | 9564 | **-28** | 1725 | **103** |
| PBMC-015 | Donor#1 | 3203 | 127 | 10485 | 100 | 15425 | **-69** | 1497 | **126** |
| | | 3203 | 127 | 10485 | 20 | 11497 | **-14** | 1611 | **124** |
| | | 3203 | 127 | 10485 | 4 | 11641 | **-16** | 1359 | **128** |
| | | 3203 | 127 | 10485 | 0.8 | 12807 | **-32** | 1490 | **126** |
| | Donor#2 | 3680 | 175 | 15145 | 100 | 21432 | **-56** | 1792 | **118** |
| | | 3680 | 175 | 15145 | 20 | 16998 | **-16** | 1779 | **118** |
| | | 3680 | 175 | 15145 | 4 | 17729 | **-23** | 1965 | **117** |
| | | 3680 | 175 | 15145 | 0.8 | 17245 | **-19** | 2217 | **115** |
| | Donor#3 | 2734 | 152 | 19775 | 100 | 22967 | **-19** | 1664 | **107** |
| | | 2734 | 152 | 19775 | 20 | 21224 | **-9** | 1848 | **106** |
| | | 2734 | 152 | 19775 | 4 | 20658 | **-5** | 1534 | **108** |
| | | 2734 | 152 | 19775 | 0.8 | 18923 | **5** | 1262 | **110** |
| PBMC-016 | | 1118 | 36 | 13531 | 0.1 | 10928 | **21** | 745 | **103** |
| | | 1118 | 36 | 13531 | 0.05 | 11467 | **17** | 962 | **102** |
| | | 1118 | 36 | 13531 | 0.01 | 11942 | **13** | 3013 | **85** |
| PBMC-017 | | 962 | 75 | 12510 | 1 | 13597 | **-9** | 258 | **107** |
| Average % inhibition of human PBMC at 100 *µ*g/ml | | | | | | | | **-42** | **107** |
| Average % inhibition of human PBMC at 10 *µ*g/ml | | | | | | | | **-69** | **98** |
| Average % inhibition of human PBMC at 1 *µ*g/ml | | | | | | | | **-41** | **107** |
| Average % inhibition of human PBMC at 0.1 *µ*g/ml | | | | | | | | **-28** | **117** |
| Average % inhibition of human PBMC at 0.01 *µ*g/ml | | | | | | | | **-44** | **64** |
| Average % inhibition of human PBMC | | | | | | | | **-35** | **101** |

| | | | | | | | | | |
|---|---|---|---|---|---|---|---|---|---|
| % of inhibition: 100-(CPM with Abs-PBMC alone-CHO-CD40L alone)/(CPM of PBMC+CHO-CD40L-PBMC alone-CHO-CD40L alone)*100% | | | | | | | | | |

| **Table 4B. Effect of mAb Choir-12.12 on CD40L-induced PBMC proliferation.** | | | | | | | | | | |
|---|---|---|---|---|---|---|---|---|---|---|
| | | | | | | **HulgG1** | | **CHIR-12.12** | | |
| **Exp#** | | **PBMC alone** | **CHO-CD40L alone** | **PBMC+ CHO-CD40L** | **Abs Conc (*µ*g/ml)** | **CPM** | **of inhibition** | **CPM** | **% of inhibition** | **IC50(nM)** |
| PBMC-025 | Donor#1 | 4051 | 32 | 42292 | 0.1 | 33354 | **23** | 440 | **110** | |
| | | 4051 | 32 | 42292 | 0.01 | 37129 | **14** | 8696 | **88** | |
| | | 4051 | 32 | 42292 | 0.001 | 40271 | **5** | 32875 | **25** | |
| | | 4051 | 32 | 42292 | 0.0001 | 40034 | **6** | 37261 | **13** | **24.22** |
| | Donor#2 | 2260 | 31 | 14987 | 0.1 | 15767 | **-6** | 365 | **115** | |
| | | 2260 | 31 | 14987 | 0.01 | 17134 | **-17** | 6734 | **65** | |
| | | 2260 | 31 | 14987 | 0.001 | 20142 | **-41** | 16183 | **-9** | |
| | | 2260 | 31 | 14987 | 0.0001 | 17847 | **-23** | 16187 | **-9** | **65.96** |
| PBMC-026 | Donor#1 | 2039 | 35 | 19071 | 0.1 | 17136 | **11** | 624 | **109** | |
| | | 2039 | 35 | 19071 | 0.01 | 16445 | **15** | 6455 | **74** | |
| | | 2039 | 35 | 19071 | 0.001 | 16195 | **17** | 17833 | **7** | |
| | | 2039 | 35 | 19071 | 0.0001 | 18192 | **5** | 17924 | **7** | **45** |
| | Donor#2 | 2016 | 64 | 17834 | 0.1 | 17181 | **4** | 2078 | **100** | |
| | | 2016 | 64 | 17834 | 0.01 | 16757 | **7** | 10946 | **44** | |
| | | 2016 | 64 | 17834 | 0.001 | 18613 | **-5** | 17924 | **-1** | |
| | | 2016 | 64 | 17834 | 0.0001 | 17169 | **4** | 18569 | **-5** | **84** |
| PBMC-028 | Donor#1 | 4288 | 45 | 22547 | 1 | 18204 | **24** | 2098 | **112** | |
| | | 4288 | 45 | 22547 | 0.1 | 20679 | **10** | 1827 | **114** | |
| | | 4288 | 45 | 22547 | 0.01 | 22799 | **-1** | 6520 | **88** | |
| | | 4288 | 45 | 22547 | 0.001 | 23547 | **-5** | 22327 | **1** | |
| | | 4288 | 45 | 22547 | 0.0001 | 24778 | **-12** | 24124 | **-9** | **30.07** |
| | Donor#2 | 2148 | 58 | 54894 | 1 | 48545 | **12** | 5199 | **94** | |
| | | 2148 | 58 | 54894 | 0.1 | 45708 | **17** | 5091 | **95** | |
| | | 2148 | 58 | 54894 | 0.01 | 51741 | **6** | 18890 | **68** | |
| | | 2148 | 58 | 54894 | 0.001 | 52421 | **5** | 50978 | **7** | |
| | | 2148 | 58 | 54894 | 0.0001 | 54778 | **0** | 52581 | **4** | **34.68** |
| PBMC-029 | Donor#1 | 609 | 69 | 10054 | 0.1 | 11027 | **-10** | 2098 | **85** | |
| | | 609 | 69 | 10054 | 0.01 | 10037 | **0** | 1827 | **88** | |
| | | 609 | 69 | 10054 | 0.001 | 10222 | **-2** | 6520 | **38** | |
| | | 609 | 69 | 10054 | 0.0001 | 11267 | **-13** | 22327 | **-131** | **28.06** |
| | Donor#2 | 7737 | 57 | 23132 | 0.1 | 21254 | **12** | 2536 | **134** | |
| | | 7737 | 57 | 23132 | 0.01 | 21726 | **9** | 10249 | **84** | |
| | | 7737 | 57 | 23132 | 0.001 | 22579 | **4** | 23380 | **-2** | |
| | | 7737 | 57 | 23132 | 0.0001 | 22491 | **4** | 23183 | **0** | **55.35** |
| PBMC-030 | Donor#1 | 2739 | 47 | 53426 | 0.1 | 60116 | **-13** | 2132 | **101** | |
| | | 2739 | 47 | 53426 | 0.01 | 56411 | **-6** | 14297 | **77** | |
| | | 2739 | 47 | 53426 | 0.001 | 59167 | **-11** | 55868 | **-5** | |
| | | 2739 | 47 | 53426 | 0.0001 | 59290 | **-12** | 60865 | **-15** | **35.52** |
| | Donor#2 | 4310 | 50 | 53781 | 0.1 | 52881 | **2** | 3208 | **102** | |
| | | 4310 | 50 | 53781 | 0.01 | 51741 | **4** | 30716 | **47** | |
| | | 4310 | 50 | 53781 | 0.001 | 53072 | **1** | 53628 | **0** | |
| | | 4310 | 50 | 53781 | 0.0001 | 58045 | **-9** | 54343 | **-1** | **102.88** |
| PBMC-032 | Donor#1 | 2458 | 42 | 14058 | 0.1 | 16579 | **-22** | 636 | **116** | **40.36** |
| | | 2458 | 42 | 14058 | 0.01 | 19250 | **-45** | 3358 | **93** | |
| | | 2458 | 42 | 14058 | 0.001 | 19852 | **-50** | 20639 | **-57** | |
| | | 2458 | 42 | 14058 | 0.0001 | 19161 | **-44** | 18907 | **-42** | |
| Average % inhibition of human PBMC at 0.1 *µ*g/ml | | | | | | | **3** | | **107** | |
| Average % inhibition of human PBMC at 0.01 *µ*g/ml | | | | | | | **-1** | | **74** | |
| Average % inhibition of human PBMC at 0.001 *µ*g/ml | | | | | | | **-7** | | **0** | |
| Average % inhibition of human PBMC at 0.0001 *µ*g/ml | | | | | | | **-8** | | **-17** | **49.65** |
| % of inhibition: 100-(CPM with Abs-PBMC alone-CHO-CD40L alone)/(CPM of PBMC+CHO-CD40L-PBMCPBMC alone-CCHO-CD40L alone)*100% | | | | | | | | | | |

In addition to B cells, human PBMC also contain natural killer cells that can mediate antibody dependent cytotoxicity (ADCC). To clarify the mechanism of antibody-mediated inhibition of proliferation, assays were performed with B cells purified from human PBMC. Similar to results obtained with PBMC, both antibodies potently inhibited the CD40 ligand-induced proliferation of purified B cells (Table 5, n=3). These data demonstrate that the antagonist activity of the candidate antibodies, and not the mechanism of ADCC, is the cause of proliferation inhibition in these assays.

**Table 5. Effect of anti-CD40 antibodies on CD40 ligand-induced proliferation of purified human B cells**

| | | **CPM** | | | | **HuIgG1** | | **CHIR-5.9** | | **CHIR-12.12** | |
|---|---|---|---|---|---|---|---|---|---|---|---|
| **Exp#** | **Donor #** | **B cells** | **CHO-CD40L** | **B. cells + CHO-CD40L** | **Abs Conc(*µ*g/ml)** | **CPM** | **% inhibition** | **CPM** | **% inhibition** | **CPM** | **% inhibition** |
| B cell-004 | 1 | 418 | 89 | 3132 | 100 | 429 | **103** | 271 | **109** | 152 | **114** |
| | | 418 | 89 | 3132 | 20 | 3193 | **-2** | 316 | **107** | 222 | **111** |
| | | 418 | 89 | 3132 | 4 | 3175 | **-2** | 144 | **114** | 235 | **110** |
| | | 418 | 89 | 3132 | 0.8 | 6334 | **-122** | 245 | **110** | 63 | **117** |
| | 2 | 81 | 73 | 27240 | 100 | 28311 | **-4** | 85 | **100** | 77 | **100** |
| | | 81 | 73 | 27240 | 20 | 24707 | **9** | 65 | **100** | 94 | **100** |
| | | 81 | 73 | 27240 | 4 | 23081 | **15** | 108 | **100** | 68 | **100** |
| | | 81 | 73 | 27240 | 0.8 | 26252 | **4** | 87 | **100** | 77 | **100** |
| B cell-005 | 3 | 267 | 75 | 24552 | 1 | 25910 | **-6** | 291 | **100** | 102 | **101** |
| | | 267 | 75 | 24552 | 0.1 | 28447 | **-16** | 259 | **100** | 108 | **101** |
| | | 267 | 75 | 24552 | 0.01 | 26706 | **-9** | 2957 | **89** | 4922 | **81** |
| **Average % inhibition** | | | | | | | **-3** | | **103** | | **103** |

| | | | | | | | | | | | |
|---|---|---|---|---|---|---|---|---|---|---|---|
| % of inhibition: 100-(CPM with Abs-B cells alone-CHO-CD40L alone)/( CPM of B cell with CHO-CD40LB cells alone-CHO-CD40L alone)* 100% | | | | | | | | | | | |

### Example 7: CHIR-5.9 and CHIR-12.12 Do Not Induce Strong Proliferation of Human B Cells from Normal Subjects

CD40 ligand activates normal B cells and B-cell lymphoma cells to proliferate. Binding of some anti-CD40 antibodies (agonist) can provide a similar stimulatory signal for the proliferation of normal and cancer B cells. Antibodies with strong B cell stimulatory activity are not suitable candidates for therapeutic treatment of B cell lymphomas. The two candidate antibodies were tested for their ability to induce proliferation of B cells from normal volunteer donors. The B cells purified by Ficoll-Hypaque Plus gradient centrifugation from normal donor PBMC were cultured in 96-well plates with varying concentrations of candidate antibodies (range of 0.001 to 100 µg/ml) for a total of 4 days. In the positive control group, PBMC were cultured with formaldehyde-fixed CHO cells expressing CD40-ligand. The B cell proliferation was measured by incorporation of tritiated-labeled thymidine at 37°C for 14-18 hours. While the CD40 ligand presented on CHO cells induced vigorous proliferation of B cells resulting in an average stimulation index (SI) of 145, the candidate antibodies induced only a weak proliferation with a stimulation index of 2.89 and 5.08 for CHIR-12.12 and CHIR-5.9, respectively (n=3) (Table 6).

**Table 6. Proliferation of B cells purified from normal human subjects in response to candidate anti-CD40 mAbs**

| | | **B cells** | **Bcells+CHO-CD40L** | | | **B cells+hulpGl** | | **B cells+CHIR-5.9** | | **B cells+CHIR-12.12** | |
|---|---|---|---|---|---|---|---|---|---|---|---|
| **Exp#** | **Donor#** | **CPM** | **CPM** | **S. index(1)** | **Abs conc (µg/ml)** | **CPM** | **S. index(2)** | **CPM** | **S. index (2)** | **CPM** | **S. index(2)** |
| B cell-004 | 1 | 418 | 3132 | **7.49** | 100 | 498 | **1.19** | 401 | **0.96** | 458 | **1.10** |
| Frozen | | 418 | 3132 | | 20 | 245 | **0.59** | 232 | **0.56** | 370 | **0.89** |
| | | 418 | 3132 | | 4 | 241 | **0.58** | 232 | **0.56** | 211 | **0.50** |
| | | 418 | 3132 | | 0.8 | 376 | **0.90** | 298 | **0.71** | 230 | **0.55** |
| Frozen | 2 | 81 | 27240 | **336.30** | 100 | 34 | **0.42** | 454 | **5.60** | 122 | **1.51** |
| | | 81 | 27240 | | 20 | 48 | **0.59** | 706 | **8.72** | 255 | **3.15** |
| | | 81 | 27240 | | 4 | 41 | **0.51** | 567 | **7.00** | 367 | **4.53** |
| | | 81 | 27240 | | 0.8 | 34 | **0.42** | 736 | **9.09** | 408 | **5.04** |
| B cell-005 | 3 | 267 | 24552 | **91.96** | 1 | 691 | **2.59** | 2101 | **7.87** | 1223 | **4.58** |
| | | 267 | 24552 | | 0.1 | 686 | **2.57** | 2267 | **8.49** | 1557 | **5.83** |
| | | 267 | 24552 | | 0.01 | 808 | **3.03** | 2203 | **8.25** | 1027 | **3.85** |
| | | 267 | 24552 | | 0.001 | 567 | **2.12** | 846 | **3.17** | 826 | **3.09** |
| **Average Stimulation Index (SI) 145.25** | | | | | | | **1.29** | | **5.08** | | **2.88** |
| S. index(1): = CPM (B cells+CHO-CD40L)/CPM (B cells alone) | | | | | | | | | | | |
| S. index(2): = CPM (B cells+Abs)/CPM (PBMC alone) | | | | | | | | | | | |

In addition to B cells, human PBMC contain cell types that bear Fc receptors (FcR) for IgG1 molecules that can provide cross linking of anti-CD40 antibodies bound to CD40 on B cells. This cross-linking could potentially enhance stimulatory activity of anti-CD40 antibodies. To confirm the lack of B cell stimulatory activity of CHIR-5.9 and CHIR-12.12 antibodies in the presence of cross-linking cells, proliferation experiments were performed with total PBMC containing B cells as well as FcR+ cells. Data from these experiments (Table 7A, mAb CHIR-5.9; Table 7B, mAb CHIR-12.12) confirm that these candidate antibodies even in the presence of FcR-bearing cells in general do not stimulate B cells to proliferate over background proliferation induced by control human IgG1 (n=10). The CD40 ligand induced an average stimulation index (SI) SI of 7.41. The average SI with candidate antibodies were 0.55 and 1.05 for CHIR-12.12 and CHIR-5.9, respectively. Only one of the 10 donor PBMC tested showed some stimulatory response to CHIR-5.9 antibody (donor #2 in Table 7). The lack of stimulatory activity by candidate mAbs was further confirmed by measuring the PBMC proliferation in response to candidate anti-CD40 antibodies immobilized on the plastic

**Table 7A. Proliferation of PBMC from normal human subjects in response to mAb CHIR-5.9.**

| | | | **PBMC+CHO-CD40L** | | **Abs conc (*µ*g/m 1)** | **PBMC+hulgG1** | | **PBMC+CHIR-5.9** | |
|---|---|---|---|---|---|---|---|---|---|
| **Exp#** | | **PBM C** | **CPM** | **index(1)** | | **CPM** | **index 2)** | **(CP M** | **index(2)** |
| PBMC-010 | | 1417 | 5279 | **3.73** | 1 | 1218 | **0.86** | 597 3 | **4.22** |
| | | 1417 | 5279 | | 0.25 | 1712 | **1.21** | 481 5 | **3.40** |
| | | 1417 | 5279 | | 0.062 5 | 1449 | **1.02** | 364 2 | **2.57** |
| | | 1417 | 5279 | | 0.015 6 | 1194 | **0.84** | 324 2 | **2.29** |
| PBMC-011 | Donor#1 | 2138 | 8247 | **3.86** | 10 | 3047 | **1.43** | 317 7 | **1.49** |
| | | 2138 | 8247 | | 1 | 2726 | **1.28** | 361 7 | **1.69** |
| | | 2138 | 8247 | | 0.1 | 2026 | **0.95** | 201 1 | **0.94** |
| | | 2138 | 8247 | | 0.01 | 2424 | **1.13** | 186 0 | **0.87** |
| | Donor#2 | 2374 | 1156 1 | **4.87** | 10 | 4966 | **2.09** | 452 3 | **1.91** |
| | | 2374 | 1156 1 | | 1 | 2544 | **1.07** | 244 5 | **1.03** |
| | | 2374 | 1156 1 | | 0.1 | 2177 | **0.92** | 146 2 | **0.62** |
| | | 2374 | 1156 1 | | 0.01 | 4672 | **1.97** | 189 6 | **0.80** |
| | Donor#3 | 3229 | 7956 | **2.46** | 10 | 5035 | **1.56** | 211 9 | **0.66** |
| | | 3229 | 7956 | | 1 | 2826 | **0.88** | 109 9 | **0.34** |
| | | 3229 | 7956 | | 0.1 | 2277 | **0.71** | 105 2 | **0.33** |
| | | 3229 | 7956 | | 0.01 | 3078 | **0.95** | 189 9. | **0.59** |
| | Donor#4 | 4198 | 1431 4 | **3.41** | 10 | 5012 | **1.19** | 517 6 | **1.23** |
| | | 4198 | 1431 4 | | 1 | 3592 | **0.86** | 470 2 | **1.12** |
| | | 4198 | 1431 4 | | 0.1 | 5298 | **1.26** | 431 9 | **1.03** |
| | | 4198 | 1431 4 | | 0.01 | 5758 | **1.37** | 540 0 | **1.29** |
| PBMC-014 | | 2350 | 8787 | **3.74** | 100 | 2722 | **1.16** | 247 1 | **1.05** |
| | | 2350 | 8787 | | 20 | 2315 | **0.99** | 244 7 | **1.04** |
| | | 2350 | 8787 | | 4 | 2160 | **0.92** | 165 9 | **0.71** |
| | | 2350 | 8787 | | . 0.8 | 2328 | **0.99** | 167 1 | **0.71** |
| PBMC-015 | Donor#1 | 3284 | 1293 6 | **3.94** | 100 | 3598 | **1.10** | 168 2 | **0.51** |
| | | 3284 | 1293 6 | | 20 | 2751 | **0.84** | 156 2 | **0.48** |
| | | 3284 | 1293 6 | | 4 | 3135 | **0.95** | 110 5 | **0.34** |
| | | 3284 | 1293 6 | | 0.8 | 4027 | **1.23** | 141 9 | **0.43** |
| | Donor#2 | 6099 | 1912 1 | **3.14** | 100 | 2999 | **0.49** | 510 4 | **0.84** |
| | | 6099 | 1912 1 | | 20 | 4025 | **0.66** | 391 7 | **0.64** |
| | | 6099 | 1912 1 | | 4 | 4496 | **0.74** | 334 1 | **0.55** |
| | | 6099 | 1912 1 | | 0.8 | 3834 | **0.63** | 413 9 | **0.68** |
| | Donor#3 | 2479 | 1982 6 | **8.00** | 100 | 3564 | **1.44** | 120 4 | **0.49** |
| | | 2479 | 1982 6 | | 20 | 1874 | **0.76** | 782 | **0.32** |
| | | 2479 | 1982 6 | | 4 | 1779 | **0.72** | 634 | **0.26** |
| | | 2479 | 1982 6 | | 0.8 | 2274 | **0.92** | 937 | **0.38** |
| PBMC-016 | | 1148 | 1578 9 | **13.75** | 0.1 | 1255 | **1.09** | 103 6 | **0.90** |
| | | 1148 | 1578 9 | | 0.05 | 1284 | **1.12** | 871 | **0.76** |
| | | 1148 | 1578 9 | | 0.01 | 1446 | **1.26** | 952 | **0.83** |
| **Average SI of PBMC** | | | | **5.09** | | | **1.06** | | **1.03** |

| | | | | | | | | | |
|---|---|---|---|---|---|---|---|---|---|
| index(1): =(PBMC+CHO-CD40L)/PBMC index(2): =(PBMC+Abs)/PBMC | | | | | | | | | |

**Table 7B. Proliferation of PBMC from normal human subjects in response to mAb CHIR-12.12.**

| | | | **PBMC+CHO-CD40L** | | **Abs conc** | **PBMC+hulgG1** | | **PBMC+CHIR-12.12** | |
|---|---|---|---|---|---|---|---|---|---|
| **Exp#** | | **PBMC** | **CPM** | **index(1)** | **(*µ*g/ml)** | **CPM** | **index(2)** | **CPM** | **index(2)** |
| PBMC-025 | Donor#1 | 4051 | 42292 | **10.44** | 0.1 | 2909 | **0.72** | 2451 | **0.61** |
| | | 4051 | 42292 | | 0.01 | 4725 | **1.17** | 8924 | **2.20** |
| | | 4051 | 42292 | | 0.001 | 8080 | **1.99** | 8782 | **2.17** |
| | | 4051 | 42292 | | 0.0001 | 4351 | **1.07** | 4342 | **1.07** |
| | Donor#2 | 2260 | 14987 | **6.63** | 0.1 | 2538 | **1.12** | 6741 | **2.98** |
| | | 2260 | 14987 | | 0.01 | 3524 | **1.56** | 8921 | **3.95** |
| | | 2260 | 14987 | | 0.001 | 3159 | **1.40** | 4484 | **1.98** |
| | | 2260 | 14987 | | 0.0001 | 2801 | **1.24** | 2533 | **1.12** |
| PBMC-026 | Donor#1 | 2085 | 18313 | **8.78** | 0.1 | 1386 | **0.66** | 2761 | **1.32** |
| | | 2085 | 18313 | | 0.01 | 2871 | **1.38** | 3162 | **1.52** |
| | | 2085 | 18313 | | 0.001 | 2602 | **1.25** | 3233 | **1.55** |
| | | 2085 | 18313 | | 0.0001 | 1709 | **0.82** | 1766 | **0.85** |
| | Donor#2 | 676 | 18054 | **26.71** | 0.1 | 660 | **0.98** | 2229 | **3.30** |
| | | 676 | 18054 | | 0.01 | 2864 | **4.24** | 1238 | **1.83** |
| | | 676 | 18054 | | 0.001 | 693 | **1.03** | 1507 | **2.23** |
| | | 676 | 18054 | | 0.0001 | 984 | **1.46** | 811 | **1.20** |
| PBMC-027 | Donor#1 | 2742 | 13028 | **4.75** | 0.1 | 4725 | **1.72** | 2795 | **1.02** |
| | | 2742 | 13028 | | 0.01 | 4575 | **1.67** | 5353 | **1.95** |
| | | 2742 | 13028 | | 0.001 | 3218 | **1.17** | 3501 | **1.28** |
| | | 2742 | 13028 | | 0.0001 | 5107 | **1.86** | 4272 | **1.56** |
| | Donor#2 | 1338 | 11901 | **8.89** | 0.1 | 1633 | **1.22** | 1943 | **1.45** |
| | | 1338 | 11901 | | 0.01 | 1520 | **1.14** | 5132 | **3.84** |
| | | 1338 | 11901 | | 0.001 | 1517 | **1.13** | 2067 | **1.54** |
| | | 1338 | 11901 | | 0.0001 | 1047 | **0.78** | 2076 | **1.55** |
| PBMC-028 | Donor#1 | 4288 | 22547 | **5.26** | 0.1 | 3686 | **0.86** | 2525 | **0.59** |
| | | 4288 | 22547 | | 0.01 | 3113 | **0.73** | 2047 | **0.48** |
| | | 4288 | 22547 | | 0.001 | 4414 | **1.03** | 3515 | **0.82** |
| | | 4288 | 22547 | | 0.0001 | 2452 | **0.57** | 4189 | **0.98** |
| | Donor#2 | 2148 | 54894 | **25.56** | 0.1 | 9127 | **4.25** | 5574 | **2.59** |
| | | 2148 | 54894 | | 0.01 | 4566 | **2.13** | 6515 | **3.03** |
| | | 2148 | 54894 | | 0.001 | 5285 | **2.46** | 5919 | **2.76** |
| | | 2148 | 54894 | | 0.0001 | 4667 | **2.17** | 4298 | **2.00** |
| PBMC-029 | Donor#1 | 609 | 10054 | **16.51** | 0.1 | 359 | **0.59** | 363 | **0.60** |
| | | 609 | 10054 | | 0.01 | 473 | **0.78** | 956 | **1.57** |
| | | 609 | 10054 | | 0.001 | 461 | **0.76** | 1159 | **1.90** |
| | | 609 | 10054 | | 0.0001 | 625 | **1.03** | 558 | **0.92** |
| | Donor#2 | 7737 | 23132 | **2.99** | 0.1 | 4940 | **0.64** | 3493 | **0.45** |
| | | 7737 | 23132 | | 0.01 | 6041 | **0.78** | 3644 | **0.47** |
| | | 7737 | 23132 | | 0.001 | 5098 | **0.66** | 5232 | **0.68** |
| | | 7737 | 23132 | | 0.0001 | 5135 | **0.66** | 5241 | **0.68** |
| PBMC-030 | Donor#1 | 4164 | 57205 | **13.74** | 10 | 2713 | **0.65** | 1046 | **0.25** |
| | | 4164 | 57205 | | 1 | 3627 | **0.87** | 1576 | **0.38** |
| | | 4164 | 57205 | | 0.1 | 4590 | **1.10** | 1512 | **0.36** |
| | | 4164 | 57205 | | 0.01 | 4384 | **1.05** | 2711 | 0.65 |
| | Donor#2 | 3324 | 53865 | **16.20** | 10 | 6376 | **1.92** | 4731 | **1.42** |
| | | 3324 | 53865 | | 1 | 4720 | **1.42** | 5219 | **1.57** |
| | | 3324 | 53865 | | 0.1 | 3880 | **1.17** | 5869 | **1.77** |
| | | 3324 | 53865 | | 0.01 | 3863 | **1.16** | 5657 | **1.70** |
| PBMC-032 | Donor#1 | 1808 | 15271 | **8.45** | 10 | 2349 | **1.30** | 4790 | **2.65** |
| | | 1808 | 15271 | | 1 | 3820 | **2.11** | 5203 | **2.88** |
| | | 1808 | 15271 | | 0.1 | 2098 | **1.16** | 6332 | **3.50** |
| | | 1808 | 15271 | | 0.01 | 1789 | **0.99** | 5005 | **2.77** |
| **Average SI of PBMC** | | | | **11.92** | | | **1.30** | | **1.62** |

| | | | | | | | | | |
|---|---|---|---|---|---|---|---|---|---|
| index(1): =CPM of (PBMC+CHO-CD40L)/CPM of PBMC index(2): =CPM of (PBMC+Abs)/CPM of PBMC | | | | | | | | | |

surface of the culture wells (n=2). The average SI with CD40 ligand, CHIR-12.12, and CHIR-5.9 stimulation were 22, 0.67, and 1.2, respectively (Table 8). Taken together these data show that the candidate antiCD40 antibodies do not possess strong B cell stimulatory properties.

**Table 8. Proliferation of PBMC from normal human subjects in response to immobilized anti-CD40 antibodies**

| | **PBMC** | **PBMC+CHO-CD40L** | | | **PBMC+huIgG1** | | **PBMC+CHIR-5.9** | | **PBMC+CHIR-12.12** | |
|---|---|---|---|---|---|---|---|---|---|---|
| **Exp#** | **CPM** | **CPM** | **S.index (1)** | **Abs conc (ug/ml)** | **CPM** | **S. index(2)** | **CPM** | **S.index (2)** | **CPM** | **S.index (2)** |
| PBMC-012 | 225 | 6808 | **30.26** | 10 | 279 | **1.24** | 734 | **3.26** | 200 | **0.89** |
| | 225 | 6808 | | 1 | 175 | **0.78** | 178 | **0.79** | 161 | **0.72** |
| | 225 | 6808 | | 0.1 | 156 | **0.69** | 226 | **1.00** | 249 | **1.11** |
| | 225 | 6808 | | 0.01 | 293 | **1.30** | 232 | **1.03** | 254 | **1.13** |
| Immoblize-004 | 857 | 11701 | **13.65** | 1000 | 479 | **0.56** | 1428 | **1.67** | 384 | **0.45** |
| | 857 | 11701 | | 100 | 543 | **0.63** | 839 | **0.98** | 265 | **0.31** |
| | 857 | 11701 | | 10 | 487 | **0.57** | 411 | **0.48** | 262 | **0.31** |
| | 857 | 11701 | | 1 | 632 | **0.74** | 372 | **0.43** | 376 | **0.44** |
| **Average Stimulation index** | **21.96** | | | | **0.81** | | | **1.21** | | **0.67** |

| | | | | | | | | | | |
|---|---|---|---|---|---|---|---|---|---|---|
| S. index (1): =CPM (PBMC+CHO-CD40L)/CPM (PBMC) S. index (2): = CPM (PBMC+mAbs)/CPM (PBMC) | | | | | | | | | | |

### Example 8: CHIR-5.9 and CHIR-12.12 Are Able to Kill CD40-Bearing Target Cells by ADCC

The candidate antibodies can kill CD40-bearing target cells (lymphoma lines) by the mechanism of ADCC. Both CHIR-5.9 and CHIR-12.12 are fully human antibodies of IgG1 isotype and are expected to have the ability to induce the killing of target cells by the mechanism of ADCC. They were tested for their ability to kill cancer cell lines in *in vitro* assays. Two human lymphoma cell lines (Ramos and Daudi) were selected as target cells for these assays. PBMC or enriched NK cells from 8 normal volunteer donors were used as effector cells in these assays. A more potent ADCC response was observed with CHIR-12.12 compared with CHIR-5.9 against both the lymphoma cancer cell line target cells. Lymphoma cell lines also express CD20, the target antigen for rituximab (Rituxan®), which allowed for comparison of the ADCC activity of these two candidate mAbs with rituximab ADCC activity. For lymphoma cell line target, an average specific lysis of 35%, 59%, and 47% was observed for CHIR-5.9, CHIR-12.12, and rituximab respectively when used at 1 µg/ml concentration (Table 9). The two antibodies did not show much activity in complement dependent cytotoxicity (CDC) assays.

**Table 9. Anti-CD40 mAB dependent killing of lymphoma cell lines by ADCC.**

| **Anti-CD40 mAb dependent killing of lymphoma cell lines by ADCC** | | | | | | | | | | |
|---|---|---|---|---|---|---|---|---|---|---|
| Exp# | **Effector cell** | **E:T ratio** | **Target cells: Human lymphoma cell line (Ramos or Daudi)** | | | | | | | |
| | | | **% lysis IgG1** | Abs conc(µg/ml) | **CHIR-5.9** | | **CHIR-12.12** | | **Rituxan** | |
| | | | | | **% lysis** | **% lysis-% lysis IgG1** | **% lysis** | **% lysis-% lysis IgG1** | **% lysis** | **% lysis-% lysis IgG1** |
| ADCC-005 | huNK | 3 | 17.05 | 5 | 30.75 | **13.70** | 65.22 | **48.17** | ND | ND |
| Alarmor Blue | huNK | 3 | 40.81 | 5 | 58.62 | **17.81** | 87.87 | **47.06** | ND | ND |
| ADCC-006 | huNK | 2 | -3.09 | 10 | 3.50 | **6.59** | 43.71 | **46.8** | 34.82 | **37.91** |
| Alarmor Blue | | | -8.62 | 1 | -10.10 | **-1.48** | 45.13 | **53.75** | 37.07 | **45.69** |
| | | | -11 | 0.1 | -14.80 | **-3.80** | 39.82 | **50.82** | 33.61 | **44.61** |
| | | | -4.54 | 0.01 | 2.53 | **7.07** | 50.07 | **54.61** | 28.49 | **33.03** |
| 51Cr | huNK | 5 | 1.5 | 10 | 32.09 | **30.59** | 47.24 | **45.742** | ND | ND |
| | | | 2.4 | 1 | 18.01 | **15.61** | 37.42 | **35.022** | ND | ND |
| | | | 2.5 | 0.1 | 14.67 | **12.17** | 37.63 | **35.131** | ND | ND |
| ADCC-009 | huNK | 10 | 2.32 | 5 | 66.20 | **63.88** | 97.70 | **95.38** | 86.2 | **83.88** |
| Calcein AM | | | 0.48 | 1 | 67.20 | **66.72** | 123.00 | **122.52** | 88.2 | **87.72** |
| | | | -1.43 | 0.2 | 78.40 | **79.83** | 118.00 | **119.43** | 88.8 | **90.23** |
| | | | 3.39 | 0.04 | 69.10 | **65.71** | 109.00 | **105.61** | 84.9 | **81.51** |
| ADCC-011 | huNK | 8 | 3.18 | 1 | 15.36 | **12.19** | 51.59 | **48.42** | 22.44 | **19.27** |
| Calcein AM | | | 4.58 | 0.01 | 7.39 | **2.81** | 46.80 | **42.22** | 14.68 | **10.10** |
| | | | 5.41 | 0.002 | 6.35 | **0.94** | 5.10 | **-0.31** | 9.58 | **4.16** |
| | | | 7.03 | 0.0004 | 7.76 | **0.73** | 5.99 | **-1.04** | 5.99 | **-1.04** |
| ADCC-012 | huNK | 10 | 13.34 | 10 | 73.31 | **59.97** | 117.80 | **104.46** | 50.75 | **37.41** |
| Calcein AM | | | 13.50 | 1 | 74.76 | **61.26** | 88.64 | **75.14** | 65.97 | **52.47** |
| | | | 12.27 | 0.01 | 58.52 | **46.25** | 72.88 | **60.61** | 50.16 | **37.89** |
| | | | 13.61 | 0.005 | 57.50 | **43.89** | 69.45 | **55.84** | 39.28 | **25.67** |
| | | | 11.95 | 0.001 | 56.81 | **44.86** | 65.17 | **53.22** | 33.07 | **21.12** |
| ADCC-013 | PBMC | 100 | 2.54 | 1 | 21.03 | **18.49** | 37.94 | **35.40** | 32.28 | **29.74** |
| 51Cr | | | 2.45 | 0.1 | 15.50 | **13.05** | 30.82 | **28.37** | 27.18 | **24.73** |
| | | | 2.92 | 0.01 | 14.53 | **11.61** | 22.59 | **19.67** | 12.79 | **9.87** |
| | | | 2.78 | 0.001 | 3.90 | **1.12** | 8.99 | **6.21** | 3.13 | **0.35** |
| ADCC-014 | PBMC | 100 | 4.64 | 10 | 53.54 | **48.90** | 56.12 | **51.48** | ND | ND |
| 51Cr | | | 4.64 | 1 | 46.84 | **42.20** | 43.00 | **38.36** | ND | ND |
| | | | 4.64 | 0.1 | 45.63 | **40.99** | 39.94 | **35.30** | ND | ND |
| | | | 4.64 | 0.01 | 7.73 | **3.09** | 9.79 | **5.15** | ND | ND |
| | | | 4.64 | 0.001 | 8.83 | **4.19** | 10.81 | **6.17** | ND | ND |
| Average % lysis at 1 *µ*g/ml concentration of mAbs | | | | | | **35.31** | | **59.03** | | **47.23** |
| * The greater than 100% killing are due to incomplete killing by detergent used for 100% killing control. | | | | | | | | | | |

### Example 9: CD40 Is Present on the Surface of NHL Cells from Lymph Node Biopsy Patients

NHL cells were isolated from biopsied lymph nodes from patients and were preserved in liquid nitrogen until use. Cell viability at the time of analysis exceeded 90%. The cells from two rituximab-sensitive and three rituximab-resistant patients (five patients in total) were stained with either a direct labeled 15B8-FITC or 15B8 plus anti-huIgG₂-FITC and analyzed by Flow cytometry. NHL cells from all the patients were found to express CD40. Table 10 shows that an average of 76% of NHL cells express CD40 (a range of 60-91%).

**Table 10.**

| **Patient ID^{a}** | **Patient type**^{b} | **% positive^{c}** | |
|---|---|---|---|
| | | **MS81**^{d} | **15B8**^{e} |
| B | CR | n.d.^{f} | 91.02 |
| J | CR | n.d. | 60.36 |
| H | NR | n.d. | 85.08 |
| H | NR | 72.24 | 81.19 |
| K | NR | n.d. | 70.69 |
| L | NR | n.d. | 66.82 |
| *Average % positive* | | *76* | |

| | | | |
|---|---|---|---|
| ***^{a} NHL patients treated with anti-CD20 mAb*** ***^{b} patient response to anti- CD20 mAb; CR=complete responder; NR=nonresponder*** ***^{c} % of cells in lymphocyte gate that stain positive*** ***^{d} MS81, agonist anti-CD40 mAb*** ***^{e} 15B8, antagonist anti-CD40 Mab*** ***^{f} n.d., not done*** | | | |

### Example 10: CHIR-5.9 and CHIR-12.12 Do Not Stimulate Proliferation of Cancer Cells from the Lymph Nodes of NHL Patients

CD40 ligand is known to provide a stimulatory signal for the survival and proliferation of lymphoma cells from NHL patients. Binding of some anti-CD40 antibodies (agonist) can provide a similar stimulatory signal for the proliferation of patient cancer cells. Antibodies with strong B cell stimulatory activity are not suitable candidate for therapeutic treatment of B cell lymphomas. The two candidate antibodies were tested for their ability to induce proliferation of NHL cells from 3 patients. The cells isolated from lymph node (LN) biopsies were cultured with varying concentrations of candidate antibodies (range of 0.01 1 to 300 µg/ml) for a total of 3 days. The cell proliferation was measured by incorporation of tritiated thymidine. Neither of the two candidate mAbs induced any proliferation of cancer cells at any concentration tested (Table 11). Antibodies even in the presence of exogenously added IL-4, a B cell growth factor, did not induce proliferation of NHL cells (tested in one of the thee patient cells. These results indicate that CHIR-5.9 and CHIR-12.12 are not agonist anti-CD40 antibodies and do not stimulate proliferation *in vitro* of NHL cells from patients.

**Table 11. Proliferation of cancer cells from LN ofNHL patients in response to candidate anti-CD40 mAbs**

| **Donor#** | **Abs conc(ug/ml)** | **CPM** | | **S. index** | **CPM cells + CHIR-12.12** | **S. index** | **CPM cells+MS81** | **S. index** |
|---|---|---|---|---|---|---|---|---|
| | | **Cells + IgG1** | **cells + CHIR-5.9** | **CHIR -5.9** | | **CHIR-12.12** | | **MS81** |
| PP | 0.01 | 180 | 203 | **1.23** | 133.67 | **0.74** | ND | **ND** |
| | 0.1 | 107.5 | 151.67 | **1.41** | 136 | **1.27** | ND | **ND** |
| | 1 | 130-67 | 206.67 | **1.58** | 197.33 | **1.51** | 179 | **1.37** |
| | 10 | 152.5 | 245 | **1.61** | 137.33 | **0.90** | 871.67 | **5.71** |
| | 100 | 137.67 | 332.33 | **2.41** | 157.33 | **1.14** | ND | **ND** |
| | 300 | 137.67 | 254.33 | **1.85** | 100.67 | **0.73** | ND | **ND** |
| MM | 0.01 | 165 | 180.33 | **1.09** | 124 | **0.75** | ND | **ND** |
| | 0.1 | 180.5 | 149.67 | **0.83** | 111.33 | **0.62** | ND | **ND** |
| | 1 | 62 | 109.67 | **1.77** | 104.67 | **1.69** | ND | **ND** |
| | 10 | 91.5 | 93.33 | **1.02** | 100 | **1.09** | 763 | **8.34** |
| | 100 | 123 | 173 | **1.41** | 105.33 | **0.86** | ND | **ND** |
| | 300 | 109 | 183.67 | **1.69** | 157 | **1.44** | ND | **ND** |
| BD (IL-4) | 0.01 | 1591.5 | 1623.67 | **1.02** | 1422 | **0.89** | ND | **ND** |
| | 0.1 | 1405 | 1281 | **0.91** | 1316.33 | **0.94** | ND | **ND** |
| | 1 | 1526 | 1352.33 | **0.89** | 1160 | **0.76** | 1508.33 | **0.99** |
| | 10 | 1450 | 1424 | **0.98** | 1244 | **0.86** | 4146.67 | **2.86** |
| | 100 | 1406.67 | 1497.67 | **1.06** | 1255.33 | **0.89** | ND | **ND** |
| | 300 | 1410.33 | 1466.67 | **1.04** | 1233 | **0.87** | ND | **ND** |

### Example 11: CHIR-5.9 and CHIR-12.12 Can Block CD40 Ligand-Mediated Proliferation of Cancer Cells from Non-Hodgkin's Lymphoma Patients

Engagement of CD40 by CD40 ligand induces proliferation of cancer cells from NHL patients. An antagonist anti-CD40 antibody is expected to inhibit this proliferation. The two candidate anti-CD40 antibodies were tested at varying concentrations (0.01 µg /ml to 100 µg /ml) for their ability to inhibit CD40 ligand-induced proliferation of NHL cells from patients. NHL cells from patients were cultured in suspension over CD40L-expressing feeder in the presence of IL-4. The NHL cell proliferation was measured by ³H-thymidine incorporation. Both antibodies were found to be very effective for inhibiting CD40 ligand-induced proliferation of NHL cells (Table 12, n=2). Nearly complete inhibition of CD40 ligand-induced proliferation could be achieved at 1.0 to 10 µg/ml concentration of antibodies.

**Table 12. Inhibition of CD40 ligand-induced proliferation of cancer cells from the LN of NHL patients.**

| | | **CPM** | **CHIR-5.9** | | **CHIR-12.12** | | **Rituximab** | |
|---|---|---|---|---|---|---|---|---|
| **Patient** | **Abs Conc (ug/ml)** | **IgG1** | **CPM** | **% inhibition** | **CPM** | **% inhibition** | **CPM** | **% inhibition** |
| BD | 0.01 | 29525.5 | 25369 | **14** | 24793 | **16** | 29490.3 | 0 |
| | 0.1 | 29554 | 20265.33 | **31** | 13671 | **54** | 29832.7 | -1 |
| | 1 | 29486.67 | 6785.33 | **77** | 453 | **98** | 26355.3 | 11 |
| | 10 | 29710 | 506.33 | **98** | 371 | **99** | 29427.3 | 1 |
| | 100 | 29372.33 | 512.33 | **98** | 386.67 | **99** | ND | ND |
| PP | 0.01 | 23572 | 23229.33 | **1** | 23666 | **0** | 25317.3 | -7 |
| | 0.1 | 22520 | 19092.33 | **15** | 17197 | **24** | 26349.7 | -17 |
| | 1 | 23535.67 | 1442.33 | **94** | 802.67 | **97** | 26515.7 | -13 |
| | 10 | 23101.5 | 608.67 | **97** | 221.33 | **99** | 25478.3 | -10 |
| | 100 | 23847.33 | ND | **ND** | 252 | **99** | ND | ND |

| | | | | | | | | |
|---|---|---|---|---|---|---|---|---|
| % inhibition: 100-(CPM with test Abs/CPM with control mAb) *100% | | | | | | | | |

### Example 12: Effect of CHIR-5.9 on Number of Viable NHL Cells When Cultured with CD40-Ligand Bearing Cells

Effects of CHIR-5.9 on the viable NHL cell numbers when cultured with CD40-ligand bearing cells over an extended period of time (days 7, 10, and 14) were investigated. CD40 ligand-mediated signaling through CD40 is important for B cell survival. This set of experiments evaluated the effect of anti-CD40 antibodies on NHL cell numbers at days 7, 10, and 14. NHL cells from five patients were cultured in suspension over CD40L-expressing irradiated feeder cells in the presence of IL-4. The control human IgG and CHIR-5.9 antibodies were added at concentrations of 10 µg/ml at day 0 and day 7. The viable cells under each condition were counted on the specified day. Cell numbers in the control group (IgG) increased with time as expected. Reduced numbers of cells were recovered from CHIR-5.9-treated cultures compared to control group. The greatest levels of reduction in cell numbers by CHIR-5.9 antibody were observed at day 14 and were on average 80.5% (a range of 49-94%) compared to isotype control (n=5). These data are summarized in Table 13.

**Table 13. Effect of anti-CD40 antibody (CHIR-5.9/5.11) on NHL patient cell numbers over prolonged culture period (day 7, 10, and 14)**

| **Patient** | **Days in culture** | **Viable cell numbers** | | **% reduction compared to IgG control** |
|---|---|---|---|---|
| | | **IgG** | **mAb CHIR-5.9/5.11** | |
| PS | 0 | 100000 0 | 1000000 | **0.00** |
| | 7 | 935000 | 447500 | **52.14** |
| | 10 | 127090 0 | 504100 | **60.34** |
| | 14 | 102910 0 | 525000 | **48.98** |
| MT | 0 | 100000 0 | 1000000 | **0.00** |
| | 7 | 267600 | 182500 | **31.80** |
| | 10 | 683400 | 191600 | **71.96** |
| | 14 | 145000 0 | 225000 | **84.48** |
| BRF | 0 | 250000 | 250000 | **0.00** |
| | 7 | 145000 | 86667 | **40.23** |
| | 10 | 207500 | 65000 | **68.67** |
| | 14 | 570500 | 33330 | **94.16** |
| DP | 0 | 250000 | 250000 | **0.00** |
| | 7 | 188330 | 136670 | **27.43** |
| | 10 | 235000 | 128330 | **45.39** |
| | 14 | 428330 | 58330 | **86.38** |
| PP | 0 | 250000 | 250000 | **0.00** |
| | 7 | 270000 | 176670 | **34.57** |
| | 10 | 311670 | 128330 | **58.83** |
| | 14 | 458330 | 53330 | **88.36** |

| | | | | |
|---|---|---|---|---|
| * % reduction compare to ctrl Abs=100-(test Abs/ctrl Abs)*100 | | | | |

### Example 13: CHIR-5.9 and CHIR-12-12 Are Able to Kill Cancer Cells from the Lymph Nodes of NHL Patients by ADCC

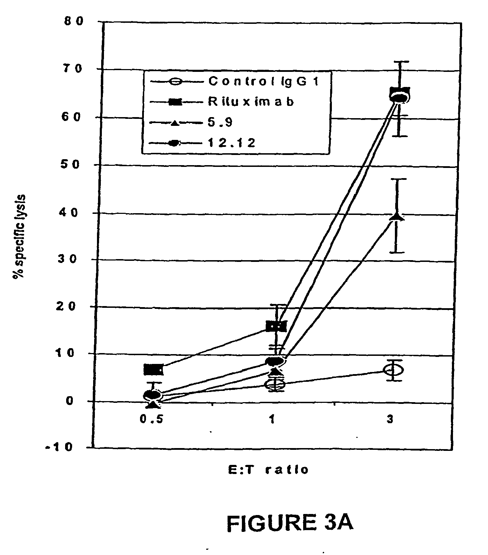

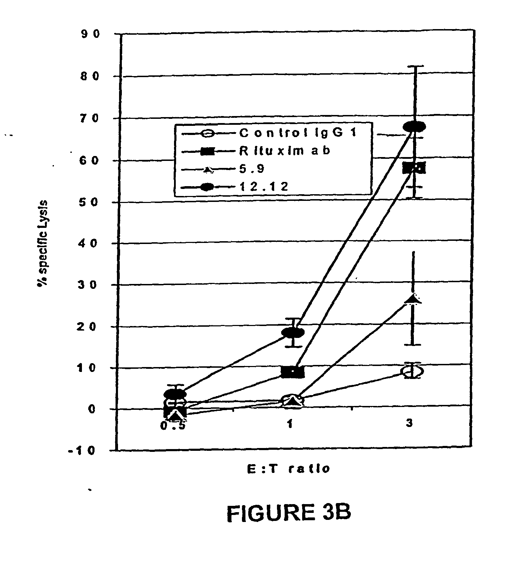

Both CHIR-5.9 and CHIR-12.12 are fully human antibodies of IgG₁ isotype and were shown to induce the killing of lymphoma cell lines *in vitro* by the mechanism of ADCC (Table 9). They were tested for their ability to kill cancer cells from a single NHL patient in *in vitro* assays. Enriched NK cells from normal volunteer donor either fresh after isolation or after culturing overnight at 37°C were used as effector cells in this assay. Similar results were obtained with both freshly isolated NK cells and NK cells used after overnight culture. The higher level of ADCC was observed with CHIR-12.12 compared with CHIR-5.9 against the NHL cells from the patient. NHL cells also express CD20, the target antigen for rituximab (Rituxan®), which allowed for comparison of the ADCC activity of these two candidate mAbs with rituximab. Antibody CHIR-12.12 and rituximab show similar level of ADCC activity with CHIR-5.9 scoring lower in this assay. These data are shown in Figures 3A and 3B.

### Example 14: CHIR-5.9 and CHIR-12.12 Can Block CD40-Mediated Survival and Proliferation of Cancer Cells from CLL Patients

The candidate antibodies can block CD40-mediated survival and proliferation of cancer cells from CLL patients. CLL cells from patients were cultured in suspension over CD40L-expressing formaldehyde-fixed CHO cells under two different conditions: addition of human isotype antibody IgG (control); and addition of either CHIR-5.9 or CHIR-12.12 monoclonal antibody. All antibodies were added at concentrations of 1, 10, and 100 µg/mL in the absence of IL-4. The cell counts were performed at 24 and 48 h by MTS assay. Reduced numbers of cells were recovered from CHIR-5.9- (n=6) and CHIR-12.12- (n=2) treated cultures compared to control group. The greater differences in cell numbers between anti-CD40 mAb-treated and control antibody-treated cultures were seen at the 48-h time point. These data are summarized in Table 14.

**Table 14. The effect of candidate antibodies on CD40-induced survival and proliferation of cancer cells from CLL patients measured at 48 h after the culture initiation**

| **Patient#** | **Ab conc(µg/ml)** | **Relative cell numbers** | | | % **reduction in cell numbers*** | |
|---|---|---|---|---|---|---|
| | | IgG1 | CHIR-5.9/5.11 | CHIR-12.12 | **CDR-5.9/5.11** | **CHIR-12.12** |
| 1 | 1 | 269.3 1 | 25.27 | ND | **90.62** | ND |
| | 10 | 101.5 8 | 33.07 | ND | **67.44** | ND |
| | 100 | 130.7 1 | 40.16 | ND | **69.28** | ND |
| 2 | 1 | 265.5 5 | 75.8 | ND | **71.46** | ND |
| | 10 | 227.5 7 | 128.5 | ND | **43.53** | ND |
| | 100 | 265.9 9 | 6.4 | ND | **97.59** | ND |
| 3 | 1 | 85.9 | 35.39 | ND | **58.80** | ND |
| | 10 | 70.44 | 39.51 | ND | **43.91** | ND |
| | 100 | 77.65 | 20.95 | ND | **73.02** | ND |
| 4 | 1 | 80.48 | 15.03 | ND | **81.32** | ND |
| | 10 | 63.01 | 19.51 | ND | **69.04** | ND |
| | 100 | 55.69 | 3.65 | ND | **93.45** | ND |
| 5 | 1 | 90.63 | 91.66 | 89.59 | **-1.14** | **1.15** |
| | 10 | 78.13 | 82.28 | 60.41 | **-5.31** | **22.68** |
| | 100 | 63.53 | 86.47 | 39.59 | **-36.11** | **37.68** |
| 6 | 1 | 130.2 1 | 77.6 | 71.88 | **40.40** | **44.80** |
| | 10 | 131.7 7 | 78.13 | 73.96 | **40.71** | **43.87** |
| | 100 | 127.0 8 | 76.56 | 82.29 | **39.75** | **35.25** |

| | | | | | | |
|---|---|---|---|---|---|---|
| * % reduction compared to control Abs=100-(test Abs/control Abs)*100 | | | | | | |

### Example 15: CHIR-5.9 and CHIR-12.12 Show Anti-Tumor Activity in Animal Models Pharmacology/in vivo efficacy

The candidate mAbs are expected to produce desired pharmacological effects to reduce tumor burden by either/both of two anti-tumor mechanisms, blockade of proliferation/survival signal and induction of ADCC. The currently available human lymphoma xenograft models use long-term lymphoma cell lines that, in contrast to primary cancer cells, do not depend on CD40 stimulation for their growth and survival. Therefore the component of these mAbs' anti-tumor activity based on blocking the tumor proliferation/survival signal is not expected to contribute to anti-tumor efficacy in these models. The efficacy in these models is dependent on the ADCC, the second anti-tumor mechanism associated with the CHIR-5.9 and CHIR-12.12 mAbs. Two xenograft human lymphoma models based on Namalwa and Daudi cell lines were assessed for anti-tumor activities of candidate mAbs. To further demonstrate their therapeutic activity, these candidate mAbs were evaluated in an unstaged and staged xenograft human lymphoma model based on the Daudi cell line.

### Summary of in vivo efficacy data

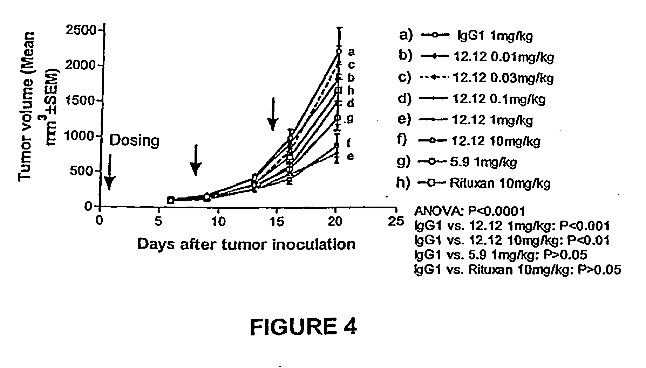

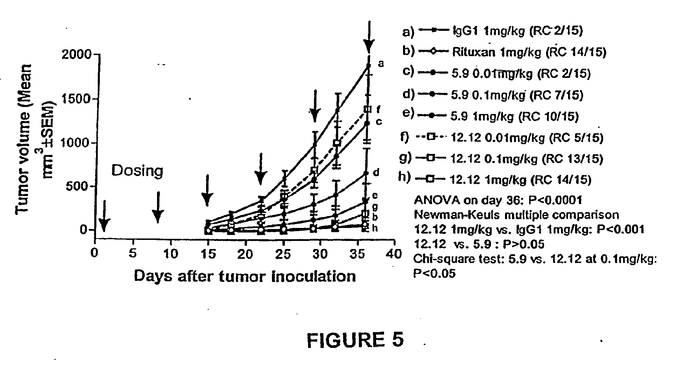

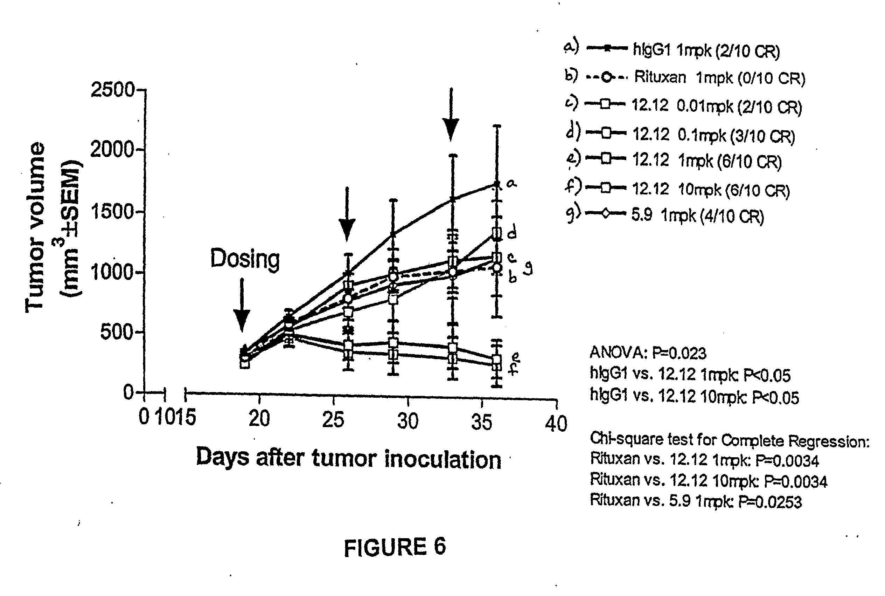

When administered intraperitoneally (i.p.) once a week for a total of 3 doses, CHIR-12.12, one of the two candidate mAbs, significantly inhibited the growth of aggressive unstaged B cell lymphoma (Namalwa) in a dose-dependent manner (Figure 4). The second candidate mAb, CHIR-5.9, was tested only at a single dose in this study and was less effective than CHIR-12.12 at the same dose. Interestingly, CHIR-12.12 was found to be more efficacious in this model than rituximab. It is possible that lower efficacy by rituximab could be due to low CD20 expression on the Namalwa lymphoma cell line. The efficacy observed with candidate mAbs has greater importance because only one of the two cancer cell killing mechanisms (ADCC) is operative in the current xenograft lymphoma model. Two killing mechanisms, ADCC and blocking of survival signal, are expected to contribute to anti-tumor activities in human lymphoma patients. This is likely to increase the chance of achieving efficacy in human lymphoma patients. The candidate anti-CD40 mAbs also showed a trend toward tumor growth inhibition in a second B cell lymphoma model (non-validated Daudi model, data not shown). In follow-up studies, the two candidate antibodies were shown to have dose-dependent anti-tumor efficacy in both the unstaged and staged Daudi lymphoma models (Figures 5 and 6, respectively). In the staged Daudi model, the CHIR-12.12 mAb was more efficacious at reducing tumor volume than was a similar dose of Rituxan®.

### Xenograft human B cell Lymphoma models

To ensure consistent tumor growth, T cell-deficient nude mice were whole-body irradiated at 3 Gy to further suppress the immune system one day before tumor inoculation. Tumor cells were inoculated subcutaneously in the right flank at 5x10⁶ cells per mouse. Treatment was initiated either one day after tumor implantation (unstaged subcutaneous xenograft human B cell lymphoma models, Namalwa and Daudi) or when tumor volume reached 200-400 mm³ (staged Daudi model, usually 15 days after tumor inoculation). Tumor-bearing mice were injected anti-CD40 mAbs intraperitoneally (i.p.) once a week at the indicated doses. Tumor volumes were recorded twice a week. When tumor volume in any group reached 2500 mm³, the study was terminated. Note that in the staged Daudi model, tumor volume data was analyzed up to day 36 due to the death of some mice after that day. Complete regression (CR) was counted until the end of the study. Data were analyzed using ANOVA or Kruskal-Wallis test and corresponding post-test for multi-group comparison.

In the unstaged Namalwa model, anti-CD40 mAb CHIR-12.12, but not Rituxan® (rituximab), significantly (p= <0.01) inhibited the growth of Namalwa tumors (tumor volume reduction of 60% versus 25% for rituxamab, n=10 mice/group) (Figure 4). Thus, in this model, anti-CD40 mAb CHIR-12.12 was more potent than rituximab. It is noteworthy that the second candidate mAb, CHIR-5.9, was at least as efficacious as rituximab at a dose 1/10^{th} that of rituximab. Both anti-CD40 mAb CHIR-12.12 and rituxan significantly prevented tumor development in the unstaged Daudi tumor model (14/15 resistance to tumor challenge) (Figure 5).

When these anti-CD40 monoclonal antibodies were further compared in a staged xenograft Daudi model, in which treatment started when the subcutaneous tumor was palpable, anti-CD40 mAb CHIR-12.12 at 1 mg/kg caused significant tumor reduction (p=0.003) with 60% complete regression (6/10), while rituximab at the same dose did not significantly inhibit the tumor growth nor did it cause complete regression (0/10). See Figure 6.

In summary, the anti-CD40 mAb CHIR-12.12 significantly inhibited tumor growth in experimental lymphoma models. At the same dose and regimen, mAb CHIR-12.12 showed better anti-cancer activity than did Rituxan® (rituximab). Further, no clinical sign of toxicity was observed at this dose and regimen. These data suggest that the anti-CD40 mAb CHIR-12.12 has potent anti-human lymphoma activity *in vitro* and in xenograft models and could be clinically effective for the treatment of lymphoma.

### Example 16: Pharmacokinetics of CHIR-5.9 and CHIR-12.12

The pharmacokinetics of anti-CD40 mAb in mice was studied after single IV and IP dose administration. Anti-CD40 mAb exhibited high systemic bioavailability after IP administration, and prolonged terminal half-life (>5 days) (data not shown). This pilot study was conducted to aid in the design of pharmacology studies; however, it is of little to no importance for the development activity of this mAb since this fully human mAb does not cross react with mouse CD40.

### Example 17: Characterization of Epitope for Monoclonal Antibodies CHIR-12.12 and CHIR-5.9

To determine the location of the epitope on CD40 recognized by monoclonal antibodies CHIR-12.12 and CHIR-5.9, SDS-PAGE and Western blot analysis were performed. Purified CD40 (0.5 µg) was separated on a 4-12% NUPAGE gel under reducing and non-reducing conditions, transferred to PVDF membranes, and probed with monoclonal antibodies at 10 µg/ml concentration. Blots were probed with alkaline phosphatase conjugated anti-human IgG and developed using the Western Blue^{R} stabilized substrate for alkaline phosphatase (Promega).

Results indicate that anti-CD40 monoclonal antibody CHIR-12.12 recognizes epitopes on both the non-reduced and reduced forms of CD40, with the non-reduced form of CD40 exhibiting greater intensity than the reduced form of CD40 (Table 15; blots not shown). The fact that recognition was positive for both forms of CD40 indicates that this antibody interacts with a conformational epitope part of which is a linear sequence. Monoclonal antibody CHIR-5.9 primarily recognizes the non-reduced form of CD40 suggesting that this antibody interacts with a primarily conformational epitope (Table 15; blots not shown).

**Table 15. Domain identification.**

| | Domain 1 | Domain 2 | Domain 3 | Domain 4 |
|---|---|---|---|---|
| mAb CHIR-12.12 | - | + | - | - |
| mAb CHIR-5.9 | - | + | - | - |
| mAb 15B8 | + | - | - | - |

To map the antigenic region on CD40, the four extracellular domains of CD40 were cloned and expressed in insect cells as GST fusion proteins. The secretion of the four domains was ensured with a GP67 secretion signal. Insect cell supernatant was analyzed by SDS-PAGE and western blot analysis to identify the domain containing the epitope.

Monoclonal antibody CHIR-12.12 recognizes an epitope on Domain 2 under both reducing and non-reducing conditions (Table 16; blots not shown). In contrast, monoclonal antibody CHIR-5.9 exhibits very weak recognition to Domain 2 (Table 16; blots not shown). Neither of these antibodies recognize Domains 1, 3, or 4 in this analysis.

**Table 16. Domain 2 analysis.**

| | Reduced | Non-reduced |
|---|---|---|
| mAb CHIR-12.12 | ++ | +++ |
| mAb CHIR-5.9 | + | + |

To define more precisely the epitope recognized by mAb CHIR-12.12, peptides were synthesized from the extracellular Domain 2 of CD40, which corresponds to the sequence PCGESEFLDTWNRETHCHQHKYCDPNLGLRVQQKGTSETDTICT (residues 61-104 of the sequence shown in SEQ ID NO:10 or SEQ ID NO:12). SPOTs membranes (Sigma) containing thirty-five 10mer peptides with a 1-amino-acid offset were generated. Western blot analysis with mAb CHIR-12.12 and anti-human IgG beta-galactosidase as secondary antibody was performed. The blot was stripped and reprobed with mAb CHIR-5.9 to determine the region recognized by this antibody

SPOTs analysis probing with anti-CD40 monoclonal antibody CHIR-12.12 at 10 µg/ml yielded positive reactions with spots 18 through 22. The sequence region covered by these peptides is shown in Table 17.

**Table 17. Results of SPOTs analysis probing with anti-CD40 monoclonal antibody CHIR-12.12.**

| **Spot Number** | **Sequence Region** | | | | |
|---|---|---|---|---|---|
| 18 | **HQHKYCDPNL** (residues 78-87 of SEQ ID NO:10 or SEQ ID NO:12) | | | | |
| 19 | | **QHKYCDPNL**G (residues 79-88 of SEQ ID NO:10 or SEQ ID NO:12) | | | |
| 20 | | | **HKYCDPNL**GL (residues 80-89 of SEQ ID NO:10 or SEQ ID NO:12) | | |
| 21 | | | | **KYCDPNL**GLR (residues 81-90 of SEQ ID NO:10 or SEQ ID NO:12) | |
| 22 | | | | | **YCDPNL**GLRV (residues 82-91 of SEQ ID NO:10 or SEQ ID NO:12) |

These results correspond to a linear epitope of: YCDPNL (residues 82-87 of the sequence shown in SEQ ID NO:10 or SEQ ID NO:12). This epitope contains Y82, D84, and N86, which have been predicted to be involved in the CD40-CD40 ligand interaction.

SPOTs analysis with mAb CHIR-5.9 showed a weak recognition of peptides represented by spots 20-22 shown in Table 18, suggesting involvement of the region YCDPNLGL (residues 82-89 of the sequence shown in SEQ ID NO:10 or SEQ ID NO:12) in its binding to CD40. It should be noted that the mAbs CHIR-12.12 and CHIR-5.9 compete with each other for binding to CD40 in BIACORE analysis.

**Table 18. Results of SPOTs analysis probing with anti-CD40 monoclonal antibody CHIR-5.9.**

| **Spot Number** | **Sequence Region** | | |
|---|---|---|---|
| 20 | **HKYCDPNLGL** (residues 80-89 of SEQ ID NO:10 or SEQ ID NO: 12) | | |
| 21 | | **KYCDPNLGLR** (residues 81-90 of SEQ ID NO:10 or SEQ ID NO:12) | |
| 22 | | | **YCDPNLGLRV** (residues 82-91 of SEQ ID NO:10 or SEQ ID NO:12) |

The linear epitopes identified by the SPOTs analyses are within the CD40 B1 module. The sequence of the CD40 B1 module is:
HKYCDPNLGLRVQQKGTSETDTIC (residues 80-103 of SEQ ID NO:10 or SEQ ID NO:12).

Within the linear epitope identified for CHIR-12.12 is C83. It is known that this cysteine residue forms a disulphide bond with C103. It is likely that the conformational epitope of the CHIR-12.12 mAb contains this disulfide bond (C83-C103) and/or surrounding amino acids conformationally close to C103.

### Example 18: Number of CD20 and CD40 Molecules on Namalwa and Daudi Cells

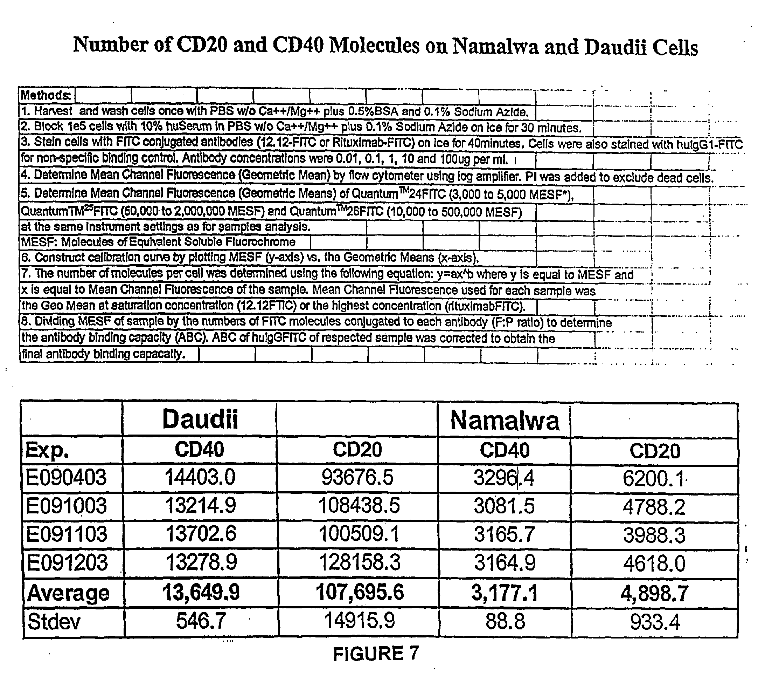

The number of CD20 and CD40 molecules on Namalwa and Daudi cells was determined as outlined in Figure 7, using antibody concentrations of 0.01, 0.1, 1, 10, and 100 µg/ml. As can be seen in Figure 7, the average number of CD20 molecules (target for rituximab) is greater on both the Namalwa and Daudi cell lines than is the number of CD40 molecules (target for mAb CHIR-12.12).

### Example 19: ADCC of mAb CHIR-12.12 and Rituximab Against Daudi Lymphoma Cells

The rituximab and candidate mAb CHIR-12.12 were tested *in vitro* for ADCC activity at variable concentrations against lymphoma cell line Daudi as target (T) cells and purified NK cells from healthy human volunteers as effector (E) cells. Freshly isolated human NK cells were mixed with calcein-labeled Daudi lymphoma cells at an E:T ratio of 10. The cell mixture was incubated for 4 hours at 37°C in the presence of the stated concentrations of either mAb CHIR-12.12 or rituximab. The calcein level released from lysed target cells in the supernatant was measured as Arbitrary Fluorescent Units (AFU). The percent specific lysis was calculated as: 100 x (AFU test - AFU spontaneous release)/(AFU maximal release - AFU spontaneous release), where AFU spontaneous release is the calcein released by target cells in the absence of antibody or NK cells, and AFU maximal release is the calcein released by target cells upon lysis by detergent.

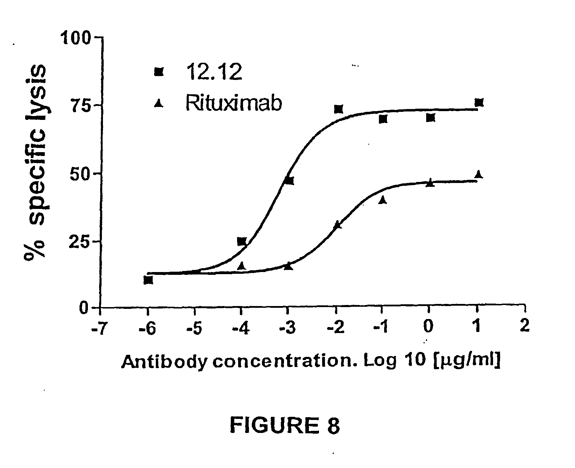

Antibody concentration-dependent Daudi cell lysis was observed (Figure 8; Table 19 below). The maximum specific lysis of target lymphoma cells induced by anti-CD40 mAb was greater compared to the lysis induced by rituximab (63.6% versus 45.9%, n=6; paired t test of mAb CHIR-12.12 versus rituximab, p=0.0002). In addition, ED50 for rituximab was on average (n=6) 51.6 pM, 13-fold higher than ED50 for the anti-CD40 mAb CHIR-12.12 for this activity.

**Table 19. Comparative ADCC of mAb CHIR-12.12 and rituximab against Daudi lymphoma cells.**

| | Maximal Killing (%) | | ED50 (pM) | |
|---|---|---|---|---|
| NK Cell Donor | mAb CHIR-12.12 | Rituximab | mAb CHIR-12.12 | Rituximab |
| 1 | 50.2 | 34.9 | 3.2 | 14.2 |
| 2 | 83.1 | 68.6 | 2.2 | 27.2 |
| 3 | 64.2 | 36.9 | 4.1 | 66.9 |
| 4 | 53.3 | 39.5 | 2.4 | 47.6 |
| 5 | 74.8 | 56.6 | 2.8 | 24.1 |
| 6 | 56.2 | 38.9 | 7.9 | 129.5 |
| Average | 63.6 | 45.9 | 3.8 | 51.6 |

### Example 20: CHIR-12.12 Blocks CD40L-Mediated CD40 Survival and Signaling Pathways in Normal Human B Cells

Soluble CD40 ligand (CD40L) activates B cells and induces various aspects of functional responses, including enhancement of survival and proliferation, and activation of NFκB, ERK/MAPK, PI3K/Akt, and p38 signaling pathways. In addition, CD40L-mediated CD40 stimulation provides survival signals by reduction of cleaved PARP and induction of the anti-apoptotic proteins, XIAP and Mcl-1, in normal B cells. CD40L-mediated CD40 stimulation also recruits TRAF2 and TRAF3 to bind CD40 cytoplasmic domain.

The following studies demonstrate that CHIR-12.12 directly inhibited all of these stimulation effects on normal human B cells. For example, CHIR-12.12 treatment resulted in increased cleavage of caspase-9, caspase-3, and PARP as well as reduction of XIAP and Mcl-1 in a time- and dose-dependent manner, restoring B cell apoptosis. Treatment with CHIR-12.12 also inhibited phosphorylation of IκB kinase (IKK) α and β (NFκB pathway), ERK, Akt, and p38 in response to CD40L-mediated CD40 stimulation. Further, it was found that CHIR-12.12 did not trigger these apoptotic effects without initial CD40L-mediated CD40 stimulation.

### CHIR-12.12 inhibited survival mediated by CD40 ligand by inducing cleavage of PARP.

In these experiments, 0.6x10⁶ normal human B cells from healthy donors (percent purity between 85-95%) were stimulated with 1 µg/ml sCD40L (Alexis Corp., Bingham, Nottinghamshire, UK). CHIR-12.12 (10 µg/ml) and control IgG were then added. Cells were collected at 0, 20 minutes, 2 hours, 6 hours, 18 hours, and 26 hours. Cleaved caspase-9, cleaved caspase-3, cleaved PARP, and β-actin controls were detected in cell lysates by Western blot.

Briefly, it was observed that CD40L-mediated CD40 stimulation provided survival signals as it did not result in increases of cleaved caspase-9, cleaved caspase-3, or cleaved PARP over time, indicating that the cells were not undergoing apoptosis. However, treatment with CHIR-12.12 resulted in an increase of these cleavage products, indicating that CHIR-12.12 treatment abrogated the effects of CD40L binding on survival signaling in sCD40L-stimulated normal B cells, restoring B cell apoptosis (data not shown).

### CHIR-12.12 inhibited expression of "survival" anti-apoptotic proteins.

In these experiments, 0.6 x 10⁶ normal human B cells from healthy donors (percent purity between 85-95%) were stimulated with 1 µg/ml sCD40L (Alexis Corp., Bingham, Nottinghamshire, UK). CHIR-12.12 (10 µg/ml) and control IgG were then added. Cells were collected at 0, 20 minutes, 2 hours, 6 hours, 18 hours, and 26 hours. Mcl-1, XIAP, CD40, and β-actin controls were detected in cell lysates by Western blot.

Briefly, sCD40L stimulation resulted in sustained expression of Mcl-1 and XIAP over time. However, treatment of the sCD40L-stimulated cells with CHIR 12.12 resulted in a decrease in expression of these proteins overtime (data not shown). Since Mcl-1 and XIAP are "survival" signals capable of blocking the apoptotic pathway, these results demonstrate that CHIR-12.12 treatment removes the blockade against apoptosis in sCD40L-stimulated normal B cells.

### CHIR-12.12 treatment inhibited phosphorylation of IKKα (Ser180) and IKKβ (Ser 181) in normal B cells.

In these experiments, 1.0 x10⁶ normal human B cells from healthy donors (percent purity between 85-95%) were stimulated with 1 µg/ml sCD40L (Alexis Corp., Bingham, Nottinghamshire, UK). CHIR-12.12 (10 µg/ml) and control IgG were then added. Cells were collected at 0 and 20 minutes. Phosphorylated IKKα (Ser180) and INK β (Ser 181) and total IKKβ controls were detected in cell lysates by Western blot.

Briefly, stimulation by sCD40L resulted in phosphorylation of IKKα (Ser180) and IKK β (Ser 181) over time; however, treatment with CHIR-12.12 abrogated this response to sCD40L stimulation in normal B cells (data not shown).

### CHIR-12.12 treatment inhibited survival mediated by CD40 ligand in a dose-dependent manner.

In these experiments, 0.6 x 10⁶ normal human B cells from healthy donors percent purity between 85-95%) were stimulated with 1 µg/ml sCD40L (Alexis Corp., Bingham, Nottinghamshire, UK). CHIR-12.12 (0.01, 0.1, 0.2, 0.5, 1.0 µg/ml) and control IgG were then added. Cells were collected at 24 hours. Cleaved PARP, and β-actin controls were detected in cell lysates by Western blot.

Briefly, CHIR-12.12 treatment resulted in increase ofPARP cleavage in sCD40L stimulated cells in a dose-dependent manner and therefore abrogated the survival signaling pathway in sCD40L-stimulated normal B cells (data not shown).

### CHIR-12.12 inhibited expression of "survival" anti-apoptotic proteins in a dose-dependent manner.

In these experiments, 0.6 x 10⁶ normal human B cells from healthy donors (percent purity between 85-95%) were stimulated with 1 µg/ml sCD40L (Alexis Corp., Bingham, Nottinghamshire, UK). CHIR-12.12 (0.5, 2, and 10 µg/ml) and control IgG were then added. Cells were collected at 22 hours. Mcl-1, XIAP, cleaved PARP, and β-actin controls were detected in cell lysates by Western blot.

Briefly, CHIR-12.12 treatment reduced Mcl-1 and XIAP expression and increased cleaved PARP expression in sCD40L-stimulated cells in a dose-dependent manner, and thus abrogated these blockades to the apoptotic pathway in sCD40L-stimulated normal B cells (data not shown).

### CHIR-12.12 did not affect expression of anti-apoptotic proteins, cleaved-P ARP, and XIAP, in the absence of soluble CD40L signaling.

In these experiments, 1.0 x 10⁶ normal human B cells from healthy donors (percent purity between 85-95%) were treated with CHIR-12.12 (10 µg/ml) and control IgG only (i.e., cells were not pre-stimulated with sCD40L before adding antibody). Cells were collected at 0, 4, 14, and 16 hours. XIAP, cleaved PARP, and β-actin controls were detected in cell lysates by Western blot.

Briefly, the results show that without sCD40L stimulation, the cells expressed increased concentrations of cleaved PARP, while expression of XIAP remained constant, in both IgG treated control cells and CHIR-12.12 cells (data not shown). These data indicate that CHIR-12.12 does not trigger apoptosis in normal human B cells without CD40L stimulation.

### CHIR-12.12 inhibits phosphorylation of IKKα (Ser180) and IKKβ (Ser181), Akt, ERK, and p38 in normal B cells.

In these experiments, 1.0 x 10⁶ normal human B cells from healthy donors (percent purity between 85-95%) were serum starved in 1% FBS-containing media and stimulated with 1 µg/ml sCD40L (Alexis Corp., Bingham, Nottinghamshire, UK). The cultures were treated with CHIR-12.12 (1 and 10 µg/ml) and control IgG. Cells were collected at 0 and 20 minutes. Phospho-IKKα, phospho-IKKβ, total IKKβ, phospho-ERK, total ERK, phospho-Akt, total Akt, phospho-p38, and total p38 were detected in cell lysates by Western blot.

Briefly, sCD40L stimulation resulted in increases in IKKα/β phosphorylation, ERK phosphorylation, Akt phosphorylation, and p38 phosphorylation, thus leading to survival and or proliferation of the cells. Treatment of the cells with CHIR-12.12 abrogated the effects of sCD40L stimulation on these signaling pathways in normal B cells (data not shown).

### CHIR 12.12 inhibits multiple signaling pathways such as PI3K and MEK /ERK in the CD40 signaling cascade.

In these experiments, 1.0 x 10⁶ normal human B cells from healthy donors (percent purity between 85-95%) were serum starved in 1% FBS-containing media and stimulated with 1 µg/ml sCD40L (Alexis Corp., Bingham, Nottinghamshire, UK). The cultures were also treated with CHIR-12.12 (1 and 10 µg/ml), Wortmanin, (a PI3K/Akt inhibitor; 1 and 10 µM), LY 294002 (a PI3K/Akt inhibitor; 10 and 30 µM), and PD 98095 (a MEK inhibitor; 10 and 30 µg/ml). Cells were collected at 0 and 20 minutes. Phospho-ERK, phospho-Akt, total Akt, phospho-IKKα/β, and total were detected in cell lysates by Western blot.

Briefly, the results show that CHIR-12.12 abrogated the phosphorylation of all of these signal transduction molecules, whereas the signal transduction inhibitors showed only specific abrogation of signaling, indicating that CHIR-12.12 likely inhibits upstream of these signal transduction molecules mediated by CD40L stimulation (data not shown).

### CHIR-12.12 inhibits the binding of signaling molecules TRAF2 and TRAF3 to the cytoplasmic domain of CD40 in normal B cells.

In these experiments, 4.0 x 10⁶ normal human B cells from healthy donors (percent purity between 85-95%) were serum starved for four hours in 1% FBS-containing media and stimulated with 1 µg/ml sCD40L (Alexis Corp., Bingham, Nottinghamshire, UK) for 20 minutes. Cells were collected at 0 and 20 minutes. CD40 was immunoprecipitated using polyclonal anti-CD40 (Santa Cruz Biotechnology, CA), and was probed in a Western blot with anti-TRAF2 mAb (Santa Cruz Biotechnology, CA), anti-TRAF3 mAb (Santa Cruz Biotechnology, CA), and anti-CD40 mAb (Santa Cruz Biotechnology, CA).

Briefly, the results show that TRAF2 and TRAF3 co-precipitated with CD40 after sCD40L stimulation. In contrast, treatment with CHIR-12.12 abrogated formation of the CD40-TRAF2/3 signaling complex in sCD40L-stimulated normal B cells. There were no changes in CD40 expression (data not shown).

Without being bound by theory, the results of these experiments, and the results in the examples outlined above, indicate that the CHIR-12.12 antibody is a dual action antagonist anti-CD40 monoclonal antibody having a unique combination of attributes. This fully human monoclonal antibody blocks CD40L-mediated CD40 signaling pathways for survival and proliferation of B cells; this antagonism leads to ultimate cell death. CHIR-12.12 also mediates recognition and binding by effector cells, initiating antibody dependent cellular cytotoxicity (ADCC). Once CHIR-12.12 is bound to effector cells, cytolytic enzymes are released, leading to B-cell apoptosis and lysis. CHIR-12.12 is a more potent anti-tumor antibody than is rituximab when compared in pre-clinical tumor models.

### Example 21: Agonist and Antagonist Activity Against Primary Cancer Cell from NHL, CLL, and NM Patients

In collaboration with clinical investigators, the candidate mAbs is tested for a variety of activities (listed below) against primary cancer cells from NHL and CLL and multiple myeloma patients.
- Agonist effect in proliferation assays (8 NHL patients, 8 CLL patients and 8 MM patients)
- Antagonist effect in proliferation assays (8 NHL patients, 8 CLL patients and 8 MM patients)
- Apoptotic effect by Annexin V assay (3-4 NHL patients, 4 CLL patients, and 4 MM patients)
- Reversing survival signal by Annexin V assay (3 NHL patients, 3 CLL patients and 3 MM patients)
- Complement dependent cytotoxicity (4 NHL patients, 4 CLL patients, and 4MM patients)
- Antibody dependent cytotoxicity (6 NHL patients, 6 CLL patients and 6 MM patients)

### Example 22: Identification of Relevant Animal Species for Toxicity Studies

As these two candidate antibodies do not cross-react with rodent CD40, other species must be identified for testing toxicologic effects.

The ability of the two candidate anti-CD40 antibodies to cross-react with animal CD40 is tested by flow cytometric assays. Rat, rabbit, dog, cynomolgus monkeys and marmoset monkeys are tested for this study.

The candidate antibodies show antagonist activity upon binding to CD40 on human B cells. To identify an animal species that has similar response to candidate antibodies, lymphocytes from species that show binding to candidate antibodies are tested in proliferation assays for antagonist activity. The lymphocytes from the species selected for antagonistic binding of candidate antibodies are further tested for their ability to serve as effector cells for killing CD40-expressing lymphoma cell lines through the mechanism of ADCC. Finally the selected animal species are tested in an IHC study for the tissue-binding pattern of candidate antibodies. The animal species responding to the candidate antibodies in these assays in a manner similar to that observed for human cells are chosen for toxicology studies.

Initial studies indicate that the candidate anti-CD40 mAbs cross-react with cynomolgus monkey CD40.

### Example 23: Tumor Targeting Profile of CHIR-5.9 and CHIR-12.12

To determine the relative tumor targeting profile of CHIR-12.12 and CHIR-5.9 mAbs, fluorescent-labeled candidate mAbs and isotype control antibodies are administered into tumor-bearing mice. Tumor specimens and normal organs are harvested at different time points after dosing. The accumulation of labeled antibody in tumors and normal organs is analyzed.

### Example 24: Mechanism of Action of CHIR-5.9 and CHIR-12.12

To elucidate the mechanism(s) that mediates the tumor growth inhibition by the CHIR-5.9 and CHIR-12.12 mAbs, the following studies are undertaken:

Fc-receptor knock-out or blockage model: ADCC is mediated by binding of effecter cells such as NK, marcrophage, and monocytes to the Fc portion of antibody through Fc receptor. Mice deficient in activating Fc receptors as well as antibodies engineered to disrupt Fc binding to those receptors will block the ADCC mediated tumor growth inhibition. Loss or significantly reduced tumor inhibition in this model will suggest that the tumor growth inhibition by these two candidate mAbs is mainly mediated by ADCC mechanism.

### Example 25: Liquid Pharmaceutical Formulation for Antagonist Anti-CD40 Antibodies

The objective of this study was to investigate the effects of solution pH on stability of the antagonist anti-CD40 antibody CHIR-12.12 by both biophysical and biochemical methods in order to select the optimum solution environment for this antibody. Differential Scanning Calorimetry (DSC) results showed that the conformation stability of CHIR-12.12 is optimal in formulations having pH 5.5-6.5. Based on a combination of SDS-PAGE, Size-Exclusion HPLC (SEC-HPLC), and Cation-Exchange HPLC (CEX-HPLC) analysis, the physicochemical stability of CHIR-12.12 is optimal at about pH 5.0-5.5. In view of these results, one recommended liquid pharmaceutical formulation comprising this antibody is a formulation comprising CHIR-12.12 at about 20 mg/ml formulated in about 10 mM sodium succinate, about 150 mM sodium chloride, and having a pH of about pH 5.5.

### Materials and Methods

The CHIR-12.12 antibody used in the formulation studies is a human monoclonal antibody produced by a CHO cell culture process. This MAb has a molecular weight of 150 kDa and consists of two light chains and two heavy chains linked together by disulfide bands. It is targeted against the CD40 cell surface receptor on CD40-expressing cells, including normal and malignant B cells, for treatment of various cancers and autoimmune/inflammatory diseases.

The anti-CD40 drug substance used for this study was a CHO-derived purified anti-CD40 (CHIR-12.12) bulk lot. The composition of the drug substance was 9.7 mg/ml CHIR-12.12 antibody in 10 mM sodium citrate, 150 mM sodium chloride, at pH 6.5. The control sample in the study was the received drug substance, followed by freezing at ≤ - 60°C, thawing at RT and testing along with stability samples at predetermined time points. The stability samples were prepared by dialysis of the drug substance against different pH solutions and the CHIR-12.12 concentration in each sample was determined by UV 280 as presented in Table 20.

**Table 20. CHIR-12.12 formulations.**

| Buffer Composition | pH | CHIR-12.12 Concentration (mg/ml) |
|---|---|---|
| 10 mM sodium citrate, 150 mM sodium chloride | 4.5 | 9.0 |
| 10 mM sodium succinate, 150 mM sodium chloride | 5.0 | 9.3 |
| 10 mM sodium succinate, 150 mM sodium chloride | 5.5 | 9.2 |
| 10 mM sodium citrate, 150 mM sodium chloride | 6.0 | 9.7 |
| 10 mM sodium citrate, 150 mM sodium chloride | 6.5 | 9.4 |
| 10 mM sodium phosphate, 150 mM sodium chloride | 7.0 | 9.4 |
| 10 mM sodium phosphate, 150 mM sodium chloride | 7.5 | 9.5 |
| 10 mM glycine, 150 mM sodium chloride | 9.0 | 9.5 |

Physicochemical stability of the CHIR-12.12 antibody in the various formulations was assayed using the following protocols.

### Differential Scanning Calorimetry (DSC

Conformational stability of different formulation samples was monitored using a MicroCal VP-DSC upon heating 15°C to 90°C at 1°C/min.

### SDS-PAGE

Fragmentation and aggregation were estimated using 4-20% Tris-Glycine Gel under non-reducing and reducing conditions. Protein was detected by Coomassie blue staining.

### Size Exclusion Chromatograph (SEGHPLC)

Protein fragmentation and aggregation were also measured by a Water Alliance HPLC with a Tosohaas TSK-GEL 3000SWXL column, 100 mM sodium phosphate, pH 7.0 as mobile phase at a flow rate of 0.7 ml/min.

### Cation Exchange Chromatography (CEX-HPLC)

Charge change related degradation was measured using Waters 600s HPLC system with a Dionex Propac WCX-10 column, 50 mM HEPEs, pH 7.3 as mobile phase A and 50 mM HEPES containing 500 mM NaCl, pH 7.3 as mobile phase B at a flow rate of 0.5°C/min.

### Results and Discussion

### Conformational stability study.

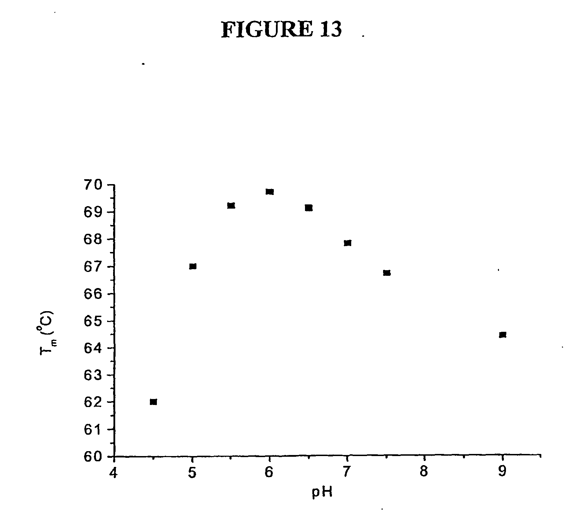

Thermal unfolding of CHIR-12.12 revealed at least two thermal transitions, probably representing unfolding melting of the Fab and the Fc domains, respectively. At higher temperatures, the protein presumably aggregated, resulting in loss of DSC signal. For the formulation screening purpose, the lowest thermal transition temperature was defined as the melting temperature, Tm, in this study. Figure 13 shows the thermal melting temperature as a function of formulation pHs. Formulations at pH 5.5-6.5 provided anti-CD40 with higher conformational stability as demonstrated by the higher thermal melting temperatures.

### SDS-PAGE analysis.

The CHIR-12.12 formulation samples at pH 4.5-9.0 were incubated at 40°C for 2 months and subjected to SDS-PAGE analysis (data not shown). Under non-reducing conditions, species with molecular weight (MW) of 23 kDa and 27 kDa were observed in formulations above pH 5.5, and species with MW of 51 kDa were observed in all formulations, but appeared less at pH 5.0-5.5. A species with MW of 100 kDa could be seen at pH 7.5 and pH 9.0.

Under reducing conditions, CHIR-12.12 was reduced into free heavy chains and light chains with MW of 50 kDa and 24 kDa, respectively. The 100 kDa species seerned not fully reducible and increased with increasing solution pH, suggesting non-disulfide covalent association might occur in the molecules. Since there were other species with unknown identities on SDS-PAGE, stability comparison of each formulation is based on the remaining purity of CHIR-12.12. Formulations at pH 5.0-6.0 provided a more stable environment to CHIR-12.12. Few aggregates were detected by SDS-PAGE (data not shown).

### SEC-HPLC analysis.

SEC-HPLC analysis detected the intact CHIR-12.12 as the main peak species, an aggregation species as a front peak species separate from the main peak species, a large fragment species as a shoulder peak on the back of the main peak species, and small fragment species were detected post-main peak species. After incubation at 5°C and 25°C for 3 months, negligible amounts of protein fragments and aggregates (<1.0%) were detected in the above formulations and the CHIR-12.12 main peak species remained greater than 99% purity (data not shown). However, protein fragments gradually developed upon storage at 40°C and more fragments formed at pH 4.5 and pH 6.5-9.0, as shown in Table 21. After incubating the CHIR-12.12 formulations at 40°C for 3 months, about 2-3% aggregates were detected in pH 7.5 and pH 9.0, while less than 1% aggregates were detected in other pH formulations (data not shown). The SEC-HPLC results indicate CHIR-12.12 is more stable at about pH 5.0-6.0.

**Table 21. SEC-HPLC results of CHIR-12.12 stability samples under real-time and accelerated storage conditions.**

| Sample | Main peak % | | | | Fragments % | | | |
|---|---|---|---|---|---|---|---|---|
| | t=0 | 40°C 1 m | 40°C 2m | 40°C 3m | t=0 | 40°C 1 m | 40°C 2m | 40°C 3m |
| Control | 99.4 | 99.2 | 99.9 | 99.5 | <1.0 | <1.0 | <1.0 | <1.0 |
| pH 4.5 | 99.4 | 93.2 | 86.0 | 81.3 | <1.0 | 6.4 | 13.2 | 18.1 |
| pH 5.0 | 99.8 | 98.7 | 91.3 | 89.2 | <1.0 | <1.0 | 7.8 | 10.2 |
| pH 5.5 | 99.8 | 98.9 | 91.4 | 90.6 | <1.0 | <1.0 | 7.6 | 8.8 |
| pH 6.0 | 99.6 | 97.7 | 90.4 | 87.3 | <1.0 | 1.9 | 8.2 | 11.7 |
| pH 6.5 | 99.3 | 93.4 | 89.0 | 86.9 | <1.0 | 5.6 | 9.9 | 12.4 |
| pH 7.0 | 99.2 | 93.9 | 87.4 | 85.1 | <1.0 | 5.5 | 11.1 | 13.5 |
| pH 7.5 | 99.1 | 92.8 | 84.4 | 81.9 | <1.0 | 6.4 | 12.9 | 16.2 |
| pH 9.0 | 99.3 | 82.4 | 61.6 | 50.6 | <1.0 | 15.4 | 36.2 | 47.6 |

### CEX-HPLC analysis.

CEX-HPLC analysis detected the intact CHIR-12.12 as the main peak species, acidic variants eluted earlier than the main peak species, and C-terminal lysine addition variants eluted post-main peak species. Table 22 shows the dependence of the percentages of the remaining main peak CHIR-12.12 species and acidic variants on solution pH. The control sample already contained a high degree of acidic species (∼33%), probably due to early-stage fermentation and purification processes. The susceptibility of CHIR-12.12 to higher pH solutions is evidenced by two facts. First, the initial formulation sample at pH 9.0 (t=0) already generated 12% more acidic species than the control. Second, the percentage of acidic species increased sharply with increasing pH. The charge change-related degradation is likely due to deamidation. The above data indicate that this type of degradation of CHIR-12.12 was minimized at about pH 5.0-5.5.

Table 22. Percentage of peak area by CEX-HPLC for CHIR-12.12 in different pH formulation under real-time and accelerated storage conditions.

| Sample | Main peak % | | | | | Acidic variants % | | | | |
|---|---|---|---|---|---|---|---|---|---|---|
| | t=0 | 5°C 3m | 25°C 3m | 40°C 1 m | 40°C 2m | t=0 | 5°C 3m | 25°C 3m | 40°C 1 m | 40°C 2m |
| Control | 49.2 | 49.8 | 49.8 | 49.2 | 50.3 | 32.0 | 33.7 | 33.7 | 32.0 | 33.6 |
| pH 4.5 | 48.5 | 49.7 | 43.7 | 39.7 | 30.0 | 32.5 | 32.6 | 38.0 | 44.2 | 56.4 |
| pH 5.0 | 49.6 | 49.8 | 48.3 | 40.6 | 31.4 | 32.7 | 31.8 | 35.0 | 44.3. | 57.1 |
| pH 5.5 | 50.7 | 50.3 | 48.1 | 40.0 | 30.2 | 32.6 | 31.8 | 37.8 | 48.9 | 63.3 |
| pH 6.0 | 50.2 | 49.9 | 47.9 | 37.4 | 23.9 | 33.1 | 33.6 | 38.5 | 54.9 | 72.7 |
| pH 6.5 | 49.4 | 49.9 | 42.3 | 29.7 | 14.6 | 33.3 | 33.6 | 47.7 | 65.2 | 84.6 |
| pH 7.0 | 49.7 | 49.9 | 21.9 | - | - | 34.4 | 36.4 | 64.4 | - | - |
| pH 7.5 | 49.3 | 48.3 | 12.7 | - | - | 35.5 | 40.1 | 79.2 | - | - |
| pH 9.0 | 41.3 | 31.8 | - | - | - | 44.7 | 59.9 | - | - | - |

### Conclusion

The pH has a significant effect on conformational and physicochemical stabilities of CHIR-12.12. Charge change-related degradation was determined to be the main degradation pathway for CHIR-12.12, which was minimized at pH 5.0-5.5. Based on overall stability data, one recommended liquid pharmaceutical formulation comprising this antibody is a formulation comprising CHIR-12.12 at about 20 mg/ml formulated in about 10 mM sodium succinate, about 150 mM sodium chloride, and having a pH of about pH 5.5.

### Example 26: Clinical Studies with CHIR-5.9 and CHIR-12.12

### Clinical Objectives

The overall objective is to provide an effective therapy for B cell tumors by targeting them with an anti-CD40 IgG1. These tumors include B-cell lymphoma, Chronic Lymphocytic Lyphoma (CLL), Acute Lymphoblastic Leukemia (ALL), Multiple Myeloma (MM), Waldenstrom's Macroglobulinemia, and Systemic Castleman's Disease. The signal for these diseases is determined in phase II although some measure of activity may be obtained in phase I. Initially the agent is studied as a single agent, but will be combined with other agents, chemotherapeutics, and other antibodies, as development proceeds.

### Phase I

- Evaluate safety and pharmacokinetics - dose escalation in subjects with B cell malignancies.
- Choose dose based on safety, tolerability, and change in serum markers of CD40. In general an MTD is sought but other indications of efficacy (depletion of CD40+ B cells, etc.) may be adequate for dose finding.
- Consideration of more than one dose especially for different indications, e.g., the CLL dose may be different than the NHL. Thus, some dose finding may be necessary in phase II.
- Patients are dosed weekly with real-time pharmacokinetic (Pk) sampling. Initially a 4-week cycle is the maximum dosing allowed. The Pk may be highly variable depending on the disease studied, density of CD40 etc.
- This trial(s) is open to subjects with B-cell lymphoma, CLL, and potentially other B cell malignancies.
- Decision to discontinue or continue studies is based on safety, dose, and preliminary evidence of anti-tumor activity.
- Activity of drug as determined by response rate is determined in Phase II.
- Identify dose(s) for Phase II.

### Phase II

Several trials will be initiated in the above-mentioned tumor types with concentration on B-cell lymphoma, CLL, and Multiple Myeloma (MM). Separate trials may be required in low grade and intermediate/high grade NHL as CD40 may have a different function depending on the grade of lymphoma. In low-grade disease, CD40 acts more as a survival factor, preventing apoptosis. In higher-grade disease, interruption of CD40 signaling may lead to cell death. More than one dose, and more than one schedule may be tested in a randomized phase II setting.

In each disease, target a population that has failed current standard of care:
- CLL: patients who were resistant to Campath® and chemotherapy.
- Low grade NHL: Rituxan® or CHOP-R failures
- Intermediate NHL: CHOP-R failures
- Multiple Myeloma: Chemotherapy failures
   ✔ Decision to discontinue or continue with study is based on proof of therapeutic concept in Phase II
   ✔ Determine whether surrogate marker can be used as early indication of clinical efficacy
   ✔ Identify doses for Phase III

### Phase III

Phase III will depend on where the signal is detected in phase II, and what competing therapies are considered to be the standard. If the signal is in a stage of disease where there is no standard of therapy, then a single arm, well-controlled study could serve as a pivotal trial. If there are competing agents that are considered standard, then head-to-head studies are conducted.

## Claims

1. A human anti-CD40 monoclonal antibody, for use in treating a cancer **characterized by** expression of CD40, wherein said human anti-CD40 monoclonal antibody is selected from the group consisting of:
a) a monoclonal antibody that binds to an epitope capable of binding the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543;
b) a monoclonal antibody that binds to an epitope comprising residues 82-87 of the human CD40 sequence shown in SEQ ID NO:10 or SEQ ID NO:12;
c) a monoclonal antibody that binds to an epitope comprising residues 82-89 of the human CD40 sequence shown in SEQ ID NO:10 or SEQ ID NO:12; and
d) a monoclonal antibody that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543 in a competitive binding assay,
wherein said antibody is administered in combination with one or more further therapeutic agents.

2. The antibody for use according to claim 1, wherein said antibody is the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543.

3. The antibody for use according to claim 1, wherein said antibody comprises:
(i) the complementarity determining region (CDR) residues of SEQ ID NO:2 and the complementarity determining region (CDR) residues of SEQ ID NO:4; or
(ii) the complementarity determining region (CDR) residues of SEQ ID NO:6 and the complementarity determining region (CDR) residues of SEQ ID NO:7.

4. The antibody for use according to claim 1, wherein said antibody comprises an amino acid sequence selected from the group consisting of:
(i) residues 21-132 of SEQ ID NO:2;
(ii) residues 21-239 of SEQ ID NO:2;
(iii) SEQ ID NO:2;
(iv) residues 20-139 of SEQ ID NO:4;
(v) residues 20-469 of SEQ ID NO:4;
(vi) SEQ ID NO:4;
(vii) residues 20-469 of SEQ ID NO:5;
(viii) SEQ ID NO:5;
(ix) residues 21-132 of SEQ ID NO:2 and residues 20-139 of SEQ ID NO:4;
(x) residues 21-239 of SEQ ID NO:2 and residues 20-469 of SEQ ID NO:4;
(xi) residues 21-239 of SEQ ID NO:2 and residues 20-469 of SEQ ID NO:5;
(xii) SEQ ID NO:2 and SEQ ID NO:4; and
(xiii) SEQ ID NO:2 and SEQ ID NO:5.

5. The antibody for use according to claim 1, wherein said antibody comprises an amino acid sequence selected from the group consisting of:
(i) residues 21-132 of SEQ ID NO:6;
(ii) residues 21-239 of SEQ ID NO:6;
(iii) SEQ ID NO:6;
(iv) residues 20-144 of SEQ ID NO:7;
(v) residues 20-474 of SEQ ID NO:7;
(vi) SEQ ID NO:7;
(vii) residues 20-474 of SEQ ID NO:8;
(viii) SEQ ID NO:8;
(ix) residues 21-132 of SEQ ID NO:6 and residues 20-144 of SEQ ID NO:7;
(x) residues 21-239 of SEQ ID NO:6 and residues 20-474 of SEQ ID NO:7;
(xi) residues 21-239 of SEQ ID NO:6 and residues 20-474 of SEQ ID NO:8
(xii) SEQ ID NO:6 and SEQ ID NO:7; and
(xiii) SEQ ID NO:6 and SEQ ID NO:8.

6. The antibody for use according to any preceding claim, wherein said antibody binds to human CD40 with an affinity (K_{D}) of at least about 10⁻⁶ M, or at least 10⁻⁸M_{.}

7. The antibody for use according to any preceding claim, wherein said antibody is an antigen-binding fragment of a monoclonal antibody, wherein said fragment retains the capability of specifically binding to human CD40 antigen.

8. The antibody for use according to claim 7, wherein said fragment is selected from the group consisting of a Fab fragment, an F(ab')₂ fragment, an Fv fragment, and a single-chain Fv fragment.

9. The antibody for use according to any one of claims 1 and 3-8, wherein said antibody is produced in a CHO cell line.

10. The antibody for use according to any preceding claim, wherein said antibody is administered in combination with at least one other cancer therapy.

11. The antibody for use according to claim 10, wherein the other cancer therapy is administered prior to, during, or subsequent to the antagonist anti-CD40 antibody therapy.

## Patentansprüche

1. Humaner monoklonaler anti-CD40-Antikörper zur Verwendung in der Behandlung eines Krebs, der durch die Expression von CD40 gekennzeichnet ist, wobei der humane monoklonale anti-CD40-Antikörper aus der Gruppe bestehend aus folgenden stammt:
a) einem monoklonalen Antikörper, der an ein Epitop bindet, das fähig ist, den monoklonalen Antikörper CHIR-5.9, der aus der bei der ATCC als Patenthinterlegungs-Nr. PTA-5542 hinterlegten Hybridomzelllinie erhältlich ist, oder den monoklonalen Antikörper CHIR-12.12, der aus der bei der ATCC als Patenthinterlegungs-Nr. PTA-5543 hinterlegten Hybridomzelllinie erhältlich ist, zu binden;
b) einem monoklonalen Antikörper, der an ein Epitop, umfassend die Reste 82-87 der menschlichen CD40-Sequenz gemäß SEQ ID NO: 10 oder SEQ ID NO: 12, bindet;
c) einem monoklonalen Antikörper, der an ein Epitop, umfassend die Reste 82-89 der menschlichen CD40-Sequenz gemäß SEQ ID NO: 10 oder SEQ ID NO: 12, bindet; und
d) einem monoklonalen Antikörper, der mit dem monoklonalen Antikörper CHIR-5.9, der aus der bei der ATCC als Patenthinterlegungs-Nr. PTA-5542 hinterlegten Hybridomzelllinie erhältlich ist, oder dem monoklonalen Antikörper CHIR-12.12, der aus der bei der ATCC als Patenthinterlegungs-Nr. PTA-5543 hinterlegten Hybridomzelllinie erhältlich ist, in einem kompetitiven Bindungs-Assay konkurriert,
wobei der Antikörper in Kombination mit einem oder mehreren weiteren therapeutischen Agentien verabreicht wird.

2. Antikörper zur Verwendung nach Anspruch 1, wobei es sich bei dem Antikörper um den monoklonalen Antikörper CHIR-5.9, der aus der bei der ATCC als Patenthinterlegungs-Nr. PTA-5542 hinterlegten Hybridomzelllinie erhältlich ist, oder den monoklonalen Antiköper CHIR-12.12, der aus der bei der ATCC als Patenthinterlegungs-Nr. PTA-5543 hinterlegten Hybridomzelllinie erhältlich ist, handelt.

3. Antikörper zur Verwendung nach Anspruch 1, wobei der Antikörper Folgendes umfasst:
(i) die Reste der komplementaritätsbestimmenden Region (CDR) von SEQ ID NO: 2 und die Reste der komplementaritätsbestimmenden Region (CDR) von SEQ ID NO: 4, oder
(ii) die Reste der komplementaritätsbestimmenden Region (CDR) von SEQ ID NO: 6 und die Reste der komplementaritätsbestimmenden Region (CDR) von SEQ ID NO: 7.

4. Antikörper zur Verwendung nach Anspruch 1, wobei der Antikörper eine Aminosäuresequenz, ausgewählt aus der Gruppe bestehend aus:
(i) den Resten 21-132 von SEQ ID NO: 2;
(ii) den Resten 21-239 von SEQ ID NO: 2;
(iii) SEQ ID NO: 2;
(iv) den Resten 20-139 von SEQ ID NO: 4;
(v) den Resten 20-469 von SEQ ID NO: 4;
(vi) SEQ ID NO: 4;
(vii) den Resten 20-469 von SEQ ID NO: 5;
(viii) SEQ ID NO: 5;
(ix) den Resten 21-132 von SEQ ID NO: 2 und den Resten 20-139 von SEQ ID NO: 4;
(x) den Resten 21-239 von SEQ ID NO: 2 und den Resten 20-469 von SEQ ID NO: 4;
(xi) den Resten 21-239 von SEQ ID NO: 2 und den Resten 20-469 von SEQ ID NO: 5;
(xii) SEQ ID NO: 2 und SEQ ID NO: 4 und
(xiii) SEQ ID NO: 2 und SEQ ID NO: 5 umfasst.

5. Antikörper zur Verwendung nach Anspruch 1, wobei der Antikörper eine Aminosäuresequenz, ausgewählt aus der Gruppe bestehend aus:
(i) den Resten 21-132 von SEQ ID NO: 6;
(ii) den Resten 21-239 von SEQ ID NO: 6;
(iii) SEQ ID NO: 6;
(iv) den Resten 20-144 von SEQ ID NO: 7;
(v) den Resten 20-474 von SEQ ID NO: 7;
(vi) SEQ ID NO: 7;
(vii) den Resten 20-474 von SEQ ID NO: 8;
(viii) SEQ ID NO: 8;
(ix) den Resten 21-132 von SEQ ID NO: 6 und den Resten 20-144 von SEQ ID NO: 7;
(x) den Resten 21-239 von SEQ ID NO: 6 und den Resten 20-474 von SEQ ID NO: 7;
(xi) den Resten 21-239 von SEQ ID NO: 6 und den Resten 20-474 von SEQ ID NO: 8;
(xii) SEQ ID NO: 6 und SEQ ID NO: 7 und
(xiii) SEQ ID NO: 6 und SEQ ID NO: 8
umfasst.

6. Antikörper zur Verwendung nach einem vorhergehenden Anspruch, wobei der Antikörper an Human-CD40 mit einer Affinität (K_{D}) von mindestens ungefähr 10⁻⁶ M oder mindestens 10⁻⁸ M bindet.

7. Antikörper zur Verwendung nach einem vorhergehenden Anspruch, wobei es sich bei dem Antikörper um ein antigenbindendes Fragment eines monoklonalen Antikörpers handelt, wobei das Fragment die Fähigkeit, spezifisch an Human-CD40-Antigen zu binden, beibehält.

8. Antikörper zur Verwendung nach Anspruch 7, wobei das Fragment aus der Gruppe bestehend aus einem Fab-Fragment, einem F(ab')₂-Fragment, einem Fv-Fragment und einem Einzelketten-Fv-Fragment ausgewählt ist.

9. Antikörper zur Verwendung nach einem der Ansprüche 1 und 3-8, wobei der Antikörper in einer CHO-Zelllinie produziert wird.

10. Antikörper zur Verwendung nach einem vorhergehenden Anspruch, wobei der Antikörper in Kombination mit mindestens einer weiteren Krebstherapie verabreicht wird.

11. Antikörper zur Verwendung nach Anspruch 10, wobei die andere Krebstherapie vor, während oder nach der Therapie mit dem antagonistischen anti-CD40-Antikörper erfolgt.

## Revendications

1. Anticorps monoclonal anti-CD40 humain, pour utilisation dans le traitement d'un cancer **caractérisé par** l'expression de CD40, ledit anticorps monoclonal anti-CD40 humain étant choisi dans le groupe constitué de :
a) un anticorps monoclonal qui se lie à un épitope capable de se lier à l'anticorps monoclonal CHIR-5.9 pouvant être obtenu à partir de la lignée cellulaire d'hybridome déposée auprès de l'ATCC sous le dépôt de brevet n° PTA-5542 ou l'anticorps monoclonal CHIR-12.12 pouvant être obtenu à partir de la lignée cellulaire d'hybridome déposée auprès de l'ATCC sous le dépôt de brevet n° PTA-5543 ;
b) un anticorps monoclonal qui se lie à un épitope comprenant les résidus 82-87 de la séquence CD40 humaine décrite dans SEQ ID NO: 10 ou SEQ ID NO: 12 ;
c) un anticorps monoclonal qui se lie à un épitope comprenant les résidus 82-89 de la séquence CD40 humaine décrite dans SEQ ID NO: 10 ou SEQ ID NO: 12 ; et
d) un anticorps monoclonal qui entre en compétition avec l'anticorps monoclonal CHIR-5.9 pouvant être obtenu à partir de la lignée cellulaire d'hybridome déposée auprès de l'ATCC sous le dépôt de brevet n° PTA-5542 ou l'anticorps monoclonal CHIR-12.12 pouvant être obtenu à partir de la lignée cellulaire d'hybridome déposée auprès de l'ATCC sous le dépôt de brevet n° PTA-5543 dans un essai de liaison compétitif, ledit anticorps étant administré en combinaison avec un ou plusieurs autres agents thérapeutiques.

2. Anticorps pour utilisation selon la revendication 1, ledit anticorps étant l'anticorps monoclonal CHIR-5.9 pouvant être obtenu à partir de la lignée cellulaire d'hybridome déposée auprès de l'ATCC sous le dépôt de brevet n° PTA-5542 ou l'anticorps monoclonal CHIR-12.12 pouvant être obtenu à partir de la lignée cellulaire d'hybridome déposée auprès de l'ATCC sous le dépôt de brevet n° PTA-5543.

3. Anticorps pour utilisation selon la revendication 1, ledit anticorps comprenant :
(i) les résidus de région déterminant la complémentarité (CDR) de SEQ ID NO: 2 et les résidus de région déterminant la complémentarité (CDR) de SEQ ID NO: 4 ; ou
(ii) les résidus de région déterminant la complémentarité (CDR) de SEQ ID NO: 6 et les résidus de région déterminant la complémentarité (CDR) de SEQ ID NO: 7.

4. Anticorps pour utilisation selon la revendication 1, ledit anticorps comprenant une séquence d'acides aminés choisie dans le groupe constitué de :
(i) résidus 21-132 de SEQ ID NO: 2 ;
(ii) résidus 21-239 de SEQ ID NO: 2 ;
(iii) SEQ ID NO: 2 ;
(iv) résidus 20-139 de SEQ ID NO: 4 ;
(v) résidus 20-469 de SEQ ID NO: 4 ;
(vi) SEQ ID NO: 4 ;
(vii) résidus 20-469 de SEQ ID NO: 5 ;
(viii) SEQ ID NO: 5 ;
(ix) résidus 21-132 de SEQ ID NO: 2 et résidus 20-139 de SEQ ID NO: 4 ;
(x) résidus 21-239 de SEQ ID NO: 2 et résidus 20-469 de SEQ ID NO: 4 ;
(xi) résidus 21-239 de SEQ ID NO: 2 et résidus 20-469 de SEQ ID NO: 5 ;
(xii) SEQ ID NO: 2 et SEQ ID NO: 4 ; et
(xiii) SEQ ID NO: 2 et SEQ ID NO: 5.

5. Anticorps pour utilisation selon la revendication 1, ledit anticorps comprenant une séquence d'acides aminés choisie dans le groupe constitué de :
(i) résidus 21-132 de SEQ ID NO: 6 ;
(ii) résidus 21-239 de SEQ ID NO: 6 ;
(iii) SEQ ID NO: 6 ;
(iv) résidus 20-144 de SEQ ID NO: 7 ;
(v) résidus 20-474 de SEQ ID NO: 7 ;
(vi) SEQ ID NO: 7 ;
(vii) résidus 20-474 de SEQ ID NO: 8 ;
(viii) SEQ ID NO: 8 ;
(ix) résidus 21-132 de SEQ ID NO: 6 et résidus 20-144 de SEQ ID NO: 7 ;
(x) résidus 21-239 de SEQ ID NO: 6 et résidus 20-474 de SEQ ID NO: 7 ;
(xi) résidus 21-239 de SEQ ID NO: 6 et résidus 20-474 de SEQ ID NO: 8 ;
(xii) SEQ ID NO: 6 et SEQ ID NO: 7 ; et
(xiii) SEQ ID NO: 6 et SEQ ID NO: 8.

6. Anticorps pour utilisation selon l'une quelconque des revendications précédentes, ledit anticorps se liant à CD40 humain avec une affinité (K_{D}) d' au moins environ 10⁻⁶ M, ou au moins 10⁻⁸ M.

7. Anticorps pour utilisation selon l'une quelconque des revendications précédentes, ledit anticorps étant un fragment de liaison d'antigène d'un anticorps monoclonal, ledit fragment conservant la capacité à se lier spécifiquement à l'antigène CD40 humain.

8. Anticorps pour utilisation selon la revendication 7, ledit fragment étant choisi dans le groupe constitué d'un fragment Fab, un fragment F(ab')₂, un fragment Fv, et un fragment Fv monocaténaire.

9. Anticorps pour utilisation selon l'une quelconque des revendications 1 et 3 à 8, ledit anticorps étant produit dans une lignée cellulaire CHO.

10. Anticorps pour utilisation selon l'une quelconque des revendications précédentes, ledit anticorps étant administré en combinaison avec au moins une autre thérapie anticancéreuse.

11. Anticorps pour utilisation selon la revendication 10, l'autre thérapie anticancéreuse étant administrée avant, pendant, ou après la thérapie par anticorps anti-CD40 antagoniste.


### SEQUENCE LISTING

<110> Chen, Bao-Lu Hurst, Deborah
   Lee, Sang Hoon Long, Li Lu, Xiaofeng Luqman, Mohammad Yabannavar, Asha Zaror, Isabel
<120> Antagonist Anti-CD40 Monoclonal
   Antibodies and Methods for Their Use
<130> PP20107.004 (282916)
<150> 60/565,710
   <151> 2004-04-27
<150> 60/525,579
   <151> 2003-11-26
<150> 60/517,337
   <151> 2003-11-04
<160> 12
<170> FastSEQ for Windows Version 4.0
<210> 1
   <211> 720
   <212> DNA
   <213> Artificial Sequence
<220>
   <223> Coding sequence for light chain of 12.12 human anti-CD40 antibody
<221> CDS
   <222> (1)...(720)
<400> 1
   atg gcg ctc cct gct cag ctc ctg ggg ctg cta atg ctc tgg gtc tct    48
   Met Ala Leu Pro Ala Gln Leu Leu Gly Leu Leu Met Leu Trp Val Ser
    1               5                  10                  15
   gga tcc agt ggg gat att gtg atg act cag tct cca ctc tcc ctg acc    96
   Gly Ser Ser Gly Asp Ile Val Met Thr Gln Ser Pro Leu Ser Leu Thr
               20                  25                  30
   gtc acc cct gga gag ccg gcc tcc atc tcc tgc agg tcc agt cag agc    144
   Val Thr Pro Gly Glu Pro Ala Ser Ile Ser Cys Arg Ser Ser Gln Ser
           35                  40                  45
   ctc ctg tat agt aat gga tac aac tat ttg gat tgg tac ctg cag aag    192
   Leu Leu Tyr Ser Asn Gly Tyr Asn Tyr Leu Asp Trp Tyr Leu Gln Lys
       50                  55                  60
   cca ggg cag tct cca cag gtc ctg atc tct ttg ggt tct aat cgg gcc    240
   Pro Gly Gln Ser Pro Gln Val Leu Ile Ser Leu Gly Ser Asn Arg Ala
   65                  70                  75                  80
   tcc ggg gtc cct gac agg ttc agt ggc agt gga tca ggc aca gat ttt    288
   Ser Gly Val Pro Asp Arg Phe Ser Gly Ser Gly Ser Gly Thr Asp Phe
                   85                  90                  95
   aca ctg aaa atc agc aga gtg gag gct gag gat gtt ggg gtt tat tac    336
   Thr Leu Lys Ile Ser Arg Val Glu Ala Glu Asp Val Gly Val Tyr Tyr
               100                 105                 110
   tgc atg caa gct cga caa act cca ttc act ttc ggc cct ggg acc aaa    384
   Cys Met Gln Ala Arg Gln Thr Pro Phe Thr Phe Gly Pro Gly Thr Lys
           115                 120                 125
   gtg gat atc aga cga act gtg gct gca cca tct gtc ttc atc ttc ccg    432
   Val Asp Ile Arg Arg Thr Val Ala Ala Pro Ser Val Phe Ile Phe Pro
       130                 135                 140
   cca tct gat gag cag ttg aaa tct gga act gcc tct gtt gtg tgc ctg    480
   Pro Ser Asp Glu Gln Leu Lys Ser Gly Thr Ala Ser Val Val Cys Leu
   145                 150                 155                 160
   ctg aat aac ttc tat ccc aga gag gcc aaa gta cag tgg aag gtg gat    528
   Leu Asn Asn Phe Tyr Pro Arg Glu Ala Lys Val Gln Trp Lys Val Asp
                   165                 170                 175
   aac gcc ctc caa tcg ggt aac tcc cag gag agt gtc aca gag cag gac    576
   Asn Ala Leu Gln Ser Gly Asn Ser Gln Glu Ser Val Thr Glu Gln Asp
               180                 185                 190
   agc aag gac agc acc tac agc ctc agc agc acc ctg acg ctg agc aaa    624
   Ser Lys Asp Ser Thr Tyr Ser Leu Ser Ser Thr Leu Thr Leu Ser Lys
           195                 200                 205
   gca gac tac gag aaa cac aaa gtc tac gcc tgc gaa gtc acc cat cag    672
   Ala Asp Tyr Glu Lys His Lys Val Tyr Ala Cys Glu Val Thr His Gln
       210                 215                 220
   ggc ctg agc tcg ccc gtc aca aag agc ttc aac agg gga gag tgt tag    720
   Gly Leu Ser Ser Pro Val Thr Lys Ser Phe Asn Arg Gly Glu Cys  *
   225                 230                 235
<210> 2
   <211> 239
   <212> PRT
   <213> Artificial Sequence
<220>
   <223> Light chain of 12.12 human anti-CD40 antibody
<400> 2
   Met Ala Leu Pro Ala Gln Leu Leu Gly Leu Leu Met Leu Trp Val Ser
   1               5                   10                  15
   Gly Ser Ser Gly Asp Ile Val Met Thr Gln Ser Pro Leu Ser Leu Thr
               20                  25                  30
   Val Thr Pro Gly Glu Pro Ala Ser Ile Ser Cys Arg Ser Ser Gln Ser
           35                  40                  45
   Leu Leu Tyr Ser Asn Gly Tyr Asn Tyr Leu Asp Trp Tyr Leu Gln Lys
       50                  55                  60
   Pro Gly Gln Ser Pro Gln Val Leu Ile Ser Leu Gly Ser Asn Arg Ala
   65                  70                  75                  80
   Ser Gly Val Pro Asp Arg Phe Ser Gly Ser Gly Ser Gly Thr Asp Phe
                   85                  90                  95
   Thr Leu Lys Ile Ser Arg Val Glu Ala Glu Asp Val Gly Val Tyr Tyr
               100                 105                 110
   Cys Met Gln Ala Arg Gln Thr Pro Phe Thr Phe Gly Pro Gly Thr Lys
           115                 120                 125
   Val Asp Ile Arg Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe Tyr Pro Arg Glu Ala Lys Val Gln Trp Lys Val Asp
                   165                 170                 175
   Asn Ala Leu Gln Ser Gly Asn Ser Gln Glu Ser Val Thr Glu Gln Asp
               180                 185                 190
   Ser Lys Asp Ser Thr Tyr Ser Leu Ser Ser Thr Leu Thr Leu Ser Lys
           195                 200                 205
   Ala Asp Tyr Glu Lys His Lys Val Tyr Ala Cys Glu Val Thr His Gln
       210                 215                 220
   Gly Leu Ser Ser Pro Val Thr Lys Ser Phe Asn Arg Gly Glu Cys
   225                 230                 235
<210> 3
   <211> 2016
   <212> DNA
   <213> Artificial Sequence
<220>
   <223> Coding sequence for heavy chain of 12.12 human anti-CD40 antibody (with introns)
<400> 3
   atg gag ttt ggg ctg agc tgg gtt ttc ctt gtt gct att tta aga ggt 48
   gtc cag tgt cag gtg cag ttg gtg gag tct ggg gga ggc gtg gtc cag 96
   cct ggg agg tcc ctg aga ctc tcc tgt gca gcc tct gga ttc acc ttc 144
   agt agc tat ggc atg cac tgg gtc cgc cag gct cca ggc aag ggg ctg 192
   gag tgg gtg gca gtt ata tca tat gag gaa agt aat aga tac cat gca 240
   gac tcc gtg aag ggc cga ttc acc atc tcc aga gac aat tcc aag atc 288
   acg ctg tat ctg caa atg aac agc ctc aga act gag gac acg gct gtg 336
   tat tac tgt gcg aga gat ggg ggt ata gca gca cct ggg cct gac tac 384
   tgg ggc cag gga acc ctg gtc acc gtc tcc tca gca agt acc aag ggc 432
   cca tcc gtc ttc ccc ctg gcg ccc gct agc aag agc acc tct ggg ggc 480
   aca gcg gcc ctg ggc tgc ctg gtc aag gac tac ttc ccc gaa ccg gtg 528
   acg gtg tcg tgg aac tca ggc gcc ctg acc agc ggc gtg cac acc ttc 576
   ccg gct gtc cta cag tcc tca gga ctc tac tcc ctc agc agc gtg gtg 624
   acc gtg ccc tcc agc agc ttg ggc acc cag acc tac atc tgc aac gtg 672
   aat cac aag ccc agc aac acc aag gtg gac aag aga gtt ggt gag agg 720
   cca gca cag gga ggg agg gtg tct gct gga agc cag gct cag cgc tcc 768
   tgc ctg gac gca tcc cgg cta tgc agt ccc agt cca ggg cag caa ggc 816
   agg ccc cgt ctg cct ctt cac ccg gag gcc tct gcc cgc ccc act cat 864
   gct cag gga gag ggt ctt ctg gct ttt tcc cca ggc tct ggg cag gca 912
   cag gct agg tgc ccc taa ccc agg ccc tgc aca caa agg ggc agg tgc 960
   tgg gct cag acc tgc caa gag cca tat ccg gga gga ccc tgc ccc tga 1008
   cct aag ccc acc cca aag gcc aaa ctc tcc act ccc tca gct cgg aca 1056
   cct tct ctc ctc cca gat tcc agt aac tcc caa tct tct ctc tgc aga 1104
   gcc caa atc ttg tga caa aac tca cac atg ccc acc gtg ccc agg taa 1152
   gcc agc cca ggc ctc gcc ctc cag ctc aag gcg gga cag gtg ccc tag 1200
   agt agc ctg cat cca ggg aca ggc ccc agc cgg gtg ctg aca cgt cca 1248
   cct cca tct ctt cct cag cac ctg aac tcc tgg ggg gac cgt cag tct 1296
   tcc tct tcc ccc caa aac cca agg aca ccc tca tga tct ccc gga ccc 1344
   ctg agg tca cat gcg tgg tgg tgg acg tga gcc acg aag acc ctg agg 1392
   tca agt tca act ggt acg tgg acg gcg tgg agg tgc ata atg cca aga 1440
   caa agc cgc ggg agg agc agt aca aca gca cgt acc gtg tgg tca gcg 1488
   tcc tca ccg tcc tgc acc agg act ggc tga atg gca agg agt aca agt 1536
   gca agg tct cca aca aag ccc tcc cag ccc cca tcg aga aaa cca tct 1584
   cca aag cca aag gtg gga ccc gtg ggg tgc gag ggc cac atg gac aga 1632
   ggc cgg ctc ggc cca ccc tct gcc ctg aga gtg acc gct gta cca acc 1680
   tct gtc cct aca ggg cag ccc cga gaa cca cag gtg tac acc ctg ccc 1728
   cca tcc cgg gag gag atg acc aag aac cag gtc agc ctg acc tgc ctg 1776
   gtc aaa ggc ttc tat ccc agc gac atc gcc gtg gag tgg gag agc aat 1824
   ggg cag ccg gag aac aac tac aag acc acg cct ccc gtg ctg gac tcc 1872
   gac ggc tcc ttc ttc ctc tat agc aag ctc acc gtg gac aag agc agg 1920
   tgg cag cag ggg aac gtc ttc tca tgc tcc gtg atg cat gag gct ctg 1968
   cac aac cac tac acg cag aag agc ctc tcc ctg tct ccg ggt aaa tga 2016
<210> 4
   <211> 469
   <212> PRT
   <213> Artificial Sequence
<220>
   <223> Heavy chain of 12.12 human anti-CD40 antibody
<400> 4
   Met Glu Phe Gly Leu Ser Trp Val Phe Leu Val Ala Ile Leu Arg Gly
   1 5 10 15
   Val Gln Cys Gln Val Gln Leu Val Glu Ser Gly Gly Gly Val Val Gln
   20 25 30
   Pro Gly Arg Ser Leu Arg Leu Ser Cys Ala Ala Ser Gly Phe Thr Phe
   35 40 45
   Ser Ser Tyr Gly Met His Trp Val Arg Gln Ala Pro Gly Lys Gly Leu
   50 55 60
   Glu Trp Val Ala Val Ile Ser Tyr Glu Glu Ser Asn Arg Tyr His Ala
   65 70 75 80
   Asp Ser Val Lys Gly Arg Phe Thr Ile Ser Arg Asp Asn Ser Lys Ile
   85 90 95
   Thr Leu Tyr Leu Gln Met Asn Ser Leu Arg Thr Glu Asp Thr Ala Val
   100 105 110
   Tyr Tyr Cys Ala Arg Asp Gly Gly Ile Ala Ala Pro Gly Pro Asp Tyr
   115 120 125
   Trp Gly Gln Gly Thr Leu Val Thr Val Ser Ser Ala Ser Thr Lys Gly
   130 135 140
   Pro Ser Val Phe Pro Leu Ala Pro Ala Ser Lys Ser Thr Ser Gly Gly
   145 150 155 160
   Thr Ala Ala Leu Gly Cys Leu Val Lys Asp Tyr Phe Pro Glu Pro Val
   165 170 175
   Thr Val Ser Trp Asn Ser Gly Ala Leu Thr Ser Gly Val His Thr Phe
   180 185 190
   Pro Ala Val Leu Gln Ser Ser Gly Leu Tyr Ser Leu Ser Ser Val Val
   195 200 205
   Thr Val Pro Ser Ser Ser Leu Gly Thr Gln Thr Tyr Ile Cys Asn Val
   210 215 220
   Asn His Lys Pro Ser Asn Thr Lys Val Asp Lys Arg Val Glu Pro Lys
   225 230 235 240
   Ser Cys Asp Lys Thr His Thr Cys Pro Pro Cys Pro Ala Pro Glu Leu
   245 250 255
   Leu Gly Gly Pro Ser Val Phe Leu Phe Pro Pro Lys Pro Lys Asp Thr
   260 265 270
   Leu Met Ile Ser Arg Thr Pro Glu Val Thr Cys Val Val Val Asp Val
   275 280 285
   Ser His Glu Asp Pro Glu Val Lys Phe Asn Trp Tyr Val Asp Gly Val
   290 295 300
   Glu Val His Asn Ala Lys Thr Lys Pro Arg Glu Glu Gln Tyr Asn Ser
   305 310 315 320
   Thr Tyr Arg Val Val Ser Val Leu Thr Val Leu His Gln Asp Trp Leu
   325 330 335
   Asn Gly Lys Glu Tyr Lys Cys Lys Val Ser Asn Lys Ala Leu Pro Ala
   340 345 350
   Pro Ile Glu Lys Thr Ile Ser Lys Ala Lys Gly Gln Pro Arg Glu Pro
   355 360 365
   Gln Val Tyr Thr Leu Pro Pro Ser Arg Glu Glu Met Thr Lys Asn Gln
   370 375 380
   Val Ser Leu Thr Cys Leu Val Lys Gly Phe Tyr Pro Ser Asp Ile Ala
   385 390 395 400
   Val Glu Trp Glu Ser Asn Gly Gln Pro Glu Asn Asn Tyr Lys Thr Thr
   405 410 415
   Pro Pro Val Leu Asp Ser Asp Gly Ser Phe Phe Leu Tyr Ser Lys Leu
   420 425 430
   Thr Val Asp Lys Ser Arg Trp Gln Gln Gly Asn Val Phe Ser Cys Ser
   435 440 445
   Val Met His Glu Ala Leu His Asn His Tyr Thr Gln Lys Ser Leu Ser
   450 455 460
   Leu Ser Pro Gly Lys
   465
<210> 5
   <211> 469
   <212> PRT
   <213> Artificial Sequence
<220>
   <223> Heavy chain of variant of 12.12 human anti-CD40 antibody
<400> 5
   Met Glu Phe Gly Leu Ser Trp Val Phe Leu Val Ala Ile Leu Arg Gly
    1               5                  10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Thr Phe
           35                  40                  45
   Ser Ser Tyr Gly Met His Trp Val Arg Gln Ala Pro Gly Lys Gly Leu
       50                  55                  60
   Glu Trp Val Ala Val Ile Ser Tyr Glu Glu Ser Asn Arg Tyr His Ala
   65                  70                  75                  80
   Asp Ser Val Lys Gly Arg Phe Thr Ile Ser Arg Asp Asn Ser Lys Ile
                   85                  90                  95
   Thr Leu Tyr Leu Gln Met Asn Ser Leu Arg Thr Glu Asp Thr Ala Val
               100                 105                 110
   Tyr Tyr Cys Ala Arg Asp Gly Gly Ile Ala Ala Pro Gly Pro Asp Tyr
           115                 120                 125
   Trp Gly Gln Gly Thr Leu Val Thr Val Ser Ser Ala Ser Thr Lys Gly
       130                 135                 140
   Pro Ser Val Phe Pro Leu Ala Pro Ser Ser Lys Ser Thr Ser Gly Gly
   145                 150                 155                 160
   Thr Ala Ala Leu Gly Cys Leu Val Lys Asp Tyr Phe Pro Glu Pro Val
                   165                 170                 175
   Thr Val Ser Trp Asn Ser Gly Ala Leu Thr Ser Gly Val His Thr Phe
               180                 185                 190
   Pro Ala Val Leu Gln Ser Ser Gly Leu Tyr Ser Leu Ser Ser Val Val
           195                 200                 205
   Thr Val Pro Ser Ser Ser Leu Gly Thr Gln Thr Tyr Ile Cys Asn Val
       210                 215                 220
   Asn His Lys Pro Ser Asn Thr Lys Val Asp Lys Arg Val Glu Pro Lys
   225                 230                 235                 240
   Ser Cys Asp Lys Thr His Thr Cys Pro Pro Cys Pro Ala Pro Glu Leu
                   245                 250                 255
   Leu Gly Gly Pro Ser Val Phe Leu Phe Pro Pro Lys Pro Lys Asp Thr
               260                 265                 270
   Leu Met Ile Ser Arg Thr Pro Glu Val Thr Cys Val Val Val Asp Val
           275                 280                 285
   Ser His Glu Asp Pro Glu Val Lys Phe Asn Trp Tyr Val Asp Gly Val
       290                 295                 300
   Glu Val His Asn Ala Lys Thr Lys Pro Arg Glu Glu Gln Tyr Asn Ser
   305                 310                 315                 320
   Thr Tyr Arg Val Val Ser Val Leu Thr Val Leu His Gln Asp Trp Leu
                   325                 330                 335
   Asn Gly Lys Glu Tyr Lys Cys Lys Val Ser Asn Lys Ala Leu Pro Ala
               340                 345                 350
   Pro Ile Glu Lys Thr Ile Ser Lys Ala Lys Gly Gln Pro Arg Glu Pro
           355                 360                 365
   Gln Val Tyr Thr Leu Pro Pro Ser Arg Glu Glu Met Thr Lys Asn Gln
       370                 375                 380
   Val Ser Leu Thr Cys Leu Val Lys Gly Phe Tyr Pro Ser Asp Ile Ala
   385                 390                 395                 400
   Val Glu Trp Glu Ser Asn Gly Gln Pro Glu Asn Asn Tyr Lys Thr Thr
                   405                 410                 415
   Pro Pro Val Leu Asp Ser Asp Gly Ser Phe Phe Leu Tyr Ser Lys Leu
               420                 425                 430
   Thr Val Asp Lys Ser Arg Trp Gln Gln Gly Asn Val Phe Ser Cys Ser
           435                 440                 445
   Val Met His Glu Ala Leu His Asn His Tyr Thr Gln Lys Ser Leu Ser
       450                 455                 460
   Leu Ser Pro Gly Lys
   465
<210> 6
   <211> 239
   <212> PRT
   <213> Artificial Sequence
<220>
   <223> Light chain of 5.9 human anti-CD40 antibody
<400> 6
   Met Ala Leu Leu Ala Gln Leu Leu Gly Leu Leu Met Leu Trp Val Pro
    1               5                  10                  15
   Gly Ser Ser Gly Ala Ile Val Met Thr Gln Pro Pro Leu Ser Ser Pro
               20                  25                  30
   Val Thr Leu Gly Gln Pro Ala Ser Ile Ser Cys Arg Ser Ser Gln Ser
           35                  40                  45
   Leu Val His Ser Asp Gly Asn Thr Tyr Leu Asn Trp Leu Gln Gln Arg
       50                  55                  60
   Pro Gly Gln Pro Pro Arg Leu Leu Ile Tyr Lys Phe Phe Arg Arg Leu
   65                  70                  75                  80
   Ser Gly Val Pro Asp Arg Phe Ser Gly Ser Gly Ala Gly Thr Asp Phe
                   85                  90                  95
   Thr Leu Lys Ile Ser Arg Val Glu Ala Glu Asp Val Gly Val Tyr Tyr
               100                 105                 110
   Cys Met Gln Val Thr Gln Phe Pro His Thr Phe Gly Gln Gly Thr Arg
           115                 120                 125
   Leu Glu Ile Lys Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe Tyr Pro Arg Glu Ala Lys Val Gln Trp Lys Val Asp
                   165                 170                 175
   Asn Ala Leu Gln Ser Gly Asn Ser Gln Glu Ser Val Thr Glu Gln Asp
               180                 185                 190
   Ser Lys Asp Ser Thr Tyr Ser Leu Ser Ser Thr Leu Thr Leu Ser Lys
           195                 200                 205
   Ala Asp Tyr Glu Lys His Lys Val Tyr Ala Cys Glu Val Thr His Gln
       210                 215                 220
   Gly Leu Ser Ser Pro Val Thr Lys Ser Phe Asn Arg Gly Glu Cys
   225                 230                 235
<210> 7
   <211> 474
   <212> PRT
   <213> Artificial Sequence
<220>
   <223> Heavy chain of 5.9 human anti-CD40 antibody
<400> 7
   Met Gly Ser Thr Ala Ile Leu Ala Leu Leu Leu Ala Val Leu Gln Gly
    1               5                  10                  15
   Val Cys Ala Glu Val Gln Leu Val Gln Ser Gly Ala Glu Val Lys Lys
   20 25 30
   Pro Gly Glu Ser Leu Lys Ile Ser Cys Lys Gly Ser Gly Tyr Ser Phe
   35 40 45
   Thr Ser Tyr Trp Ile Gly Trp Val Arg Gln Met Pro Gly Lys Gly Leu
   50 55 60
   Glu Trp Met Gly Ile Ile Tyr Pro Gly Asp Ser Asp Thr Arg Tyr Ser
   65 70 75 80
   Pro Ser Phe Gln Gly Gln Val Thr Ile Ser Ala Asp Lys Ser Ile Ser
   85 90 95
   Thr Ala Tyr Leu Gln Trp Ser Ser Leu Lys Ala Ser Asp Thr Ala Met
   100 105 110
   Tyr Tyr Cys Ala Arg Gly Thr Ala Ala Gly Arg Asp Tyr Tyr Tyr Tyr
   115 120 125
   Tyr Gly Met Asp Val Trp Gly Gln Gly Thr Thr Val Thr Val Ser Ser
   130 135 140
   Ala Ser Thr Lys Gly Pro Ser Val Phe Pro Leu Ala Pro Ala Ser Lys
   145 150 155 160
   Ser Thr Ser Gly Gly Thr Ala Ala Leu Gly Cys Leu Val Lys Asp Tyr
   165 170 175
   Phe Pro Glu Pro Val Thr Val Ser Trp Asn Ser Gly Ala Leu Thr Ser
   180 185 190
   Gly Val His Thr Phe Pro Ala Val Leu Gln Ser Ser Gly Leu Tyr Ser
   195 200 205
   Leu Ser Ser Val Val Thr Val Pro Ser Ser Ser Leu Gly Thr Gln Thr
   210 215 220
   Tyr Ile Cys Asn Val Asn His Lys Pro Ser Asn Thr Lys Val Asp Lys
   225 230 235 240
   Arg Val Glu Pro Lys Ser Cys Asp Lys Thr His Thr Cys Pro Pro Cys
   245 250 255
   Pro Ala Pro Glu Leu Leu Gly Gly Pro Ser Val Phe Leu Phe Pro Pro
   260 265 270
   Lys Pro Lys Asp Thr Leu Met Ile Ser Arg Thr Pro Glu Val Thr Cys
   275 280 285
   Val Val Val Asp Val Ser His Glu Asp Pro Glu Val Lys Phe Asn Trp
   290 295 300
   Tyr Val Asp Gly Val Glu Val His Asn Ala Lys Thr Lys Pro Arg Glu
   305 310 315 320
   Glu Gln Tyr Asn Ser Thr Tyr Arg Val Val Ser Val Leu Thr Val Leu
   325 330 335
   His Gln Asp Trp Leu Asn Gly Lys Glu Tyr Lys Cys Lys Val Ser Asn
   340 345 350
   Lys Ala Leu Pro Ala Pro Ile Glu Lys Thr Ile Ser Lys Ala Lys Gly
   355 360 365
   Gln Pro Arg Glu Pro Gln Val Tyr Thr Leu Pro Pro Ser Arg Glu Glu
   370 375 380
   Met Thr Lys Asn Gln Val Ser Leu Thr Cys Leu Val Lys Gly Phe Tyr
   385 390 395 400
   Pro Ser Asp Ile Ala Val Glu Trp Glu Ser Asn Gly Gln Pro Glu Asn
   405 410 415
   Asn Tyr Lys Thr Thr Pro Pro Val Leu Asp Ser Asp Gly Ser Phe Phe
   420 425 430
   Leu Tyr Ser Lys Leu Thr Val Asp Lys Ser Arg Trp Gln Gln Gly Asn
   435 440 445
   Val Phe Ser Cys Ser Val Met His Glu Ala Leu His Asn His Tyr Thr
   450 455 460
   Gln Lys Ser Leu Ser Leu Ser Pro Gly Lys
   465 470
<210> 8
   <211> 474
   <212> PRT
   <213> Artificial Sequence
<220>
   <223> Heavy chain of variant of 5.9 human anti-CD40 antibody
<400> 8
   Met Gly Ser Thr Ala Ile Leu Ala Leu Leu Leu Ala Val Leu Gln Gly
   1               5                   10                  15
   Val Cys Ala Glu Val Gln Leu Val Gln Ser Gly Ala Glu Val Lys Lys
               20                  25                  30
   Pro Gly Glu Ser Leu Lys Ile Ser Cys Lys Gly Ser Gly Tyr Ser Phe
           35                  40                  45
   Thr Ser Tyr Trp Ile Gly Trp Val Arg Gln Met Pro Gly Lys Gly Leu
       50                  55                  60
   Glu Trp Met Gly Ile Ile Tyr Pro Gly Asp Ser Asp Thr Arg Tyr Ser
   65                  70                  75                  80
   Pro Ser Phe Gln Gly Gln Val Thr Ile Ser Ala Asp Lys Ser Ile Ser
                   85                  90                  95
   Thr Ala Tyr Leu Gln Trp Ser Ser Leu Lys Ala Ser Asp Thr Ala Met
               100                 105                 110
   Tyr Tyr Cys Ala Arg Gly Thr Ala Ala Gly Arg Asp Tyr Tyr Tyr Tyr
           115                 120                 125
   Tyr Gly Met Asp Val Trp Gly Gln Gly Thr Thr Val Thr Val Ser Ser
       130                 135                 140
   Ala Ser Thr Lys Gly Pro Ser Val Phe Pro Leu Ala Pro Ser Ser Lys
   145                 150                 155                 160
   Ser Thr Ser Gly Gly Thr Ala Ala Leu Gly Cys Leu Val Lys Asp Tyr
                   165                 170                 175
   Phe Pro Glu Pro Val Thr Val Ser Trp Asn Ser Gly Ala Leu Thr Ser
               180                 185                 190
   Gly Val His Thr Phe Pro Ala Val Leu Gln Ser Ser Gly Leu Tyr Ser
           195                 200                 205
   Leu Ser Ser Val Val Thr Val Pro Ser Ser Ser Leu Gly Thr Gln Thr
       210                 215                 220
   Tyr Ile Cys Asn Val Asn His Lys Pro Ser Asn Thr Lys Val Asp Lys
   225                 230                 235                 240
   Arg Val Glu Pro Lys Ser Cys Asp Lys Thr His Thr Cys Pro Pro Cys
                   245                 250                 255
   Pro Ala Pro Glu Leu Leu Gly Gly Pro Ser Val Phe Leu Phe Pro Pro
               260                 265                 270
   Lys Pro Lys Asp Thr Leu Met Ile Ser Arg Thr Pro Glu Val Thr Cys
           275                 280                 285
   Val Val Val Asp Val Ser His Glu Asp Pro Glu Val Lys Phe Asn Trp
       290                 295                 300
   Tyr Val Asp Gly Val Glu Val His Asn Ala Lys Thr Lys Pro Arg Glu
   305                 310                 315                 320
   Glu Gln Tyr Asn Ser Thr Tyr Arg Val Val Ser Val Leu Thr Val Leu
                   325                 330                 335
   His Gln Asp Trp Leu Asn Gly Lys Glu Tyr Lys Cys Lys Val Ser Asn
               340                 345                 350
   Lys Ala Leu Pro Ala Pro Ile Glu Lys Thr Ile Ser Lys Ala Lys Gly
           355                 360                 365
   Gln Pro Arg Glu Pro Gln Val Tyr Thr Leu Pro Pro Ser Arg Glu Glu
       370                 375                 380
   Met Thr Lys Asn Gln Val Ser Leu Thr Cys Leu Val Lys Gly Phe Tyr
   385                 390                 395                 400
   Pro Ser Asp Ile Ala Val Glu Trp Glu Ser Asn Gly Gln Pro Glu Asn
                   405                 410                 415
   Asn Tyr Lys Thr Thr Pro Pro Val Leu Asp Ser Asp Gly Ser Phe Phe
               420                 425                 430
   Leu Tyr Ser Lys Leu Thr Val Asp Lys Ser Arg Trp Gln Gln Gly Asn
           435                 440                 445
   Val Phe Ser Cys Ser Val Met His Glu Ala Leu His Asn His Tyr Thr
       450                 455                 460
   Gln Lys Ser Leu Ser Leu Ser Pro Gly Lys
   465                 470
<210> 9
   <211> 612
   <212> DNA
   <213> Homo sapiens
<220>
   <221> CDS
   <222> (1)...(612)
<221> misc_feature
   <222> (0)...(0)
   <223> Coding sequence for short isoform of human CD40
<400> 9
   atg gtt cgt ctg cct ctg cag tgc gtc ctc tgg ggc tgc ttg ctg acc    48
   Met Val Arg Leu Pro Leu Gln Cys Val Leu Trp Gly Cys Leu Leu Thr
    1               5                  10                  15
   gct gtc cat cca gaa cca ccc act gca tgc aga gaa aaa cag tac cta    96
   Ala Val His Pro Glu Pro Pro Thr Ala Cys Arg Glu Lys Gln Tyr Leu
               20                  25                  30
   ata aac agt cag tgc tgt tct ttg tgc cag cca gga cag aaa ctg gtg    144
   Ile Asn Ser Gln Cys Cys Ser Leu Cys Gln Pro Gly Gln Lys Leu Val
           35                  40                  45
   agt gac tgc aca gag ttc act gaa acg gaa tgc ctt cct tgc ggt gaa    192
   Ser Asp Cys Thr Glu Phe Thr Glu Thr Glu Cys Leu Pro Cys Gly Glu
       50                  55                  60
   agc gaa ttc cta gac acc tgg aac aga gag aca cac tgc cac cag cac    240
   Ser Glu Phe Leu Asp Thr Trp Asn Arg Glu Thr His Cys His Gln His
   65                  70                  75                  80
   aaa tac tgc gac ccc aac cta ggg ctt cgg gtc cag cag aag ggc acc    288
   Lys Tyr Cys Asp Pro Asn Leu Gly Leu Arg Val Gln Gln Lys Gly Thr
                   85                  90                  95
   tca gaa aca gac acc atc tgc acc tgt gaa gaa ggc tgg cac tgt acg    336
   Ser Glu Thr Asp Thr Ile Cys Thr Cys Glu Glu Gly Trp His Cys Thr
               100                 105                 110
   agt gag gcc tgt gag agc tgt gtc ctg cac cgc tca tgc tcg ccc ggc    384
   Ser Glu Ala Cys Glu Ser Cys Val Leu His Arg Ser Cys Ser Pro Gly
           115                 120                 125
   ttt ggg gtc aag cag att gct aca ggg gtt tct gat acc atc tgc gag    432
   Phe Gly Val Lys Gln Ile Ala Thr Gly Val Ser Asp Thr Ile Cys Glu
       130                 135                 140
   ccc tgc cca gtc ggc ttc ttc tcc aat gtg tca tct gct ttc gaa aaa    480
   Pro Cys Pro Val Gly Phe Phe Ser Asn Val Ser Ser Ala Phe Glu Lys
   145                 150                 155                 160
   tgt cac cct tgg aca agg tcc cca gga tcg gct gag agc cct ggt ggt    528
   Cys His Pro Trp Thr Arg Ser Pro Gly Ser Ala Glu Ser Pro Gly Gly
                   165                 170                 175
   gat ccc cat cat ctt cgg gat cct gtt tgc cat cct ctt ggt gct ggt    576
   Asp Pro His His Leu Arg Asp Pro Val Cys His Pro Leu Gly Ala Gly
               180                 185                 190
   ctt tat caa aaa ggt ggc caa gaa gcc aac caa taa                    612
   Leu Tyr Gln Lys Gly Gly Gln Glu Ala Asn Gln  *
           195                 200
<210> 10
   <211> 203
   <212> PRT
   <213> Homo sapiens
<400> 10
   Met Val Arg Leu Pro Leu Gln Cys Val Leu Trp Gly Cys Leu Leu Thr
    1               5                  10                  15
   Ala Val His Pro Glu Pro Pro Thr Ala Cys Arg Glu Lys Gln Tyr Leu
               20                  25                  30
   Ile Asn Ser Gln Cys Cys Ser Leu Cys Gln Pro Gly Gln Lys Leu Val
           35                  40                  45
   Ser Asp Cys Thr Glu Phe Thr Glu Thr Glu Cys Leu Pro Cys Gly Glu
       50                  55                  60
   Ser Glu Phe Leu Asp Thr Trp Asn Arg Glu Thr His Cys His Gln His
   65                  70                  75                  80
   Lys Tyr Cys Asp Pro Asn Leu Gly Leu Arg Val Gln Gln Lys Gly Thr
                   85                  90                  95
   Ser Glu Thr Asp Thr Ile Cys Thr Cys Glu Glu Gly Trp His Cys Thr
               100                 105                 110
   Ser Glu Ala Cys Glu Ser Cys Val Leu His Arg Ser Cys Ser Pro Gly
           115                 120                 125
   Phe Gly Val Lys Gln Ile Ala Thr Gly Val Ser Asp Thr Ile Cys Glu
       130                 135                 140
   Pro Cys Pro Val Gly Phe Phe Ser Asn Val Ser Ser Ala Phe Glu Lys
   145                 150                 155                 160
   Cys His Pro Trp Thr Arg Ser Pro Gly Ser Ala Glu Ser Pro Gly Gly
                   165                 170                 175
   Asp Pro His His Leu Arg Asp Pro Val Cys His Pro Leu Gly Ala Gly
               180                 185                 190
   Leu Tyr Gln Lys Gly Gly Gln Glu Ala Asn Gln
           195                 200
<210> 11
   <211> 834
   <212> DNA
   <213> Homo sapiens
<220>
   <221> CDS
   <222> (1)...(834)
<221> misc_feature
   <222> (0)...(0)
   <223> Coding sequence for long isoform of human CD40
<400> 11
   atg gtt cgt ctg cct ctg cag tgc gtc ctc tgg ggc tgc ttg ctg acc     48
   Met Val Arg Leu Pro Leu Gln Cys Val Leu Trp Gly Cys Leu Leu Thr
    1               5                  10                  15
   gct gtc cat cca gaa cca ccc act gca tgc aga gaa aaa cag tac cta     96
   Ala Val His Pro Glu Pro Pro Thr Ala Cys Arg Glu Lys Gln Tyr Leu
               20                  25                  30
   ata aac agt cag tgc tgt tct ttg tgc cag cca gga cag aaa ctg gtg    144
   Ile Asn Ser Gln Cys Cys Ser Leu Cys Gln Pro Gly Gln Lys Leu Val
           35                  40                  45
   agt gac tgc aca gag ttc act gaa acg gaa tgc ctt cct tgc ggt gaa    192
   Ser Asp Cys Thr Glu Phe Thr Glu Thr Glu Cys Leu Pro Cys Gly Glu
       50                  55                  60
   agc gaa ttc cta gac acc tgg aac aga gag aca cac tgc cac cag cac    240
   Ser Glu Phe Leu Asp Thr Trp Asn Arg Glu Thr His Cys His Gln His
    65                  70                  75                  80
   aaa tac tgc gac ccc aac cta ggg ctt cgg gtc cag cag aag ggc acc     288
   Lys Tyr Cys Asp Pro Asn Leu Gly Leu Arg Val Gln Gln Lys Gly Thr
                   85                  90                  95
   tca gaa aca gac acc atc tgc acc tgt gaa gaa ggc tgg cac tgt acg     336
   Ser Glu Thr Asp Thr Ile Cys Thr Cys Glu Glu Gly Trp His Cys Thr
               100                 105                 110
   agt gag gcc tgt gag agc tgt gtc ctg cac cgc tca tgc tcg ccc ggc     384
   Ser Glu Ala Cys Glu Ser Cys Val Leu His Arg Ser Cys Ser Pro Gly
           115                 120                 125
   ttt ggg gtc aag cag att gct aca ggg gtt tct gat acc atc tgc gag     432
   Phe Gly Val Lys Gln Ile Ala Thr Gly Val Ser Asp Thr Ile Cys Glu
       130                 135                 140
   ccc tgc cca gtc ggc ttc ttc tcc aat gtg tca tct gct ttc gaa aaa     480
   Pro Cys Pro Val Gly Phe Phe Ser Asn Val Ser Ser Ala Phe Glu Lys
   145                 150                 155                 160
   tgt cac cct tgg aca agc tgt gag acc aaa gac ctg gtt gtg caa cag     528
   Cys His Pro Trp Thr Ser Cys Glu Thr Lys Asp Leu Val Val Gln Gln
                   165                 170                 175
   gca ggc aca aac aag act gat gtt gtc tgt ggt ccc cag gat cgg ctg     576
   Ala Gly Thr Asn Lys Thr Asp Val Val Cys Gly Pro Gln Asp Arg Leu
               180                 185                 190
   aga gcc ctg gtg gtg atc ccc atc atc ttc ggg atc ctg ttt gcc atc     624
   Arg Ala Leu Val Val Ile Pro Ile Ile Phe Gly Ile Leu Phe Ala Ile
           195                 200                 205
   ctc ttg gtg ctg gtc ttt atc aaa aag gtg gcc aag aag cca acc aat     672
   Leu Leu Val Leu Val Phe Ile Lys Lys Val Ala Lys Lys Pro Thr Asn
       210                 215                 220
   aag gcc ccc cac ccc aag cag gaa ccc cag gag atc aat ttt ccc gac     720
   Lys Ala Pro His Pro Lys Gln Glu Pro Gln Glu Ile Asn Phe Pro Asp
   225                 230                 235                 240
   gat ctt cct ggc tcc aac act gct gct cca gtg cag gag act tta cat     768
   Asp Leu Pro Gly Ser Asn Thr Ala Ala Pro Val Gln Glu Thr Leu His
                   245                 250                 255
   gga tgc caa ccg gtc acc cag gag gat ggc aaa gag agt cgc atc tca     816
   Gly Cys Gln Pro Val Thr Gln Glu Asp Gly Lys Glu Ser Arg Ile Ser
               260                 265                 270
   gtg cag gag aga cag tga                                             834
   Val Gln Glu Arg Gln  *
           275
<210> 12
   <211> 277
   <212> PRT
   <213> Homo sapiens
<400> 12
   Met Val Arg Leu Pro Leu Gln Cys Val Leu Trp Gly Cys Leu Leu Thr
   1 5 10 15
   Ala Val His Pro Glu Pro Pro Thr Ala Cys Arg Glu Lys Gln Tyr Leu
   20 25 30
   Ile Asn Ser Gln Cys Cys Ser Leu Cys Gln Pro Gly Gln Lys Leu Val
   35 40 45
   Ser Asp Cys Thr Glu Phe Thr Glu Thr Glu Cys Leu Pro Cys Gly Glu
   50 55 60
   Ser Glu Phe Leu Asp Thr Trp Asn Arg Glu Thr His Cys His Gln His
   65 70 75 80
   Lys Tyr Cys Asp Pro Asn Leu Gly Leu Arg Val Gln Gln Lys Gly Thr
   85 90 95
   Ser Glu Thr Asp Thr Ile Cys Thr Cys Glu Glu Gly Trp His Cys Thr
   100 105 110
   Ser Glu Ala Cys Glu Ser Cys Val Leu His Arg Ser Cys Ser Pro Gly
   115 120 125
   Phe Gly Val Lys Gln Ile Ala Thr Gly Val Ser Asp Thr Ile Cys Glu
   130 135 140
   Pro Cys Pro Val Gly Phe Phe Ser Asn Val Ser Ser Ala Phe Glu Lys
   145 150 155 160
   Cys His Pro Trp Thr Ser Cys Glu Thr Lys Asp Leu Val Val Gln Gln
   165 170 175
   Ala Gly Thr Asn Lys Thr Asp Val Val Cys Gly Pro Gln Asp Arg Leu
   180 185 190
   Arg Ala Leu Val Val Ile Pro Ile Ile Phe Gly Ile Leu Phe Ala Ile
   195 200 205
   Leu Leu Val Leu Val Phe Ile Lys Lys Val Ala Lys Lys Pro Thr Asn
   210 215 220
   Lys Ala Pro His Pro Lys Gln Glu Pro Gln Glu Ile Asn Phe Pro Asp
   225 230 235 240
   Asp Leu Pro Gly Ser Asn Thr Ala Ala Pro Val Gln Glu Thr Leu His
   245 250 255
   Gly Cys Gln Pro Val Thr Gln Glu Asp Gly Lys Glu Ser Arg Ile Ser
   260 265 270
   Val Gln Glu Arg Gln
   275